# EUROPEAN PATENT SPECIFICATION

(11) **EP 2 941 245 B1**
(45) Date of publication and mention of the grant of the patent: **26.06.2024**
(21) Application number: 14702108.3
(22) Date of filing: 03.01.2014
(51) Int. Cl.: A61K 9/28, A61K 31/155, A61P 1/12, A61P 1/18, A61P 3/00, A61P 1/00, A61P 3/10, A61P 43/00

(54) **DELAYED-RELEASE COMPOSITION COMPRISING BIGUANIDE**
BIGUANIDFORMULIERUNG MIT VERZÖGERTER FREISETZUNG
FORMULATION À LIBÉRATION RETARDÉE COMPRENANT DE BIGUANIDE

(30) Priority: 05.01.2013 US 201361749307 P
(43) Date of publication of application: 11.11.2015
(73) Proprietor: Anji Pharmaceuticals Inc., Cambridge, MA 02142 (US)
(72) Inventor: BARON, Alain D., San Diego, CA 92130 (US); FINEMAN, Mark S., San Diego, CA 92131 (US); KIM, Terri, Carlsbad, CA 92009 (US); DORDUNOO, Stephen Kwaku, Halethorpe, MD 21227 (US)
(74) Representative: Ricker, Mathias
(86) International application number: PCT/US2014/010240
(87) International publication number: WO 2014/107617

(56) References cited:
- WO-A2-2013/103919
- US-A1- 2006 222 709
- US-A1- 2012 177 730
- LEVY JULIANA ET AL: "Assessment of efficacy and tolerability of once-daily extended release metformin in patients with type 2 diabetes mellitus", DIABETOLOGY & METABOLIC SYNDROME, BIOMED CENTRAL LTD, LONDON, UK, vol. 2, no. 1, 18 March 2010 (2010-03-18), page 16, XP021070597, ISSN: 1758-5996

## Description

### FIELD OF THE INVENTION

The present invention relates generally to improved delayed-release compositions for biguanide compound delivery and to the use of the compositions in methods of treating metabolic disorders, and to improving the pharmacokinetics and gastrointestinal tolerability of such compounds.

### BACKGROUND OF THE INVENTION

Hyperglycemia, hyperglycæmia, or high blood sugar, is a condition in which an excessive amount of glucose, e.g., greater than about 125 mg/dL, circulates in the blood plasma. Chronic hyperglycemia at levels that are more than slightly above normal can produce a wide variety of serious complications over a period of years, including kidney damage, neurological damage, cardiovascular damage, damage to the retina, or damage to the feet and legs. Diabetic neuropathy may be a result of long-term hyperglycemia.

Hyperglycemia may be caused by or associated with dysfunction of the thyroid, adrenal, and pituitary glands, diseases of the pancreas, severe sepsis, and intracranial diseases such as encephalitis, brain tumors, and meningitis. By far the most common cause of chronic hyperglycemia is diabetes mellitus, which is widely considered by many to be a looming health care epidemic. In diabetes mellitus, the hyperglycemia typically results from low insulin levels (type I diabetes) and/or insulin resistance at the cellular level (type II diabetes).

Many type II diabetes medications are designed to lower blood glucose levels. A first line drug of choice for the treatment of type II diabetes, and the most commonly prescribed antidiabetic medication in the world, is metformin. In contrast to most diabetes medications, hypoglycemia with metformin is rare; it is also weight neutral and is associated with reduced cardiovascular events and reduced mortality.

Metformin (dimethylbiguanide) belongs to a class of biguanide drugs developed based on a glucose-lowering extract containing guanidines from the *Galega officinalis* plant. (Bailey & Turner Metformin. N Engl J Med. 1996 Feb 29;334(9): 574-9; Bailey et al. Metformin: its botanical background. Practical Diabetes Int. 2004;21(3):115-7). Originally synthesized as a side product in 1921, (Werner E, Bell J. The preparation of methylguanidine, and of ββ-dimethylguanidine by the interaction of dicyanodiamide, and methylammonium and dimethylammonium chlorides respectively. J Chem Soc, Transactions. 1921;121:1790-5), metformin and other biguanides were found to lower blood glucose in animals. Studies on the glucose-lowering effects of metformin, phenformin and buformin in humans were published in the 1950s. At first, the greater potency of phenformin and buformin resulted in their more widespread use; however, their association with lactic acidosis ultimately led to discontinuation in most countries by the end of the 1970s.

Metformin improves glucose tolerance in patients by lowering both basal and postprandial plasma glucose. Metformin monotherapy generally lowers fasting blood glucose by 20% and HbA1c levels by approximately 1.5%. (Bailey & Turner, *supra;* DeFronzo & Goodman Efficacy of metformin in patients with non-insulin-dependent diabetes mellitus. The Multicenter Metformin Study Group. N Engl J Med. 1995 Aug 31;333(9):541-9). Metformin has also been shown to improve serum lipids, decreasing triglycerides, free fatty acids, and LDL-cholesterol and modestly increasing HDL-cholesterol. (Bailey & Turner, *supra*.)

Metformin's antihyperglycemic effects have been postulated to result from a wide variety of systemic biochemical interactions including, e.g., suppressing glucose production by the liver, increasing insulin sensitivity, enhancing peripheral glucose uptake (by phosphorylating GLUT-4 enhancer factor), increasing fatty acid oxidation, and/or decreasing absorption of glucose from the gastrointestinal tract. (Hundal & Inzucchi Metformin: new understandings, new uses. Drugs. 2003;63(18):1879-94). More recently, investigators have focused on its apparent impact on the secretion of glucagon-like peptide-1 (GLP-1), apparently determining that metformin does not act directly on L cells in the gut to induce GLP-1 secretion or enhance L cell sensitivity to several known secretagogues. (Mulherin et al., Mechanisms underlying metformin-induced secretion of glucagon-like peptide-1 from the intestinal L cell. Endocrinology 152:4610-19 (December 2011)). These investigators suggested that metformin stimulates GLP-1 release through an indirect mechanism involving both muscarinic (M3) receptor-dependent and Gastrin Releasing Peptide (GRP) pathways independent of intestinal L cells, such that systemic bioavailability of metformin is critical to therapeutic efficacy. Chemosensory receptor ligand-based therapies, including compositions comprising metformin and salts thereof are already disclosed in US2012/0177730 and WO2013/103919. Further, US2006/0222709 discloses enteric-coated metformin tablets for treating chronic constipation without addressing or mentioning problems of side effects and tolerability.

Unfortunately, however, systemic exposure of metformin still poses a serious risk of lactic acidosis for several patient populations. Lactic acidosis is a potentially fatal metabolic complication that occurs when lactic acid levels increase in the bloodstream. Accordingly, metformin is contraindicated in people with any condition that could increase the risk of lactic acidosis, including kidney disorders, lung disease, and liver disease. According to the prescribing information, heart failure, in particular, unstable or acute congestive heart failure, also increases risk of lactic acidosis with metformin. Thus, metformin remains unavailable to treat hyperglycemia in patients with these contraindications.

Moreover, conventional metformin formulations often produce dose-limiting adverse gastrointestinal (GI) complications including diarrhea, nausea, vomiting, dizziness, headaches and dyspepsia. Accordingly, patient administration is generally titrated upward over a period of time to a maximum tolerated dose based in not insignificant part on any resulting patient-specific adverse GI effects. Extended-release formulations have been developed in the hopes of addressing this, but have not adequately resolved these problems.

Clearly, there continues to be a need for better and safer compositions and methods for delivering biguanide compounds that address these tolerability and safety concerns. Ideally, these would also provide more effective treatment options for metabolic disorders in patients having contraindications for metformin and/or other biguanides.

### SUMMARY OF THE INVENTION

The present invention resolves these longstanding problems with conventional delivery of biguanide compounds by delaying release of the compounds in the upper gastrointestinal tract and ensuring passage to and preferably through the duodenum before dissolution. As demonstrated herein, short-term fluctuations in stomach pH due to meals and other factors can lead to aberrant release patterns and produce spikes in systemic exposure to the biguanide compound, thereby increasing the risk of adverse events including gastrointestinal complications and, more dangerously, lactic acidosis in otherwise contraindicated patients.

In particular, the invention provides pharmaceutical compositions and formulations that deliver biguanide compounds to specific segments of the intestine while substantially avoiding absorption of the biguanide in the stomach and duodenum, wherein the subject formulations are adapted to include both delayed release (DR) for delivery at a desired pH, *e.g.,* delivery at the pH of the distal small intestine, as well as a lag phase (LP) after contacting the desired pH during which drug release from the subject formulation is minimized in order to accommodate transient pH fluctuations in the stomach and ensure delayed release beyond the duodenum.

In one aspect, the formulation comprises a delayed-release pharmaceutical composition for biguanide compound delivery, comprising an oral dosage form having a core comprising a therapeutically effective amount of a biguanide compound and an enteric coating surrounding said core that delays release of said biguanide compound after ingestion until reaching the distal small intestine, wherein said oral dosage form is an enterically-coated tablet or capsule, wherein said enteric coating is applied to said oral dosage form to about 3% to about a 6% (wt/wt) weight gain, and wherein less than 15% of the biguanide compound is released after the composition contacts an aqueous medium of 0.1 N HCl for two hours and is subsequently transferred to an aqueous medium at a pH of about 6.8 for a lag phase of at least ten minutes, and at least 60% of the biguanide compound is released after the lag phase and within 60 minutes at pH 6.8, and at least 90% of the biguanide compound is released within 90 to 120 minutes at pH 6.8..

In one embodiment, an enteric coating is applied to the subject compositions at a weight gain of at least about 4.5 mg/cm², 5 mg/cm², 5.5 mg/cm², 6 mg/cm², 6.5 mg/cm², 7 mg/cm², 7.5 mg/cm², 8 mg/cm², 9 mg/cm², 10 mg/cm², 11 mg/cm², 12 mg/cm², or 15 mg/cm². In a further embodiment, the enteric coating comprises an external layer on said formulation of at least about 5 mg/cm² to 7 mg/cm², more preferably at least about 5.5 mg/cm² to at least about 6.5 mg/cm². As demonstrated herein, this ensures appropriate release at the desired intestinal location so as to avoid aberrant spikes in systemic biguanide compound exposure.

In alternative embodiments, an enteric coating of at least about 3.5% or 4% (wt/wt) weight gain is applied to the subject composition to produce the desired lag phase. For tablets and capsules, the enteric coating is applied to achieve about a 3% to at least about a 6% (wt/wt) weight gain.

In alternative embodiments, an enteric coating is applied to the pharmaceutical composition to achieve at least about a 4% to at least about a 6% (wt/wt) weight gain.

Described herein are pharmaceutical compositions comprising an enterically-coated oral dosage form comprising a biguanide compound, wherein the dosage form is adapted to release less than about 10% 5%, 4%, 3%, 2% and preferably less than 1% of the biguanide compound after contacting an aqueous medium (e.g., submersion) at a pH of less than about 2 for about two hours followed by contacting an aqueous medium at a pH equal to or less than about 5.5 for at least 30 to 45 minutes. As described herein, the enterically-coated dosage form releases less than about 5%, 2 % or 1% of the biguanide compound in an aqueous medium of 0.1 N HCl for two hours and less than about 5%, 2% or 1% when transferred to an aqueous medium at pH 5.5 for at least 30 to 45 minutes.

In further embodiments, the enterically-coated dosage form releases less than 15%, 10%, 5%, 3%, 2% or less than 1% of the biguanide compound during the lag phase after the dosage form is contacted with an aqueous medium at a pH of about 6.8, wherein the lag phase is at least ten, fifteen or twenty minutes. In a preferred embodiment, the enterically-coated dosage form releases less than about 15% of the biguanide compound when the dosage form is contacted with an aqueous medium at a pH of about 6.8 for a lag phase of at least ten minutes and releases greater than 90%, 95%, 98%, or 99% of the biguanide compound after contacting with an aqueous medium at a pH of about 6.8 for a total of ninety to 120 minutes.

In two-stage dissolution embodiments, pharmaceutical compositions are provided comprising an enterically-coated oral dosage form comprising a biguanide compound, wherein the dosage form is adapted to release less than 5%, 2% or 1% of the biguanide compound in an aqueous medium of 0.1 N HCl for two hours. In these embodiments, less than 15%, 10%, 5%, 3%, 2%, or preferably 1% of the biguanide compound is released after contacting an aqueous medium of 0.1 N HCl for two hours and subsequently transferred to an aqueous medium at a pH of about 6.8 for a lag phase of at least ten, fifteen or twenty minutes. In preferred embodiments, less than 15% of the biguanide compound is released after two hours at acid pH and a lag phase of at least ten or fifteen minutes at pH 6.8, and at least 60% of the biguanide compound is released after the lag phase and within 60 minutes at pH 6.8, and at least 90% of the biguanide compound is released within 90 to 120 minutes at pH 6.8.

In three-stage dissolution embodiments, pharmaceutical compositions are provided comprising an enterically-coated oral dosage form comprising a biguanide compound, wherein the dosage form is adapted to release less than 5%, 2% or 1% of the biguanide compound in an aqueous medium of 0.1 N HCl for two hours and less than 5%, 2% or 1% when transferred to an aqueous medium at pH 5.5 for at least one hour. In these embodiments, less than 15%, 10%, or 5% of the biguanide compound is released after two hours in aqueous medium of 0.1 N HCl, 30 minutes in an aqueous medium at pH 5.5, and during a lag phase of at least ten or fifteen minutes at pH 6.8. In preferred embodiments, less than 15%, 10% or 5% of the biguanide compound is released after two hours at acid pH, 30 minutes at pH 5.5 and a lag phase of at least ten or fifteen minutes at pH 6.8, and at least 60% of the biguanide compound is released after the lag phase and within 60 minutes at pH 6.8, and at least 90% of the biguanide compound is released within 90 to 120 minutes at pH 6.8.

In some embodiments, the enteric coating comprises a first polymer which minimizes the release of the biguanide compound for at least about 10 minutes after contacting a pH of 6.8 or 7.0, more preferably for at least about 15 or 20 minutes, still more preferably for at least about 25 or 30 minutes after contacting a pH of 6.8 or 7.0. In preferred embodiments the polymer is insoluble in acidic media, but dissolves by salt formation or the like above pH 7.0. In an exemplary preferred embodiment the polymer is selected from the group consisting of Eudragit FS, Eudragit S, shellac, and/or combinations thereof.

In further embodiments, the enteric coating further comprises a second polymer that dissolves at a lower pH than the first polymer. In preferred embodiments, the second polymer is insoluble at pH 5.5 and below, but dissolves by salt formation or the like above pH 5.5. In an exemplary preferred embodiment the second polymer is selected from the group consisting of Eudragit L, cellulose acetate succinate, hydroxy propyl methyl cellulose phthalate, hydroxy propyl methyl cellulose acetate succinate (hypromellose acetate succinate), polyvinyl acetate phthalate (PVAP) and sodium alginate, stearic acid, and/or combinations thereof.

In some embodiments the enteric coating comprises about 90% Eudragit FS and about 10% Eudragit L, about 80% Eudragit FS and about 20% Eudragit L, about 70% Eudragit FS and about 30% Eudragit L, about 60% Eudragit FS and about 40% Eudragit L, about 50% Eudragit FS and about 50% Eudragit L, about 40% Eudragit FS and about 60% Eudragit L, about 30% Eudragit FS and about 70% Eudragit L, about 20% Eudragit FS and about 80% Eudragit L, or about 10% Eudragit FS and about 90% Eudragit L. In preferred embodiments, Eudragit FS and said Eudragit L are present in about a 7:5 to about a 5:7 ratio, and more preferably about a 6:4 to about a 4:6 ratio. In an exemplary preferred embodiment, the enteric coating comprises about 60% Eudragit FS and about 40% Eudragit L.

In some embodiments, a seal coating may be added between the core comprising the biguanide compound and the enteric coating. The seal coating material may be selected so as to have no effect on the drug release. Suitable materials include, e.g., hydroxypropylmethyl cellulose (HPMC). In other embodiments, the seal coating material may be selected to extend the lag phase so as to further slow drug release after the enteric coating is breached. Suitable materials include, *e.g*. Eudragit E which dissolves in acid but swells at higher pH, and may be used to extend the lag phase after the enteric coating has been breached.

In one exemplary and preferred embodiment, the seal coating comprises a mixture of hypromellose, titanium dioxide, polyethylene glycol 400 (macrogol), and polysorbate 80, *e.g.* Opadry^{®} White YS-1-7003 available from Colorcon Inc. In an alternative exemplary and preferred embodiment, the seal coating comprises hypromellose, triacetin, and talc, *e.g*. Opadry^{®} 03K19229 Clear also available from Colorcon Inc.

Accordingly, the subject formulations and compositions may further comprise a seal coating between the biguanide compound and the enteric coating, providing a total coating thickness corresponding to at least about 4% to 8% (wt./wt.) weight gain, more preferably at least about 4.5% to 6.0% (wt./wt.) weight gain. In some embodiments, the combination of the outer enteric coating and inner seal coat comprises at least about 6.9 mg/cm² to 13.3 mg/cm², more preferably at least about 7.8 mg/cm² to at least about 11.4 mg/cm².

In alternative embodiments, the subject formulations and compositions further comprise one or more disintegrants to accelerate the dissolution of the core upon breaching of the enteric coating. In preferred embodiments, the disintegrant comprises croscarmellose sodium, sodium starch glycolate, or combinations thereof.

In one embodiment, the biguanide compound is selected from the group consisting of metformin, phenformin, buformin and imeglimin. In a preferred embodiment, the biguanide compound comprises metformin or a salt thereof, preferably metformin hydrochloride.

In additional embodiments, the oral dosage forms disclosed herein further comprise a DPP-IV inhibitor. In another embodiment, the oral dosage forms disclosed herein further comprise an additional anti-obesity and/or or anti-diabetes agent.

The oral dosage forms disclosed herein may take the form of a tablet or capsule, which is enterically coated. Preferably, the tablet or capsule is smooth and does not comprise an embossed surface. However, tablets or capsules comprising an embossed surface are alternative embodiments encompassed, wherein the coating thickness of the enteric coating and/or seal coating are adjusted accordingly to provide the delayed release and lag phase release profiles described herein.

Correspondingly, compositions are provided for use in the treatment of metabolic disorders in patients, including otherwise contraindicated patient populations, by administering the lag phase-enhanced delayed-release formulations according to the invention having the requisite coating to ensure targeted delivery of the biguanide compound to the small intestine of the patient, and preferably the distal small intestine, and thereby minimize systemic bioavailability. The compositions of the invention may be administered to a subject in need thereof to treat various metabolic disorders, including obesity, dislipidemia or other disorders of lipid metabolism as well as hyperglycemic conditions and histopathological diseases associated with hyperglycemia, including type II diabetes, prediabetes, gestational diabetes and polycystic ovary syndrome. The effective use of biguanide compounds for prophylaxis and prevention of such diseases and disorders, as well as for more general weight loss purposes in overweight or mildly to severely obese individuals, is also explicitly contemplated.

In one aspect, the pharmaceutical composition according to the invention for use in a method of reducing the risk of an adverse event from biguanide administration is provided. In one embodiment, the adverse event is lactic acidosis. In another event, the adverse event is a gastrointestinal complication selected from the group comprising nausea, diarrhea, dyspepsia, and vomiting.

In an embodiment, the pharmaceutical composition for use in a methods of reducing the risk of an adverse event from biguanide administration is provided, comprising an enterically-coated oral dosage form comprising a biguanide compound to be administered to a patient in need thereof, wherein the dosage form is adapted to release less than about 10% 5%, 4%, 3%, 2% and preferably less than 1% of the biguanide compound after contacting an aqueous medium at a pH of less than about 2 for about two hours followed by contacting an aqueous medium at a pH equal to or less than about 5.5 for at least 30 to 45 minutes. In a preferred embodiment, the enterically-coated dosage form releases less than about 5%, 2 % or 1% of the biguanide compound in an aqueous medium of 0.1 N HCl for two hours and less than about 5%, 2% or 1% when transferred to an aqueous medium at pH 5.5 for at least 30 to 45 minutes.

In additional embodiments, the enterically-coated dosage form releases less than 15%, 10%, or 5% of the biguanide compound during the lag phase after the dosage form is contacted with an aqueous medium at a pH of about 6.8, wherein the lag phase is at least ten, fifteen or twenty minutes. In a preferred embodiment, the enterically-coated dosage form releases less than about 15% of the biguanide compound when the dosage form is contacted with an aqueous medium at a pH of about 6.8 for a lag phase of at least ten minutes and releases greater than 90%, 95%, 98%, or 99% of the biguanide compound after contacting with an aqueous medium at a pH of about 6.8 for a total of ninety to 120 minutes.

Also provided herein is the pharmaceutical composition according to the invention for use in a method of treating metabolic disorders in a patient in need thereof. In some embodiments, the dosage form is adapted to release less than about 10%, 5%, 4%, 3%, 2% or 1% of the biguanide compound at a pH of less than 2 for at least two hours, followed by a pH equal to or less than about 5.5 for at least 30 to 45 minutes. In further embodiments, the dosage form is adapted to release less than 15%, 10%, or 5% of the biguanide compound when the dosage form is contacted with an aqueous medium at a pH of about 6.8 or less for at least about ten or fifteen minutes.

Also provided herein is the pharmaceutical composition according to the invention for use in a method of reducing the onset of diabetes in a subject with pre-diabetes. In some embodiments, the dosage form is adapted to release less than about 10%, 5%, 4%, 3%, 2% or 1% of the biguanide compound at a pH of less than 2 for at least two hours, followed by a pH equal to or less than about 5.5 for at least 30 to 45 minutes. In further embodiments, the dosage form is adapted to release less than 15%, 10%, or 5% of the biguanide compound when the dosage form is contacted with an aqueous medium at a pH of about 6.8 or less for at least about ten or fifteen minutes.

Also provided herein is the pharmaceutical composition according to the invention, for use in a method of inducing weight loss in a subject. In some embodiments, the dosage form is adapted to release less than about 10%, 5%, 4%, 3%, 2% or 1% of the biguanide compound at a pH of less than 2 for at least two hours, followed by a pH equal to or less than about 5.5 for at least 30 to 45 minutes. In preferred embodiments, the dosage form is adapted to release less than 15%, 10%, or 5% of the biguanide compound when the dosage form is contacted with an aqueous medium at a pH of about 6.8 or less for at least about ten or fifteen minutes.

In some embodiments, the weight loss induced clinically results in over 5 pounds lost in the subject, e.g., over 10 pounds lost, preferably over 25 pounds lost, and even more preferably over 50 pounds lost. In other embodiments, the induced weight loss results in the subject having a body mass index between 18.5 and 24.9. In another embodiment, the weight loss induced results in at least a loss of at least 0.5 inches in the waist circumference.

The compositions disclosed herein are also suitable for patients having a contraindication for the biguanide compound, e.g, metformin, phenformin or buformin. Such contraindication may be a hypoxic condition, impaired lactate clearance, and/or impaired clearance of the biguanide compound, e.g., impaired metformin clearance.

For example, in one embodiment, the pharmaceutical compositions disclosed herein may be for use in treating a patient who may have a hypoxic condition, such as but not limited to respiratory failure and heart failure. In another embodiment, the patient may have impaired lactate clearance. In another embodiment, the patient may suffer from liver failure, which may result in impaired lactate clearance. In another embodiment, the patient may have impaired clearance of the biguanide compound, which may be caused, e.g., by renal impairment and/or kidney disease. Accordingly, in one embodiment the patient may have renal impairment. Such renal impairment may be moderate or severe renal impairment, or endstage renal disease. In another embodiment, the patient may have kidney disease, which may be chronic. In another embodiment, the patient may have hyperglycemia, which may be chronic, and which may be caused by type II diabetes.

Also provided herein is the pharmaceutical composition according to the invention for use in a method of treating type I diabetes. In another aspect, the pharmaceutical composition according to the invention is provided for use in a method of treating dyslipidemia. Further provided is the pharmaceutical composition according to the invention for use in a method of treating obesity.

Suitable biguanide compounds for use in the subject invention include, *e.g.,* metformin, phenformin, buformin or imeglimin, including analogs, salts, solvates, polymorphs, hydrates, N-oxides, and prodrugs of such compounds.

In preferred embodiments, the biguanide compound has a reduced relative bioavailability of 70%, 60%, 50%, 40%, 30%, 20% or 10% in the subject formulations compared to a conventional immediate-release (IR) or extended-release (XR) composition having the same amount of the biguanide compound. Accordingly, in specific embodiments, administration of the subject delayed-release formulation minimizes the mean plasma AUC, the mean plasma Cₘₐₓ and/or the circulating plasma concentration of the biguanide compound in said patient compared to an identical protocol administering an IR or XR formulation having the same amount of the biguanide compound. In preferred embodiments, the biguanide compound is metformin, the IR composition is Glucophage^{®} and the XR composition is Glucophage^{®} XR.

In one embodiment, the mean plasma AUC₀₋₃₆ of the biguanide compound is less than about 15,000 ng*h/mL or 14,000 ng*h/mL, preferably less than about 12,000 ng*h/mL, more preferably less than about 11,000 ng*h/mL, and most preferably less than about 10,000 ng*h/mL when administered at 2000 mg total daily dose (TDD) or 1000 mg twice a day (*bis in die*; abbreviated as "b.i.d" or "BID"). In another embodiment, the mean plasma AUC₀₋₃₆ of the biguanide compound is less than about 10,000 ng*h/mL, preferably less than about 9,000 ng*h/mL, more preferably less than about 8,000 ng*h/mL or 7,000 ng*h/mL, and most preferably less than about 6,000 ng*h/mL or 5,000 ng*h/mL when administered at 1000 mg TDD, 500 mg BID, or lower effective doses.

In one embodiment, the mean plasma Cₘₐₓ of the biguanide compound is less than about 1100 ng/mL, preferably less than about 1000 ng/mL, more preferably less than about 950 ng/mL, and most preferably less than about 900 ng/mL when administered at 2000 mg TDD or 1000 mg BID. In another embodiment, the mean plasma Cₘₐₓ of the biguanide compound is less than about 800 ng/mL, preferably less than about 700 ng/mL, more preferably less than about 600 ng/mL, and most preferably less than about 600 ng/mL or 500 ng/mL when administered at 1000 mg TDD, 500 mg BID, or lower effective doses.

In one embodiment, the resulting circulating plasma concentration of the biguanide compound is below about 5 µg/ml or 4 µg/ml, preferably below about 3 µg/ml or 2.5 µg/ml, more preferably below about 2 µg/ml, 1 µg/ml, 0.5 µg/ml, or 0.25 µg/ml in the patient.

Administration of the subject formulations may be twice daily in the morning and evening, or once daily (*omni in die*, abbreviated "OD"). In certain preferred embodiments, administration may be once daily in the morning, e.g., before 1 pm, preferably before 12 noon or 11 am, more preferably before 10 or 9 am, or with the morning meal. In other preferred embodiments, administration may be once daily in the evening, e.g., after 5 pm, more preferably after 6 pm or 7 pm, or with the evening meal. In another preferred embodiment, administration may be once daily at bedtime.

The inventive compositions for use provided herein advantageously allow for lower therapeutic doses than prior art formulations, both on a per unit basis and/or on a daily dose basis. In certain embodiments of the compositions for use disclosed herein, the biguanide compound is administered twice daily in an oral dosage form at a per unit dose greater than 500 mg BID, *e.g.* 600 or 800 mg BID. In certain preferred embodiments of the compositions for use disclosed herein, the twice daily oral dosage is less than 500 mg BID, *e.g.,* less than 400 mg BID, *e.g.,* less than 300 mg BID, *e.g.,* about 150, 200 or 250 mg BID. In alternative preferred embodiments, the biguanide compound is to be administered once a day at a per unit dose of 75 mg OD, 125 mg OD, 250 mg OD, 300 mg OD, 500 mg OD, 600 mg OD, 750 mg OD, 800 mg OD or 1000 mg OD. In additional embodiments, the total daily dose of the biguanide compound is less than 2000 mg/day, preferably less than 1500 mg/day, more preferably less than 1000 or 750 mg/day, most preferably less than 500, 400, 300, or 200 mg/day.

In another embodiment, the oral dosage form may further comprise an extended-release formulation for the biguanide compound. In preferred embodiments, the biguanide compound is targeted for delivery to the distal small intestine.

In the compositions for use disclosed herein, the biguanide compound may be or comprise metformin, a metformin salt, solvate, polymorph, hydrate, N-oxide or prodrug. In preferred embodiments, the biguanide compound is a metformin salt selected from the group consisting of hydrochloride, phosphate, sulfate, hydrobromide, salicylate, maleate, hemi-maleate, benzoate, succinnate, hemi- succinnate, ethanesulfonate, fumarate, hemi-fumarate, glycolate, palmoate, oratate, acetate, isobutyrate, acetylsalicylate, nicotinateic acid, adamantoate, chlorophylin including zinc-chlorophylin, carboxylateic acid, benzoateic acid, dichloroacetateic acid, theophylin-7-acetate, clofibrate, tartrate, oxalate, hemi-oxalate, tannate and hydroxyl acid. In a particularly preferred embodiment, the biguanide compound is metformin hydrochloride.

The composition disclosed herein may also further comprise the administration of an immediate-release, extended release or delayed-release formulation of one or more additional therapeutic agents, *e.g.,* a DPP-IV inhibitor such as, *e.g.,* sitagliptin, saxagliptin, berberine, vildagliptin, linagliptin, alogliptin, and the like, a chemosensory receptor ligand (e.g., a sweet receptor ligand, bitter receptor ligand, umami receptor ligand, sour receptor ligand, fat receptor ligand or bile acid receptor ligand), an anti-obesity or anti-diabetes agent, or a chemosensory receptor antagonist, *e.g*., lactisole. Non-limiting examples include embodiments further comprising the administration of 100 mg sitagliptin OD, or 50 mg sitagliptin BID. The delayed-release formulation can be a bilayer tablet, or a capsule with the two components as encapsulated mini-tablets. The delayed-release formulation may also further comprise an immediate release component that has a pH 5.0 enteric coating for the additional therapeutic agent.

### BRIEF DESCRIPTION OF THE DRAWINGS

FIG. 1 shows the design of the study described in Example 1.
FIG. 2 shows the events during the treatment period of the study described in Example 1.
FIG. 3 shows the plasma concentration of metformin immediate-release (Metformin IR) (•) and metformin delayed-release (Metformin DR) (■) (x-axis; ng/mL) as a function of time (y-axis; min) after ingestion at t = -240 and after a meal at t = 0 min.

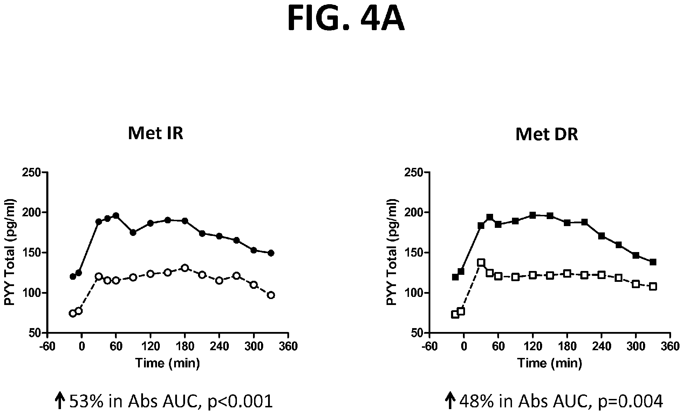

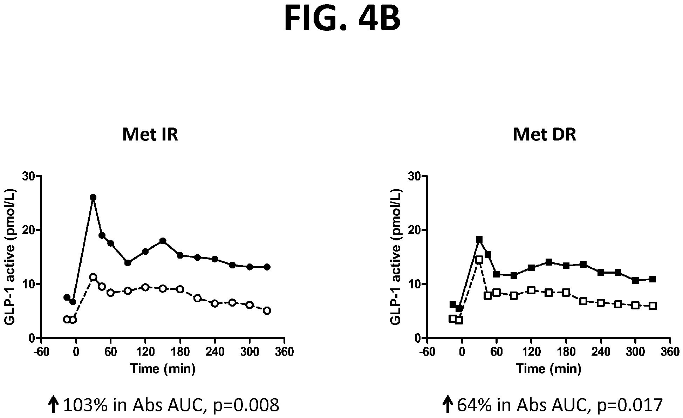

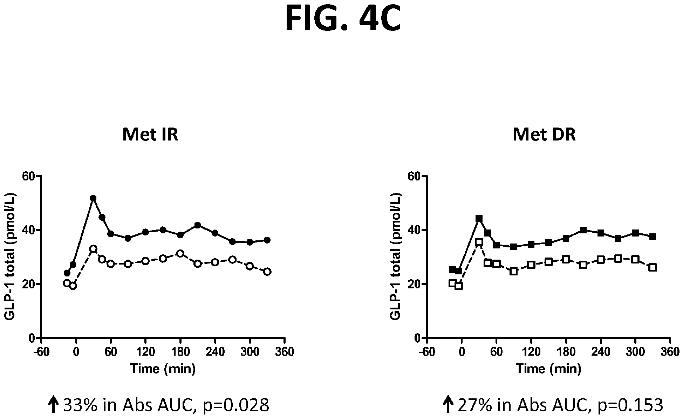

FIG. 4A shows the plasma concentration of PYY (x-axis; pg/mL) as a function of time (y-axis; min) in subjects at baseline (□,∘) or after ingestion of either Metformin IR (•) or Metformin DR (■) and after a meal at t = 0 min. FIG.4B shows the plasma concentration of active GLP-1(x-axis; GLP-1A pmol/L) as a function of time (y-axis; min) in subjects at baseline (□,∘) or after ingestion of either Metformin IR (•) or Metformin DR (■) and after a meal at t = 0 min. FIG. 4C shows the plasma concentration of total GLP-1 (x-axis; GLP-1T pmol/L) as a function of time (y-axis; min) in subjects at baseline (□,∘) or after ingestion of either Metformin IR (•) or Metformin DR (■) and after a meal at t = 0 min. For FIGs 4A-4C, percent increase in Abs AUC is compared to baseline values.
FIG. 5A shows the plasma concentration of glucose (x-axis; mg/dL) as a function of time (y-axis; min) in subjects at baseline (□,∘) or after ingestion of either Metformin IR (•) or Metformin DR (■) and after a meal at t = 0 min. FIG. 5B shows the plasma concentration of insulin (x-axis; pmol/L) as a function of time (y-axis; min) in subjects at baseline (□,∘) or after ingestion of either Metformin IR (•) or Metformin DR (■) and after a meal at t = 0 min. For FIGs 5A-5B, percent decrease in Abs AUC is compared to baseline values.
FIG. 6 is a graph that shows the area under the curve of PYY (x-axis; log transformed) as a function of the area under the curve of metformin (ng/mL*min) after ingestion of Metformin IR (•) and Metformin DR (■).
FIG. 7A shows the plasma concentration of Metformin IR (•) and Metformin DR (■) (x-axis; ng/mL) as a function of time (y-axis; min) after ingestion at t = -240 and after a meal at t = 0 min. FIG. 7B shows the plasma concentration of PYY (x-axis; pg/mL) as a function of time (y-axis; min) in subjects at baseline (□,∘) or after ingestion of either Metformin IR (•) or Metformin DR (■) and after a meal at t = 0 min.
FIG. 8 shows the mean plasma metformin concentrations (x-axis; ng/mL) at Day 5 of 500 mg (◆) and 1000 mg (■) Metformin DR, 1000 mg Metformin IR (∘) , and 500 mg Metformin IR + 1000 mg Metformin DR (A) as a function of time (y-axis; min). Dose was administered at t = -1 minute.
FIG. 9 shows the steady-state relative bioavailability in subjects with type 2 diabetes of 500 mg BID and 1000 mg BID of Metformin DR compared to 1000 mg BID of Metformin IR based on the 11 hour plasma metformin AUC on Day 5 (y-axis; % AUC₍₀₋₁₁ₕᵣ₎ ). These levels constitute a 45% and 57% reduction in the overall plasma metformin extent of exposure for 500 mg BID and 1000 mg BID of Metformin DR compared to 1000 mg BID of Metformin IR.
FIG. 10 shows the mean plasma PYY total concentrations (x-axis; pg/mL) as a function of time (y-axis; min) in subjects at baseline (∘) or Day 5 of the designated treatment (•).
FIG. 11 shows the mean plasma GLP-1 active concentration (x-axis; pmol/L) as a function of time (y-axis; min) in subjects at baseline (∘) or Day 5 of the designated treatment (•). Breakfast was administered at t = 0 min, dose was administered at t = -1 minute, and lunch was administered at t = 300 min.
FIG. 12 shows the mean plasma glucose concentration (x-axis; mg/dL) as a function of time (y-axis; min) in subjects at baseline (∘) or Day 5 of the designated treatment (•).
FIG. 13 shows the individual change in fasting plasma glucose concentrations (x-axis; mg/dL) as a function of time (y-axis; min) from baseline to Day 5 by scatterplot in subjects treated with 500 mg (◆) and 1000 mg (■) Metformin DR, 1000 mg Metformin IR (•), and 500 mg Metformin IR + 1000 mg Metformin DR (A)(y-axis) The line in the panel marks the LS Mean Change in glucose (mg/dL) for each treatment.
FIG. 14 shows the mean plasma metformin concentration (x-axis; ng/mL) of 500 mg (◆) and 1000 mg (■) Metformin DR, 1000 mg Metformin IR (∘) , and 2000 mg metformin extended release (Metformin XR) a function of time (y-axis; hours). Dose was administered at t = 0 hours. Second dose was administered for BID regimens at t=12 hours. Meals/snacks were provided at t= -.42, 2.08, 11.5, 18 and 24 hours.
FIG. 15 shows the Cₘₐₓ (left panel) and AUC₀₋₃₆ (right panel) of one day's dosing of 1000 mg BID metformin IR, 500 mg BID and 1000 mg BID of Metformin DR and 2000 mg QD metformin XR. The * signifies a statistically significant reduction in exposure compared to both metformin IR and metformin XR (all p< 0.0001)
FIG. 16 shows the relative bioavailability of one day's dosing of 500 and 1000 mg BID Metformin DR compared to 1000 mg BID Metformin IR (left panel) and the relative bioavailability of one day's dosing of 500 and 1000 mg BID Metformin DR compared to 2000 mg QD Metformin XR (right panel)
FIG. 17 shows individual patient pharmacokinetic profiles on Day 5 of dosing with the anticipated delay in metformin release following a dose of Metformin DR at t=-240 shown in the left panel and no delay in release shown in the right panel.
FIG. 18 shows the dissolution performance of exemplary delayed-release formulations having 2.4% (Batch # K111511-89A) and 3.8% (Batch # K260512-127) nominal enteric coatings.
FIG. 19 shows the two-stage dissolution performance of exemplary delayed-release formulations having 3.0% to 3.9% nominal enteric coatings. The first stage is 2 hours at pH < 2, and the second stage is 2 hours at pH 6.8.
FIG. 20 shows the three-stage dissolution performance of exemplary delayed-release formulations having 3.0% to 3.9% nominal enteric coatings. The first stage is 2 hours at pH < 2, and the second stage is 1 hour at pH 5.5, and the third stage is 3 hours at pH 6.8.

### DETAILED DESCRIPTION

Contemplated herein are compositions that consistently minimize the systemic bioavailability of biguanide compounds, such as metformin, in subjects yet still provide significant salutary metabolic effects, e.g. reducing hyperglycemia. Contrary to conventional understanding (*see, e.g.* Mulherin *et al,*, *supra*), the biguanide compounds of the disclosure actually cause release of GLP-1 through a mechanism of action which may include interaction with the luminal or epithelial aspect (*i.e.,* the gastrointestinal tract side) of enteroendocrine cells, and systemic bioavailability can therefore be minimized while still achieving meaningful therapeutic efficacy. Advantageously, the subject compositions significantly improve the pharmacokinetics of drug release and also dramatically reduce the possibility of adverse effects such as lactic acidosis and gastrointestinal complications.

Accordingly, a delayed-release pharmaceutical composition according to the invention for use in a method of reducing the risk of adverse events from biguanide administration is provided Also provided herein is a delayed-release pharmaceutical composition according to the invention for use in a method of treating metabolic disorders in subjects, including in subjects having a contraindication for biguanide compound(s). In preferred embodiments, the biguanide compound is selected from the group consisting of metformin, buformin, phenformin and imeglimin, and is to be administered at lower doses and/or with lower bioavailability than currently indicated while still achieving the desired metabolic improvements.

### Definitions

The terms "gastrointestinal tract" and "gut," as used herein, refer to the stomach and intestine. The "small" or "upper" intestine includes the duodenum, jejunum and ileum and the "large" or "lower" intestine includes the caecum, colon and rectum. The "distal" small intestine includes the jejunum and ileum.

As used herein, the term "lag phase" as applied to biguanide release from the subject formulations refers to the time period beginning when the enterically coated formulation first contacts the pH at which the enteric coating is designed to dissolve and during which there is a minimal initial rate of release, e.g. less than about 20% or 15%, more preferably less than about 10% or 5%, still more preferably less than about 3%, 2% or 1% for at least the first 5, 10, 15, or 20 minutes at the desired pH.

"Treating" or "treatment" of any condition, disease or disorder refers, in some embodiments, to ameliorating the disease, disorder, or condition (i.e., arresting or reducing the development of the disease, disorder, or condition, or at least one of the clinical symptoms thereof). In other embodiments "treating" or "treatment" refers to ameliorating at least one physical parameter, which may or may not be discernible by the subject, including physical parameters that are undesired but not clinically significant. In yet other embodiments, "treating" or "treatment" refers to inhibiting the disease, disorder, or condition, either physically, (e.g., stabilization of a discernible symptom), physiologically, (e.g., stabilization of a physical parameter) or both. In yet other embodiments, "treating" or "treatment" refers to preventing or to delaying the onset of the disease, disorder, or condition.

"Therapeutically effective amount" or "effective amount" means the amount of a composition, compound, therapy, or course of treatment that, when administered to a subject for treating a disease, disorder, or condition, is sufficient to effect such treatment for the disease, disorder, or condition. The "therapeutically effective amount" will vary depending on the composition, the compound, the therapy, the course of treatment, the disease, disorder, or condition, and its severity and the age, weight, etc., of the subject to be treated.

As used herein, the term "hyperglycemic" or "hyperglycemia," when used in reference to a condition of a patient, means a transient or chronic abnormally high level of glucose present in the blood of a patient. The condition can be caused by a delay in glucose metabolism or absorption such that the patient exhibits glucose intolerance or a state of elevated glucose not typically found in normal patients (e.g., in glucose-intolerant subdiabetic patients at risk of developing diabetes, or in diabetic patients). Fasting plasma glucose (FPG) levels for normoglycemia are less than about 110 mg/dl, for impaired glucose metabolism, between about 110 and 126 mg/dl, and for diabetics greater than about 126 mg/dl.

When the biguanide compounds described herein include one or more chiral centers, the stereochemistry of such chiral centers can independently be in the R or S configuration, or a mixture of the two. The chiral centers can be further designated as R or S or R,S or d,D, 1,L or d,l, D,L. Correspondingly, the biguanide compounds of the invention, if they can be present in optically active form, can actually be present in the form of a racemic mixture of enantiomers, or in the form of either of the separate enantiomers in substantially isolated and purified form, or as a mixture comprising any relative proportions of the enantiomers.

When the biguanide compounds described herein contain two or more chiral centers then diastereomers are possible. Such diastereomers may be present as pure diastereomeric enantiomers, pure racemic mixtures of diastereomeric enantiomers, mixtures of diastereomers which may be racemic or may have optical activity in their own right due to complex permutations of enantiomeric diastereomers in the balance of the mixtures.

When the biguanide compounds of the invention, if they can be present in geometrically isomeric forms around, for example, the guanide bond, then they can actually be present in the form of a mixture of geometric isomers comprising any relative proportions of the isomers, or in some cases in the form of either of the separate geometric isomers in substantially isolated and purified form.

When the biguanide compounds described herein include one or more isolated or linearly conjugated double bonds, the geometry around such double bonds can be independently a cis/trans, E/Z mixture or an E or Z geometric isomer thereof.

"Alkyl" means a straight or branched chain, saturated monovalent hydrocarbon radical. By way of example, the hydrocarbon chain may have from one to twenty carbons, one to sixteen carbons, one to fourteen carbons, one to twelve carbons, one to ten carbons, one to eight carbons, one to six carbons, one to four carbons, etc. "Lower alkyl" may refer to alkyls having, e.g., one to six carbons, one to four carbons, etc. In certain examples, a straight chain alkyl may have from one to six carbon atoms and a branched alkyl three to six carbon atoms, e.g., methyl, ethyl, propyl, 2-propyl, butyl (including all isomeric forms), pentyl (including all isomeric forms), and the like. "Me" means methyl, "Et" means ethyl, and "iPr" means isopropyl.

"Aryl" means a monovalent monocyclic or bicyclic aromatic hydrocarbon radical, e.g., having from of 6 to 20 or 6 to 10 ring atoms e.g., phenyl or naphthyl.

"Alkylaryl" means a (alkylene)-R radical where R is aryl as defined above.

"Cycloalkyl" means a cyclic saturated or partially saturated monovalent hydrocarbon radical (or an alicyclic radical). By way of example, the cycloalkyl may have from three to twenty carbon atoms, from three to sixteen carbon atoms, from three to fourteen carbon atoms, from three to twelve carbon atoms, from three to ten carbon atoms, from three to eight carbon atoms, from three to six carbon atoms, etc., wherein one or two carbon atoms may be replaced by an oxo group, e.g., admantanyl, cyclopropyl, cyclobutyl, cyclopentyl, cyclohexyl, cyclohexenyl, indanyl and the like.

"Alkylcycloalkyl" means a (alkylene)-R radical where R is cycloalkyl as defined above; e.g., cyclopropylmethyl, cyclobutylmethyl, cyclopentylethyl, or cyclohexylmethyl, and the like.

"Heterocyclyl" or "heterocycloalkyl" means a saturated or unsaturated monovalent monocyclic group, in which one or two ring atoms are heteroatom selected from N, O, or S, the remaining ring atoms being C. The heterocyclyl ring is optionally fused to a (one) aryl or heteroaryl ring as defined herein. The heterocyclyl ring fused to monocyclic aryl or heteroaryl ring is also referred to in this Application as "bicyclic heterocyclyl" ring. Additionally, one or two ring carbon atoms in the heterocyclyl ring can optionally be replaced by a -CO- group. More specifically the term heterocyclyl includes, but is not limited to, pyrrolidino, piperidino, homopiperidino, 2-oxopyrrolidinyl, 2-oxopiperidinyl, morpholino, piperazino, tetrahydropyranyl, thiomorpholino, and the like. When the heterocyclyl ring is unsaturated it can contain one or two ring double bonds. When the heterocyclyl group contains at least one nitrogen atom, it is also referred to herein as heterocycloamino and is a subset of the heterocyclyl group. When the heterocyclyl group is a saturated ring and is not fused to aryl or heteroaryl ring as stated above, it is also referred to herein as saturated monocyclic heterocyclyl.

"Alkylheterocycloalkyl" means a -(alkylene)-R radical where R is heterocyclyl ring as defined above e.g., tetraydrofuranylmethyl, piperazinylmethyl, morpholinylethyl, and the like.

"Heteroaryl" means a monovalent monocyclic or bicyclic aromatic radical, where one or more, preferably one, two, or three, ring atoms are heteroatom selected from N, O, or S, the remaining ring atoms being carbon. Representative examples include, but are not limited to, pyrrolyl, thienyl, thiazolyl, imidazolyl, furanyl, indolyl, isoindolyl, oxazolyl, isoxazolyl, diazolyl, pyrazolyl, triazolyl, benzothiazolyl, benzoxazolyl, quinolinyl, isoquinolinyl, pyridinyl, pyrimidinyl, pyrazinyl, pyridazinyl, tetrazolyl, and the like.

"Oxo" or "carbonyl" means =(O) group or C=O group, respectively.

The term "substituted" means that the referenced group is substituted with one or more additional group(s) individually and independently selected from groups described herein. In some embodiments, an optional substituent is selected from oxo, halogen, -CN, -NH₂, -OH, - NH(CH₃), -N(CH₃)₂, alkyl (including straight chain, branched and/or unsaturated alkyl), substituted or unsubstituted cycloalkyl, substituted or unsubstituted heterocycloalkyl, fluoroalkyl, substituted or unsubstituted heteroalkyl, substituted or unsubstituted alkoxy, fluoroalkoxy, -S-alkyl, -S(O)₂-alkyl, -CONH((substituted or unsubstituted alkyl) or (substituted or unsubstituted phenyl)), -CON(H or alkyl)₂, -OCON(substituted or unsubstituted alkyl)₂, - NHCONH((substituted or unsubstituted alkyl) or (substituted or unsubstituted phenyl)), - NHCOalkyl, -N(substituted or unsubstituted alkyl)CO(substituted or unsubstituted alkyl), - NHCOO(substituted or unsubstituted alkyl), -C(OH)( substituted or unsubstituted alkyl)₂, and - C(NH₂)(substituted or unsubstituted alkyl)₂. In some embodiments, by way of example, an optional substituent is selected from oxo, fluorine, chlorine, bromine, iodine, -CN, -NH₂, -OH, - NH(CH₃), -N(CH₃)₂, -CH₃, - CH₂CH₃, -CH(CH₃)₂, -CF₃, -CH₂CF₃, -OCH₃, -OCH₂CH₃, - OCH(CH₃)₂, -OCF₃, - OCH₂CF₃, - S(O)₂-CH₃, -CONH₂, -CONHCH₃, -NHCONHCH₃, -COCH₃, -COOH and the like. In some embodiments, substituted groups are substituted with one, two or three of the preceding groups. In some embodiments, substituted groups are substituted with one or two of the preceding groups. In some embodiments, substituted groups are substituted with one of the preceding groups. Further, unless stated to the contrary, a formula with chemical bonds shown only as solid lines and not as wedges or dashed lines contemplates each possible isomer, e.g., each enantiomer and diastereomer, and a mixture of isomers, such as racemic or scalemic mixtures.

In some embodiments, a biguanide compound of the disclosure is present in a composition as a salt. In some embodiments, salts are obtained by reacting a compound of the disclosure with acids. In some other embodiments, pharmaceutically acceptable salts are obtained by reacting a compound of the disclosure with a base. In other embodiments, the compounds are used as free-acid or free-base form in the manufacture of the compositions described herein. The type of salts, include, but are not limited to: (1) acid addition salts, formed by reacting the free base form of the compound with a pharmaceutically acceptable: inorganic acid, such as, for example, hydrochloric acid, hydrobromic acid, sulfuric acid, phosphoric acid, metaphosphoric acid, and the like; or with an organic acid, such as, for example, acetic acid, propionic acid, hexanoic acid, cyclopentanepropionic acid, glycolic acid, pyruvic acid, lactic acid, malonic acid, succinic acid, malic acid, maleic acid, fumaric acid, trifluoroacetic acid, tartaric acid, citric acid, benzoic acid, 3- (4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, toluenesulfonic acid, 2-naphthalenesulfonic acid, 4-methylbicyclo-[2.2.2]oct-2-ene-l-carboxylic acid, glucoheptonic acid, 4,4'-methylenebis-(3-hydroxy-2-ene-l-carboxylic acid), 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, butyric acid, phenylacetic acid, phenylbutyric acid, valproic acid, and the like; (2) salts formed when an acidic proton present in the parent compound is replaced by a metal ion, e.g., an alkali metal ion (e.g. lithium, sodium, potassium), an alkaline earth ion (e.g. magnesium, or calcium), or an aluminum ion. In some cases, the biguanide compound described herein are reacted with an organic base, such as, but not limited to, ethanolamine, diethanolamine, triethanolamine, tromethamine, N-methylglucamine, dicyclohexylamine, tris(hydroxymethyl)methylamine. In other cases, the compounds described herein form salts with amino acids such as, but not limited to, arginine, lysine, and the like. Acceptable inorganic bases used to form salts with compounds that include an acidic proton, include, but are not limited to, aluminum hydroxide, calcium hydroxide, potassium hydroxide, sodium carbonate, sodium hydroxide, and the like.

The term "amino acid" includes any one of the twenty naturally-occurring amino acids or the D-form of any one of the naturally-occurring amino acids. In addition, the term "amino acid" also includes other non-naturally occurring amino acids besides the D-amino acids, which are functional equivalents of the naturally-occurring amino acids. Such non-naturally-occurring amino acids include, for example, norleucine ("Nle"), norvaline ("Nva"), L- or D-naphthalanine, ornithine ("Orn"), homoarginine (homoArg) and others well known in the peptide art, such as those described in M. Bodanzsky, "Principles of Peptide Synthesis," 1st and 2nd Revised Ed., Springer-Verlag, New York, N.Y., 1984 and 1993, and Stewart and Young, "Solid Phase Peptide Synthesis," 2nd Ed., Pierce Chemical Co., Rockford, Ill., 1984.

Amino acids and amino acid analogs can be purchased commercially (Sigma Chemical Co.; Advanced Chemtech) or synthesized using methods known in the art.

In the scope of the embodiments, the biguanide compounds described herein include further forms of the compounds such as pharmaceutically acceptable salts, solvates (including hydrates), amorphous phases, partially crystalline and crystalline forms (including all polymorphs), prodrugs, metabolites, N-oxides, isotopically-labeled, epimers, pure epimers, epimer mixtures, enantiomers including but not limited to single enantiomers and enantiomeric diastereomers, meso compounds, stereoisomers, racemic mixtures and diasteroisomeric mixtures. Biguanide compounds described herein having one or more double bonds include cis/trans isomers, E/Z isomers and geometric isomers. Biguanide compounds described herein can be prepared as a pharmaceutically acceptable salts formed when an acidic proton present in the parent compound either is replaced by a metal ion, for example an alkali metal ion, an alkaline earth ion, or an aluminum ion; or coordinates with an organic base. In addition, the salt forms of the disclosed compounds can be prepared using salts of the starting materials or intermediates.

In some embodiments, the biguanide compounds described herein include solvent addition forms or crystal forms thereof, particularly solvates or polymorphs. Solvates contain either stoichiometric or non-stoichiometric amounts of a solvent, and may be formed during the process of crystallization with pharmaceutically acceptable solvents such as water, ethanol, and the like. Hydrates are formed when the solvent is water, or alcoholates are formed when the solvent is alcohol.

As noted above, in some embodiments the biguanide compounds described herein possess one or more stereocenters and each center exists independently in either the R or S configuration. The biguanide compounds presented herein include all diastereomeric, enantiomeric, and epimeric forms as well as the appropriate mixtures thereof.

In some embodiments, sites on the biguanide compounds disclosed herein are susceptible to various metabolic reactions. Therefore incorporation of appropriate substituents at the places of metabolic reactions will reduce, minimize or eliminate the metabolic pathways. In specific embodiments, the appropriate substituent to decrease or eliminate the susceptibility of the aromatic ring to metabolic reactions is, by way of example only, a halogen, deuterium or an alkyl group.

In some embodiments, the biguanide compounds described herein are isotopically-labeled, which are identical to those recited in the various formulae and structures presented herein, but for the fact that one or more atoms are replaced by an atom having an atomic mass or mass number different from the atomic mass or mass number usually found in nature. In some embodiments, one or more hydrogen atoms are replaced with deuterium. In some embodiments, metabolic sites on the compounds described herein are deuterated. In some embodiments, substitution with deuterium affords certain therapeutic advantages resulting from greater metabolic stability, such as, for example, increased in vivo half-life or reduced dosage requirements. Throughout the specification, groups and substituents thereof can be chosen by one skilled in the field to provide stable moieties and compounds.

### Biguanides

The compositions and uses disclosed herein relate to metformin and other biguanides. By way of background, metformin is one of the simplest structural variants of a class of compounds known as the biguanides. From a structural perspective metformin resembles a pharmacophore or fragment of a larger biologically active chemical structure.

In one embodiment, the biguanide compounds of the subject invention include the following: 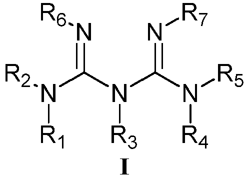 wherein:
R₁, R₂, R₃, R₄, R₅, R₆, and R₇ are independently selected from:
H, OH,
O-Rx, wherein Rx is alkyl, cycloalkyl, alkylcycloalkyl, acyl, ester, thioester;
optionally substituted alkyl (e.g., a C₁ to C₁₂ straight chain or branched chain alkyl optionally substituted with oxygen, silicon, sulphur or optionally substituted with OH, O-alkyl, SH, S-alkyl, NH₂, NH-alkyl); cycloalkyl (e.g., C₃ to C₇ cycloalkyl); alkylcycloalkyl (e.g., C₄ to C₁₂ alkylcycloalkyl); heterocycloalkyl (e.g., where the heterocycle comprises one or two hetero atoms selected from O, S, or N, including a C₂ to C₆ heterocycloalkyl); alkylheterocycloalkyl (e.g., where the heterocycle comprises one or two hetero atoms selected from O, S, or N, including a C₃ to C₁₁ alkylheterocycloalkyl, and including wherein when N is present in the heterocyclic ring, the nitrogen atom may be in the form of an amide, carbamate or urea); optionally substituted alkenyl (e.g., C₁ to C₁₂ straight chain or branched chain alkenyl optionally substituted with oxygen, silicon, sulphur or optionally substituted with OH, O-alkyl, SH, S-alkyl, NH₂, NH-alkyl); optionally substituted alkynyl (e.g., C₁ to C₁₂ straight chain or branched chain alkynyl optionally substituted with oxygen, silicon, sulphur or optionally substituted with OH, O-alkyl, SH, S-alkyl, NH₂, NH-alkyl);
optionally substituted aryl (e.g., phenyl, substituted phenyl, naphthyl, substituted naphthyl); optionally substituted alkylaryl (e.g., alkylphenyl, alkylsubstituted phenyl, alkylnaphthyl, alkylsubstituted naphthyl); optionally substituted heteroaryl (e.g., pyridyl, furanyl, thiophenyl, pyrrollyl, oxazolyl, isoxazolyl, thiazolyl, diazolyl, pyrazolyl, triazolyl all of which are optionally substituted); optionally substituted alkylheteroaryl; and
or R₆ and R₇ may join to form a bond, together forming a ring including the nitrogen atoms to which they are attached;
or R₁ and R₂ may together form a 3 to 8 membered heterocyclic ring, including the nitrogen atoms to which they are attached;
or R₄ and R₅ may together form a ring selected from the group aziridine, pyrrolyl, imidazolyl, pyrazolyl, indolyl, indolinyl, pyrrolidinyl, piperazinyl and piperidyl, including the nitrogen atoms to which they are attached.

In certain embodiments, O-Rx may be selected from: O-C₁ to C₈ straight chain or branched chain alkyl; O-C₃ to C₇ cycloalkyl; O-C₄ to C₈ alkylcycloalkyl; O-acyl; O-esters; and O-thioesters.

In other embodiments, optional substitutions may include, e.g., OH, O-alkyl, SH, S-alkyl, NH₂, NH-alkyl. Further, an alkyl, alkenyl, alkynyl, etc. may be substituted with an oxygen, silicon, sulphur, etc. to form a heteroalkyl, heteroalkenyl, heteroalkynyl, etc.

In certain embodiments, each of: R₃, R₆, and R₇, or R₃, R₄, R₅, and R₇,or R₃, R₄, R₅, and R₇, or R₃, R₄, R₅, R₆ and R₇, or R₂, R₃, R₄, R₅, R₆ and R₇ are independently selected from:
H, methyl, ethyl, propyl or isopropyl;
and each of the remaining substituent groups: R₁, R₂, R₄, and R₅, or R₁, R₂, and R₆, or R₁, R₂, and R₆, or R₁ and R₂, or R₁, respectively, are independently selected from:
H; optionally substituted alkyl (e.g., C₁ to C₁₂ straight chain or branched chain alkyl optionally hetero substituted with oxygen, silicon, sulphur or optionally substituted with OH, O-alkyl, SH, S-alkyl, NH₂, NH-alkyl); optionally substituted alkenyl (e.g., C₁ to C₁₂ straight chain or branched chain alkenyl optionally hetero substituted with oxygen, silicon, sulphur or optionally substituted with OH, O-alkyl, SH, S-alkyl, NH₂, NH-alkyl); optionally substituted alkynyl (e.g., C₁ to C₁₂ straight chain or branched chain alkynyl optionally hetero substituted with oxygen, silicon, sulphur or optionally substituted with OH, O-alkyl, SH, S-alkyl, NH₂, NH-alkyl); cycloalkyl (e.g., C₃ to C₇ cycloalkyl); alkylcycloalkyl (e.g., C₄ to C₁₂ alkylcycloalkyl); heterocycloalkyl (e.g., where the heterocycle comprises one or two hetero atoms selected from O, S, or N, including C₂ to C₆ heterocycloalkyl); alkylheterocycloalkyl (e.g., where the heterocycle comprises one or two hetero atoms selected from O, S, or N, including C₃ to C₁₁ alkylheterocycloalkyl, and including wherein when N is present in the heterocyclic ring, the nitrogen atom may be in the form of an amide, carbamate or urea); aryl (e.g., phenyl, substituted phenyl, naphthyl, substituted naphthyl); alkylaryl (e.g., alkylphenyl, alkylsubstituted phenyl, alkylnaphthyl, alkylsubstituted naphthyl); heteroaryl (e.g., pyridyl, furanyl, thiophenyl, pyrrollyl, oxazolyl, isoxazolyl, thiazolyl, diazolyl, pyrazolyl, triazolyl all of which are optionally substituted); alkylheteroaryl;
or R₁ and R₂ may together form a 3 to 8 membered heterocyclic ring, including the nitrogen atoms to which they are attached;
or R₄ and R₅ may together form a ring selected from the group aziridine, pyrrolyl, imidazolyl, pyrazolyl, indolyl, indolinyl, pyrrolidinyl, piperazinyl and piperidyl, including the nitrogen atoms to which they are attached.

Exemplary compounds and substituents of R₁, R₂, R₃, R₄, R₅, R₆, and R₇ of Formula I are shown below. Additional combinations of selections of substituents of R₁, R₂, R₃, R₄, R₅, R₆, and R₇ are envisioned and disclosed in co-pending U.S. Patent Application Serial No. 13/547,022.

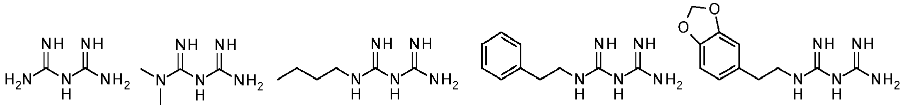

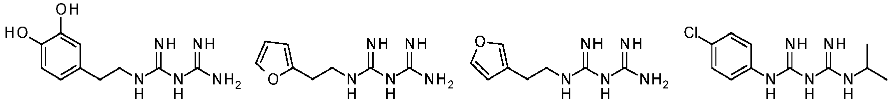

In certain embodiments, the biguanide compounds of Formula I may include an asymmetric center or centers, and may be in the form of a composition of a racemic mixture, a diastereoisomeric mixture, a single enantiomer, an enantiomeric diastereomer, a meso compound, a pure epimer, or a mixture of epimers thereof, etc. Further, the biguanide compounds may have one or more double bonds, and may be in a form of a cis/trans, E/Z mixture or an E or Z geometric isomer thereof.

The biguanide compounds of Formula I may also be prepared as a salt form, e.g., pharmaceutically acceptable salts, including suitable acid forms, e.g., salt forms selected from hydrochloride, hydrobromide, acetate, propionate, butyrate, sulphate, hydrogen sulphate, sulphite, carbonate, hydrogen carbonate, phosphate, phosphinate, oxalate, hemi-oxalate, malonate, hemi-malonate, fumarate, hemi-fumarate, maleate, hemi-maleate, citrate, hemi-citrate, tartrate, hemi-tartrate, aspartate, glutamate, etc.

Alternative embodiments of biguanide compounds specifically contemplated for use in the subject invention include the related heterocyclic compounds described in co-pending U.S. Patent Application Serial No. 13/547,022. The phrase "biguanide compound" as used herein includes these related heterocyclic compounds, exemplary embodiments of which include the following:

### Triazoles:

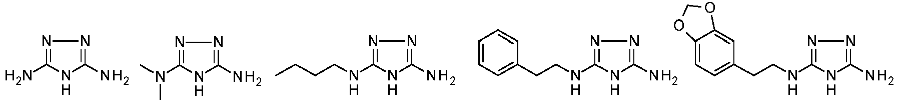

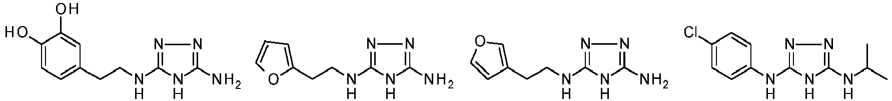

### Triazines:

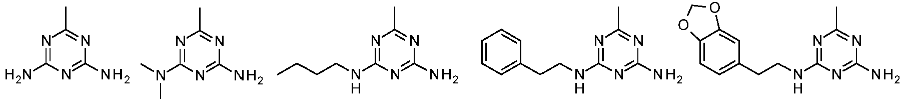

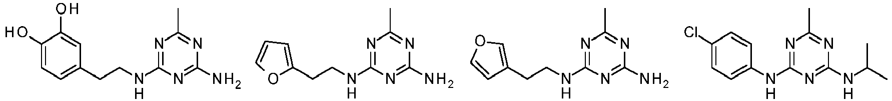

### Dihydrotriazines:

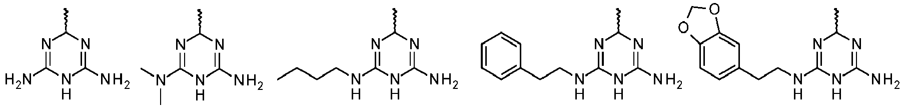

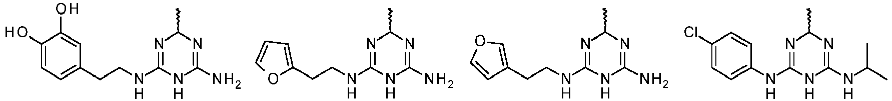

### 7-ring cyclic biguanides:

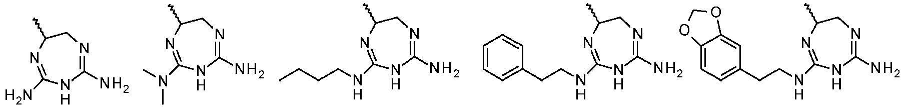

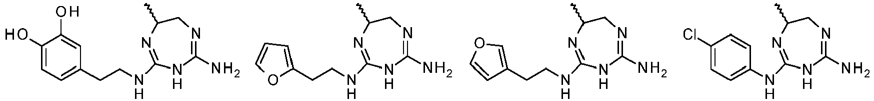

In one embodiment, the compounds of the disclosure may be prepared as a three component salt form including the components A, B, and C wherein:
A is the protonated form of a natural or unnatural amino acid;
B is the dianion of an acid; and
C is the protonated form of a Compound of Formula I.

In certain aspects, stoichiometric amounts of A, B, and C may be included wherein:
A is the protonated form of a natural amino acid selected from alanine, aspartic acid, asparagine, arginine, glycine, glutamine, glutamic acid lysine, phenylalanine, tyrosine, serine, threonine, tryptophan, leucine, isoleucine, histidine, methionine, proline, cysteine, or cystine;
B is the dianion of an acid selected from oxalic, malonic, citric, maleic, fumaric, tartaric, aspartic, glutamic acids and the like; and
C is the protonated form of a compound of Formula I.

### Contraindications for biguanide compounds, including metformin

Since systemic biguanides, including metformin are reported to be substantially excreted by the kidney, the risk of the biguanide compound accumulation and lactic acidosis increases with the degree of impairment of renal function. Other contraindications for biguanide compounds such as metformin include impaired lactate clearance, and a hypoxic condition. Accordingly, patients having these contraindications are not currently treatable with conventional biguanide compounds.

However, as demonstrated herein, the therapeutic efficacy of metformin and other biguanide compounds does not require an increase in the systemic level of the metformin that presents an increased risk of lactic acidosis. As such, the risk of metformin accumulation and lactic acidosis is dramatically lower, and the delayed-release pharmaceutical composition provided herein can therefore be used for treating a condition in a patient in need thereof, even where the patient has a contraindication for metformin. For example, the delayed-release pharmaceutical composition provided herein may be used to treat a patient in need thereof, wherein the patient has a hypoxic condition (e.g., respiratory failure and/or heart failure), impaired lactate clearance (e.g., due to liver failure), impaired metformin clearance, and/or renal impairment, which may be moderate, severe, or endstage impairment, and may be the result of chronic kidney disease.

### Metabolic Disorders

The compositions of the present invention find advantageous use in the treatment and/or prophylaxis of metabolic disorders, including being overweight, obesity, prediabetes, Polycystic Ovary Syndrome, dislipidemia or disorders of lipid metabolism, as well as hyperglycemic conditions, such as insulin-dependent (type 1) or -independent (type 2) diabetes, as well as physiological conditions or disorders associated with or that result from the hyperglycemic condition. Thus, hyperglycemic conditions treatable by a delayed-release pharmaceutical composition for use of the invention also include a histopathological change associated with chronic or acute hyperglycemia (e.g., diabetes). Particular examples include degeneration of pancreas (β-cell destruction), kidney tubule calcification, degeneration of liver, eye damage (diabetic retinopathy), diabetic foot, ulcerations in mucosa such as mouth and gums, excess bleeding, delayed blood coagulation or wound healing and increased risk of coronary heart disease, stroke, peripheral vascular disease, dyslipidemia, hypertension and obesity.

As used herein, the term "hyperglycemic" or "hyperglycemia," when used in reference to a condition of a patient, means a transient or chronic abnormally high level of glucose present in the blood of a patient. The condition can be caused by a delay in glucose metabolism or absorption such that the patient exhibits glucose intolerance or a state of elevated glucose not typically found in normal patients (e.g., in glucose-intolerant subdiabetic patients at risk of developing diabetes, or in diabetic patients). Fasting plasma glucose (FPG) levels for normoglycemia are less than about 110 mg/dl, for impaired glucose metabolism, between about 110 and 126 mg/dl, and for diabetics greater than about 126 mg/dl.

Metabolic disorders also include obesity or an undesirable body mass. Leptin, cholecystokinin, PYY and GLP-1 decrease hunger, increase energy expenditure, induce weight loss or provide normal glucose homeostasis. Thus, in various embodiments, a delayed-release pharmaceutical composition for use of the invention in treating obesity or an undesirable body mass, or hyperglycemia, involves the local administration of metformin to activate enteroendocrine cell production of cholecystokinin, oxyntomodulin, GIP, GLP-2, PYY or GLP-1. Disorders treatable also include those typically associated with obesity, for example, abnormally elevated serum/plasma LDL, VLDL, triglycerides, cholesterol, plaque formation leading to narrowing or blockage of blood vessels, increased risk of hypertension/stroke, coronary heart disease, etc.

### Synthesis of the Compounds

Compounds described herein may be synthesized using standard synthetic techniques known to those of skill in the art or using methods known in the art in combination with methods described herein. In additions, solvents, temperatures and other reaction conditions presented herein may vary according to the practice and knowledge of those of skill in the art.

The starting material used for the synthesis of compounds described herein can be obtained from commercial sources, such as Aldrich Chemical Co. (Milwaukee, Wis.), Sigma Chemical Co. (St. Louis, Mo.), or the starting materials can be synthesized. The compounds described herein, and other related compounds having different substituents can be synthesized using techniques and materials known to those of skill in the art, such as described, for example, in March, ADVANCED ORGANIC CHEMISTRY 4th Ed., (Wiley 1992); Carey and Sundberg, ADVANCED ORGANIC CHEMISTRY 4th Ed., Vols. A and B (Plenum 2000, 2001), and Green and Wuts, PROTECTIVE GROUPS IN ORGANIC SYNTHESIS 3rd Ed., (Wiley 1999). General methods for the preparation of the compounds as disclosed herein may be derived from known reactions in the field, and the reactions may be modified by the use of appropriate reagents and conditions, as would be recognized by the skilled person, for the introduction of the various moieties found in the formulae as provided herein.

Additional biguanide synthesis methods and schemes for the compounds described herein can be found in U.S. Application Ser. No. 12/593,479 (published as U.S. 2010/0130498); U.S. Application Ser. No. 12/593,398 (published as U.S. 2010/0184796); U.S. Pat. No. 7,829,299; U.S. Application Ser. No. 11/578,013 (published as U.S. 2010/0056621); U.S. Pat. No. 7,416,867; U.S. Application Ser. No. 11/455,693 (published as U.S. 2007/0037212); U.S. Application Ser. No. 13/059,730 (published as U.S. 2011/0143376), U.S. Application Ser. No. 12/996,670 (published as U.S. 2011/0311991), U.S. Pat. No. 7,811,788; U.S. Application Ser. No. 11/182,942 (published as U.S. 2006/0019346); U.S. Application Ser. No. 12/993,542 (published as U.S. 2011/0086138), U.S. Application Ser. No. 12/373,235 (published as U.S. 2010/0055209); International Application Ser. No. PCT/IL2007/000454 (published as WO 2007/116404); U.S. Application Ser. No. 10/472,056 (published as U.S. 2004/0138189); U.S. Pat. No. 5,891,919; U.S. Pat. No. 6,376,657; U.S. Application Ser. No. 11/554,982 (published as U.S. 2007/0104805); U.S. Application Ser. No. 11/926,745 (published as U.S. 2008/0108604); International Application Ser. No. PCT/CA2009/001688 (published as WO 2010/060198); U.S. Application Ser. No. 12/735,557 (published as U.S. 2010/0330205); International Application Ser. No. PCT/CA2007/001066 (published as WO 2008/000063); U.S. Application Ser. No. 11/438,204 (published as U.S. 2006/0269617); U.S. Application Ser. No. 10/563,713 (published as U.S. 2006/0172020); U.S. Application Ser. No. 10/902,352 (published as U.S. 2006/0024335); U.S. Application Ser. No. 10/538,038 (published as U.S. 2006/0275765), U.S. Application Ser. No. 11/555,617 (published as U.S. 2008/0187936); U.S. Application Ser. No. 12/739,264 (published as U.S. 2010/0316736); U.S. Application Ser. No. 12/215,609 (published as U.S. 2009/0042813); U.S. Application Ser. No. 11/893,088 (published as U.S. 2008/0050499); U.S. Pat. No. 7,807,204; U.S. Application Ser. No. 11/811,166 (published as U.S. 2008/0003268); U.S. Pat. No. 6,376,657; International Application Ser. No. PCT/US2011/041183 (published as WO 2011/163183); International Application Ser. No. PCT/EP2011/059814 (published as WO 2011/157692); U.S. Application Ser. No. 12/790,292 (published as U.S. 2011/0293753); International Application Ser. No. PCT/JP2009/071700 (published as WO 2010/076879); U.S. Application Ser. No. 13/032,530 (published as U.S. 2011/0217394); International Application Ser. No. PCT/EP2011/000110 (published as WO 2011/085979); International Application Ser. No. PCT/US2010/058467 (published as WO 2011/068814); U.S. Application Ser. No. 13/060,996 (published as U.S. 2011/0152361); U.S. Application Ser. No. 12/09,253 (published as U.S. 2011/0124609); U.S. Application Ser. No. 12/687,962 (published as U.S. 2011/0119499); and International Application Ser. No. PCT/EP2010/004623 (published as WO 2011/012298).

### Uses in Therapy

The biguanide compounds of the disclosure, including analogs, salts, solvates, polymorphs, hydrates, N-oxides, and prodrugs of such compounds, may be administered to a subject in need thereof to treat various metabolic disorders, including obesity, dislipidemia or other disorders of lipid metabolism as well as hyperglycemic conditions and histopathological diseases associated with hyperglycemia, including type II diabetes. Particularly in view of the surprising and unexpected decoupling of systemic bioavailability and therapeutic efficacy achieved herein, and consequent improvement in toxicity and safety, the effective use of such compounds for prophylaxis and prevention of such diseases and disorders, as well as use for more general weight loss purposes, is also explicitly contemplated herein.

In preferred embodiments, the compound is metformin. Prior formulations of metformin are reported to have an average bioavailability of 30% to 60% while many comparable small molecules have bioavailability of greater than 60%. See, e.g., Tucker et al., "Metformin kinetics in healthy subjects and in patients with diabetes mellitus" Br. J. Clin. Pharmacol. 1981, 12(2) 235-246. Notably, metformin administration increases plasma concentrations of GLP-1 in normal, diabetic and DPP-IV-deficient rodents, as well as in humans with and without type II diabetes, but has been reported to do so indirectly and independent of a direct impact on intestinal L cells. Mulherin et al., supra.

As demonstrated herein, however, and contrary to the well-established convention in the art, enteroendocrine activation by metformin may be triggered by luminal signals on the epithelial aspect of the gut, and therefore increased systemic bioavailability of metformin is actually unnecessary after oral ingestion in order to stimulate the release of gastrointestinal hormones such as GLP-1. Accordingly, the effective treatment of otherwise contraindicated patients is now made possible by administering compositions comprising biguanide compounds (including analogs, salts, solvates, polymorphs, hydrates, N-oxides, and prodrugs thereof) adapted to minimize the systemic bioavailability of the compound. In preferred embodiments, the subject compositions are formulated so as to minimize and preferably avoid an initial release in the stomach and/or proximal small intestine (areas with the greatest absorption) in order to reduce systemic bioavailability upon oral administration.

### Delivery to Specific Intestinal Locations

The embodiments described herein provide a a delayed-release composition comprising a biguanide compound (including any analogs, salts, solvates, polymorphs, hydrates, N-oxides, or prodrugs thereof) formulated to be delivered to one or more locations of the small intestine and/or lower intestine, and preferably distal small intestine, in order to minimize systemic bioavailability by avoiding absorption in the stomach and proximal small intestine and corresponding rapid increase in Cₘₐₓ.

The biguanide compounds are targeted beyond the stomach to one or more regions of the small intestine, and are preferably targeted downstream or distal of the duodenum. In preferred embodiments, the compounds are delivered to the jejunum, ileum, caecum and colon, or a combination thereof. In preferred embodiments, the compounds are delivered to the jejunum, ileum and caecum, or a combination thereof. In preferred embodiments, the compounds are preferentially targeted to the ileum. In additional embodiments, the compound is delivered downstream or distal of the jejunum, or solely to the lower intestine.

In yet other embodiments, the biguanide compound (including an analog, salt, solvate, polymorph, hydrate, N-oxide, or prodrug thereof) is delivered to one or more regions of the upper intestine and one or more regions of the lower intestine. For example, the compound can be delivered to the duodenum and the colon. In another non-limiting example, the compound can be delivered to the duodenum, jejunum, ileum and colon.

The administration of biguanides such as metformin to the preferred regions or locations of the intestine may be achieved by any known method. In preferred embodiments, the biguanide compound is formulated in a delayed-release composition for oral delivery that delivers the compound to the targeted regions or locations of the intestine. When delivery of the biguanide compound is targeted to two or more regions of the gastrointestinal tract, the compound may be delivered in any proportion and manner.

### Minimizing systemic exposure

As described above, the delayed-release pharmaceutical composition for use disclosed herein minimize the systemic bioavailability of the biguanide compound in contraindicated patients. In some embodiments, the biguanide compounds have reduced average systemic bioavailability. Reduced average systemic bioavailabity, in some embodiments, is lower average systemic bioavailability as compared to an immediate release or extended release formulation having an equivalent amount of the biguanide compound. In other embodiments, reduced average systemic bioavailability is when the average systemic bioavailability is less than 30%, less than 25%, less than 15%, less than 10% and less than 5% as compared to an immediate or extended release formulation having an equivalent amount of the biguanide compound. In certain instances, the average systemic bioavailability is less than 15%.

In some embodiments, the subject delayed-release pharmaceutical composition minimize the mean plasma Cₘₐₓ and/or mean AUC levels of the biguanide compound in contraindicated patients. In some embodiments, the pharmaceutical compositions for use result in minimal plasma absorption, mean Cₘₐₓ and/or mean AUC levels of the biguanide compounds in the patient. In other embodiments, the mean plasma Cₘₐₓ, and/or mean AUC levels of the biguanide compound are considered sub-therapeutic for the described compositions as compared to the reported Cₘₐₓ and/or AUC levels of conventional immediate-release and extended-release formulations having identical amounts of metformin. For example, negligible or sub-therapeutic metformin plasma Cₘₐₓ and/or AUC levels include 75%, 60%, 50%, 40% and 30% of reported Cₘₐₓ and/or AUC levels of known metformin formulations (e.g., GLUMETZA^{®}, GLUCOPHAGE^{®}, GLUCOPHAGE^{®} XR, RIOMET^{®}, FORTAMET^{®}, OBIMET^{®}, GLUFORMIN^{®}, DIANBEN^{®}, DIABEX^{®}, DIAFORMIN^{®}, Metformin IR^{®}, Metformin SR^{®}, and the like).

In specific embodiments, the inventive compositions directed to metformin produce a Cₘₐₓ that is no more than 75% or 85%, preferably no more than 50% or 60%, more preferably no more than 25% or 30% or 40% of the same dose of an immediate release metformin formulation (e.g. GLUCOPHAGE^{®}) following oral ingestion. In other embodiments, the compositions provide a Cₘₐₓ that is no more than 3x, more preferably no more than 2.5x or 2x, still more preferably no more than 1.8x or 1.5x the initial trough plasma concentration 10-12 hours after the last oral ingestion of metformin. In other embodiments, the inventive compositions provide a mean plasma AUC over the dosing interval that is no more than 75% or 80%, preferably no more than 50% or 60%, more preferably no more than 25%, 30% or 40% of the same dose of an immediate release formulation (e.g. GLUCOPHAGE^{®}) following oral ingestion.

Accordingly, in specific embodiments, administration of the subject delayed-release formulation minimizes the mean plasma AUC, the mean plasma Cₘₐₓ and/or the circulating plasma concentration of the biguanide compound in contraindicated patients compared to an identical protocol administering an IR or XR formulation having the same amount of the biguanide compound. In one embodiment, the mean plasma AUC_{0-∞} of the biguanide compound resulting from administration is less than about 15,000 ng*h/mL or 14,000 ng*h/mL, preferably less than about 12,000 ng*h/mL, 11,000 ng*h/mL or 10,000 ng*h/mL, more preferably less than about 9,000 ng*h/mL, 8,000 ng*h/mL or 7,000 ng*h/mL. In one embodiment, the resulting mean plasma Cₘₐₓ of the biguanide compound is less than about 1000 ng/mL, preferably less than about 900 ng/mL or 800 ng/mL, more preferably less than about 700 ng/mL, 600 ng/mL or 500 ng/mL. In one embodiment, the resulting circulating plasma concentration of the biguanide compound is below about 5 µg/ml or 4 µg/ml, preferably below about 3 µg/ml or 2.5 µg/ml, more preferably below about 2 µg/ml, 1 µg/ml, 0.5 µg/ml, or 0.25 µg/ml in the patient. In preferred embodiments, the biguanide compound is metformin, the IR composition is Glucophage^{®} and the XR composition is Glucophage^{®} XR.

### Formulations

To limit its systemic bioavailability, the compositions comprising the biguanide compound are adapted for delayed release so as to minimize plasma absorption. The delivery of biguanide compounds such as metformin to the enteroendocrine cells is via any known method including, e.g., oral, rectal, nasogastric tube, parenteral injection such as intraluminal intestinal injection. In preferred embodiments, oral dosage forms are to be administered. Oral delivery of biguanide compounds is described in the delayed release formulations section and include timed release systems, enteric coatings and pH dependent systems, and the like. In some embodiments, the compositions comprising the compounds described herein utilize a multicomponent system where the biguanide compound is delivered to several places in the gastrointestinal tract such as the duodenum, jejunum, ileum, lower intestine or combinations thereof following administration. For example, a delayed-release formulation comprising the biguanide compound can deliver to the lower intestine by use of timed or delayed (enteric) release components. Multicomponent systems of such compounds can be in unitary dosage forms such as bi- or tri- or multiple-layer tablets or multi-particulate forms such as encapsulated micro-tablets, granules or as separate dosage forms, e.g., separate tablets taken together or at a periodic interval.

As described herein, the delayed-release formulation releases the biguanide compound after onset of a desired pH, due to the enteric coating. pHs contemplated include about pH 6.0, more preferably about pH 6.5 and about pH 7.0. After onset of a desired pH, the compound begins release. Such compositions may release the biguanide compound in about 5 minutes, about 10 minutes, about 15 minutes, about 20 minutes, about 25 minutes or about 30 minutes after the onset of the desired pH, and/or may have timed, extended or slow release aspects that release the biguanide compound over the course of a longer time period such as about 1 hour, about 2 hours, about 3 hours, about 4 hours, about 5 hours, about 6 hours, about 7 hours or about 8 hours. Exemplary two component delivery system can be, in some embodiments, a bilayer tablet. Three, four and additional components are contemplated within the embodiments.

For delayed-release formulations comprising the biguanide compound, dosages of the compound can range from about 1 mg to about 2000 mg, about 10 mg to about 1500 mg, about 50 mg to about 1000 mg or about 100 mg or about 500 mg per day. In some instances, the dosage of the compound is about 2000 mg, about 1500 about 1000 mg, about 800 mg, about 600 mg, about 500 mg, about 400 mg, about 300 mg, about 250 mg, about 200 mg, about 150 mg, about 100 mg, about 75 mg, about 50 mg, about 25 mg, about 10 mg or about 1 mg per day. In some embodiments, the dosage of the compound is less than 400 mg. In some embodiments, the dosage of the compound is 250 mg.

Salts of biguanide compound include, but are not limited to, hydrochloride, phosphate, sulfate, hydrobromide, salicylate, maleate, benzoate, succinnate, ethanesulfonate, fumarate, glycolate, pamoate, oratate, acetate, isobutyrate, acetylsalicylate, nicotinic acid, adamantoate, zinc chlorophylin, carboxylic acid, benzoic acid, dichloroacetic acid, theophylin-7-acetate, clofibrate, tartate, oxalate, tannate and hydroxyl acid salts. In preferred embodiment, the salt is metformin hydrochloride.

The biguanide compounds of the subject invention can be advantageously administered or combined with additional therapeutic agents, such as anti-obesity and/or antidiabetic agents described herein. Notable agents for combinations with the metformin compositions described herein include DPP-IV inhibitors (e.g., sitagliptin, saxagliptin, berberine, vildagliptin, linagliptin, alogliptin, and the like), SGLT-2 and/or SGLT-1 inhibitors (e.g., dapafloglizin, canafloglizin, LX4211), agonists of GPR40, GPR120, GPR119, GPR41, GPR43, etc., thiazolidinediones (e.g., pioglitazone, rivoglitazone, rosiglitazone, troglitazone, and the like), sulfonylureas (e.g., glipzide, glibenclamide (glyburide), gliquidone, glyclopyramide, glimepiride, gliclazide, acetohexamide, carbutamide, chlorpropamide, tolbutamide, tolazamide, and the like), Dual PPAR agonists (aleglitazar, muraglitazar, tesaglitazar, and the like) , lipid-lowering agents (e.g., statins), and anti-hypertensive agents.

Formulations for the compositions provided herein include those suitable for oral administration. The formulations can conveniently be presented in unit dosage form and can be prepared by any of the methods well known in the art of pharmacy. All methods include the step of bringing into association the active ingredient with the carrier which constitutes one or more accessory ingredients.

Formulations suitable for oral administration can be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion.

Composition preparations which can be used orally include tablets, push-fit capsules made of gelatin, as well as soft, sealed capsules made of gelatin and a plasticizer, such as glycerol or sorbitol. Tablets can be made by compression or molding, optionally with one or more accessory ingredients. Compressed tablets can be prepared by compressing in a suitable machine the active ingredient in a free-flowing form such as a powder or granules, optionally mixed with binders (e.g., povidone, gelatin, hydroxypropylmethyl cellulose), inert diluents, preservative, disintegrant (e.g., sodium starch glycolate, cross-linked povidone, cross-linked sodium carboxymethyl cellulose) or lubricating, surface active or dispersing agents. Molded tablets can be made by molding in a suitable machine a mixture of the powdered compound moistened with an inert liquid diluent. The tablets can optionally be coated or scored and can be formulated so as to provide slow or controlled release of the active ingredient therein. Tablets can optionally be provided with an enteric coating, to provide release in parts of the gut other than the stomach. All formulations for oral administration should be in dosages suitable for such administration. The push-fit capsules can contain the active ingredients in admixture with filler such as lactose, binders such as starches, and/or lubricants such as talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. Dragee cores are provided with suitable coatings. For this purpose, concentrated sugar solutions can be used, which can optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or Dragee coatings for identification or to characterize different combinations of active compound doses.

It should be understood that in addition to the ingredients particularly mentioned above, the compounds and compositions described herein can include other agents conventional in the art having regard to the type of formulation in question, for example those suitable for oral administration can include flavoring agents.

The compositions described herein can also contain the biguanide compound in a form suitable for oral use, for example, as tablets, troches, lozenges, aqueous or oily suspensions, dispersible powders or granules, emulsions, hard or soft capsules, or syrups or elixirs. Compositions intended for oral use can be prepared according to any method known to the art for the manufacture of pharmaceutical compositions, and such compositions can contain one or more agents selected from, by way of non-limiting example, sweetening agents, flavoring agents, coloring agents and preserving agents in order to provide pharmaceutically elegant and palatable preparations.

### Delayed Release Formulations

Many strategies can be pursued to obtain delayed release in which the location of the release is controlled so as to minimize systemic absorption. For example, delayed release can be obtained by the appropriate selection of formulation parameters and ingredients (e.g., appropriate controlled release compositions and coatings). Examples include single or multiple unit tablet or capsule compositions, oil solutions, suspensions, emulsions, microcapsules, microspheres, nanoparticles and liposomes. The release mechanism can be controlled such that the biguanide compounds are released at period intervals or the location of the release is controlled, the release of combined agents can be simultaneous, or a delayed release of the biguanide compound in a combination can be affected when the early release of another combined therapeutic one is preferred over the other. Different delivery systems described herein can also be combined to release at an onset of multiple period intervals (e.g., about 30 minutes, about 120 minutes, about 180 minutes and about 240 minutes after oral administration) or at different locations (e.g., release in the lower intestine, upper intestine, the jejunum, ileum, caecum, colon, and/or rectum) or a combination thereof. For example, a pH dependent system can be combined with a timed release system or any other system described herein to achieve a desired release profile.

In certain preferred embodiments, the formulation comprises an oral dosage form comprising a biguanide compound and having a delayed- release system engineered to include a lag phase of at least about 10 minutes before release of the drug at the desired pH and/or intestinal location, more preferably at least about 12, 15 or 18 minutes before release of the drug, still more preferably at least about 20, 30 or 60 minutes before release of the drug at the desired pH and/or intestinal location, *e.g.* the small intestine and preferably the distal small intestine.

In certain embodiments, the biguanide compounds are provided in the form of a delayed release formulation coupled with an extended release component of the biguanide compound and/or an additional therapeutic agent in a unitary dosage form. The extended release component can be formulated by any known method such as a layer that envelops a portion of the delayed release component or the like. Exemplary ratios of extended release of an additional therapeutic agent to delayed release of a biguanide compound are about 10% XR to about 90% DR, about 15% XR to about 85% DR, about 20% XR to about 80% DR, about 25% XR to about 75% DR, about 30% XR to about 70% DR, about 35% XR to about 65% DR, about 40% XR to about 60% DR, about 45% XR to about 55% DR, or about 50% XR to about 50% DR. In certain embodiments, the extended release of an active agent to modified release of an active agent is about 25% XR to about 75% DR. In certain embodiments, the extended release of an active agent to modified release of an active agent is about 20% XR to about 80% DR. Unitary dosage forms with an XR and DR component include any known formulation including bilayer tablets, coated pellets, and the like.

In certain embodiments, the biguanide compounds are provided in the form of a delayed release formulation coupled with an immediate release component of an additional therapeutic agent in a unitary dosage form. The immediate release component can be formulated by any known method such as a layer that envelops the delayed release component or the like. Exemplary ratios of immediate release of an additional therapeutic agent to delayed release of a biguanide compound are about 10% IR to about 90% DR, about 15% IR to about 85% DR, about 20% IR to about 80% DR, about 25% IR to about 75% DR, about 30% IR to about 70% DR, about 35% IR to about 65% DR, about 40% IR to about 60% DR, about 45% IR to about 55% DR, or about 50% IR to about 50% DR. In certain embodiments, the immediate release of an active agent to delayed release of an active agent is about 25% IR to about 75% DR. In certain embodiments, the immediate release of an active agent to delayed release of an active agent is about 20% IR to about 80% DR. Unitary dosage forms with an IR and DR component include any known formulation including bilayer tablets, coated pellets, and the like.

### Enteric coatings and pH Dependent Systems

The formulation may also be coated with an enteric coating, which protects an active agent, for example a biguanide compound, from degradation in an acidic environment, such as the stomach, and allows a delayed release into a target area, for example the ileum, for uptake.

The enteric coating may be, as a non-limiting example, wax or wax like substance, such as carnauba wax, fatty alcohols, hydrogenated vegetable oils, zein, shellac, sucrose, Arabic gum, gelatin, dextrin, psyllium husk powder, polymethacrylates, anionic polymethacrylates, mixtures of poly(methacrylic acid, methyl methacrylate), polymers or copolymers derived from acrylic and/or methacrylic acid esters, cellulose acetate phthalate, cellulose acetate trimelliate, hydroxypropyl methylcellulose phthalate (HPMCP), cellulose propionate phthalate, cellulose acetate maleate, polyvinyl alcohol phthalate, hydroxypropyl methylcellulose acetate succinate (HPMCAS), hydroxypropyl methylcellulose hexahydrophthalate, polyvinyl acetate phthalate, mixtures of poly(methacrylic acid, ethyl acrylate), ethylcellulose, methylcellulose, propylcellulose, chitosan succinate, chitosan succinate, polyvinyl acetate phthalate (PVAP), polyvinyl acetate polymers carboxymethylethyl cellulose and compatible mixtures thereof. In addition, an inactive intermediate film may be provided between the biguanide compound, and the enteric coating to prevent interaction of the biguanide compound with the enteric coating.

In one non-limiting example, silicone microspheres for pH-controlled gastrointestinal drug delivery have been described by Carelli et al., Int. J. Pharmaceutics 179: 73-83, 1999. The microspheres are pH-sensitive semi-interpenetrating polymer hydrogels made of varying proportions of poly(methacrylic acid-co-methylmethacrylate) (EUDRAGIT^{®} L100 or EUDRAGIT^{®} S100) and crosslinked polyethylene glycol 8000 that are encapsulated into silicone microspheres. The EUDRAGIT^{®} series of methacrylic acid copolymers are commercially available from Evonik Industries in Darmstadt, Germany.

The enteric coatings can be formulated to release a biguanide compound at a desired pH using combinations of enteric polymers. It is well-known that different locations of the gastrointestinal system have specific pHs. For example, the duodenum may correspond to a pH 5.5 environment and the jejunum may correspond to pH 6.0 environment. As described herein, the enteric coatings are formulated to release the compound at an onset of a desired pH, e.g., in the distal small intestine and lower intestine, i.e., at about pH 6, about pH 6.5, or about pH 7. In embodiments with multiple releases, the enteric coatings are formulated to release at an onset of two or more pH values. In certain embodiments, the enteric coatings are formulated to release at the jejunum, ileum, and lower intestine. In yet other embodiments, the enteric coatings are used in combination with alternative release systems such as a timed release system.

In certain embodiments, the enteric coating is applied to the oral dosage form at a thickness of at least about 4.5mg/cm², 5 mg/cm², 5.5 mg/cm², 6 mg/cm², 6.5 mg/cm², 7 mg/cm², 7.5 mg/cm², 8 mg/cm², 9 mg/cm², 10mg/cm², 11 mg/cm² 12 mg/cm², 15 mg/cm², or 20 mg/cm². For tablets and capsules, the enteric coating can be applied to achieve about a 2.5% to about a 5%, 6%, 7%, 8%, 9% 10% or 12% (wt/wt) weight gain, more preferably about a 3.0% to about a 6% (wt/wt) weight gain, still more preferably at least about a 3.5% or 4% (wt/wt) weight gain. For granules and other multi-particulate dosage forms up to 20 or 30% (wt/wt) weight gain or more can be applied, preferably from about 20% - 50% (wt/wt), more preferably from about 30% - 50% (wt/wt). As demonstrated herein, this ensures appropriate release at the desired intestinal location to avoid aberrant spikes in systemic biguanide compound exposure.

In other embodiments, pharmaceutical compositions are provided comprising an enterically-coated oral dosage form comprising a biguanide compound, wherein the dosage form is an enterically-coated tablet or capsule, wherein said enteric coating is applied to said oral dosage form to about 3% to about a 6% (wt/wt) weight gain, and wherein less than 15% of the biguanide compound is released after the composition contacts an aqueous medium of 0.1 N HCl for two hours and is subsequently transferred to an aqueous medium at a pH of about 6.8 for a lag phase of at least ten minutes, and at least 60% of the biguanide compound is released after the lag phase and within 60 minutes at pH 6.8, and at least 90% of the biguanide compound is released within 90 to 120 minutes at pH 6.8. In one embodiment, an enteric coating is applied to the pharmaceutical composition at a weight gain of at least about 4.5 mg/cm², 5 mg/cm², 5.5 mg/cm², 6 mg/cm², 6.5 mg/cm², 7 mg/cm², 7.5 mg/cm², 8 mg/cm², 9 mg/cm², 10 mg/cm², 11 mg/cm², 12 mg/cm², or 15 mg/cm². In another embodiment, the enteric coating is applied at a weight gain of at least about 5 mg/cm² to 9.5 mg/cm², more preferably at least about 5.5 mg/cm² to at least about 7.6 mg/cm². In alternative embodiments, an enteric coating is applied to the pharmaceutical composition to achieve at least about a 4% to at least about a 6% (wt/wt) weight gain.

In particular embodiments, pharmaceutical compositions are provided comprising an enterically-coated oral dosage form comprising a biguanide compound, wherein the dosage form is adapted to release less than about 10% 5%, 4%, 3%, 2% and preferably less than 1% of the biguanide compound after contacting an aqueous medium (e.g., submersion) at a pH of less than about 2 for about two hours followed by contacting an aqueous medium at a pH equal to or less than about 5.5 for at least 30 to 45 minutes. In a preferred embodiment, the enterically-coated dosage form releases less than about 5%, 2 % or 1% of the biguanide compound in an aqueous medium of 0.1 N HCl for two hours and less than about 5%, 2% or 1% when transferred to an aqueous medium at pH 5.5 for at least 30 to 45 minutes.

In further embodiments, the enterically-coated dosage form releases less than 10%, 5%, 3%, 2% or less than 1% of the biguanide compound during the lag phase after the dosage form is contacted with an aqueous medium at a pH of about 6.8, wherein the lag phase is at least ten, fifteen or twenty minutes. In a preferred embodiment, the enterically-coated dosage form releases less than about 15% of the biguanide compound when the dosage form is contacted with an aqueous medium at a pH of about 6.8 for a lag phase of at least ten minutes and releases greater than 90%, 95%, 98%, or 99% of the biguanide compound after contacting with an aqueous medium at a pH of about 6.8 for a total of ninety to 120 minutes.

In two-stage dissolution embodiments, pharmaceutical compositions are provided comprising an enterically-coated oral dosage form comprising a biguanide compound, wherein the dosage form is adapted to release less than 5%, 2% or 1% of the biguanide compound in an aqueous medium of 0.1 N HCl for two hours. In these embodiments, less than 10%, 5%, 3%, 2%, or preferably 1% of the biguanide compound is released after contacting an aqueous medium of 0.1 N HCl for two hours and subsequently transferred to an aqueous medium at a pH of about 6.8 for a lag phase of at least ten, fifteen or twenty minutes. In preferred embodiments, less than 15% of the biguanide compound is released after two hours at acid pH and a lag phase of at least ten or fifteen minutes at pH 6.8, and at least 60% of the biguanide compound is released after the lag phase and within 60 minutes at pH 6.8, and at least 90% of the biguanide compound is released within 90 to 120 minutes at pH 6.8.

In three-stage dissolution embodiments, pharmaceutical compositions are provided comprising an enterically-coated oral dosage form comprising a biguanide compound, wherein the dosage form is adapted to release less than 5%, 2% or 1% of the biguanide compound in an aqueous medium of 0.1 N HCl for two hours and less than 5%, 2% or 1% when transferred to an aqueous medium at pH 5.5 for at least one hour. In these embodiments, less than 15%, 10%, or 5% of the biguanide compound is released after two hours in aqueous medium of 0.1 N HCl, 30 minutes in an aqueous medium at pH 5.5, and during a lag phase of at least ten or fifteen minutes at pH 6.8. In preferred embodiments, less than 10% or 5% of the biguanide compound is released after two hours at acid pH, 30 minutes at pH 5.5 and a lag phase of at least ten or fifteen minutes at pH 6.8, and at least 60% of the biguanide compound is released after the lag phase and within 60 minutes at pH 6.8, and at least 90% of the biguanide compound is released within 90 to 120 minutes at pH 6.8.

In alternative embodiments, the subject formulations and compositions further comprise one or more disintegrants to accelerate the dissolution of the core upon breaching of the enteric coating. In preferred embodiments, the disintegrant comprises croscarmellose sodium, sodium starch glycolate, or combinations thereof.

In alternative embodiments, the subject formulations and compositions further comprise a seal coating between the biguanide compound and the enteric coating, to provide a total coating thickness corresponding to at least about 4% to 8% (wt./wt.) weight gain, more preferably at least about a 4.5% to 6.0% (wt./wt.) weight gain. In some embodiments, the combination of the outer enteric coating and inner seal coat comprises at least about 6.9 mg/cm² to 13.3 mg/cm², more preferably at least about 7.8 mg/cm² to at least about 11.4 mg/cm².

In some embodiments, the enteric coating comprises a first polymer which releases the biguanide compound at least about 10 minutes after contacting a pH of 6.8 or 7.0, more preferably at least about 15 or 20 minutes, still more preferably at least about 25, 30, 45 or 60 minutes after contacting a pH of 6.8 or 7.0. In preferred embodiments the polymer is insoluble in acidic media, but dissolves by salt formation or the like above pH 7.0. In an exemplary preferred embodiment the polymer is Eudragit FS, or Eudragit S.

In further embodiments, the enteric coating further comprises a second polymer that dissolves at a lower pH than the first polymer. In preferred embodiments, the second polymer is insoluble at pH 5.5 and below, but dissolves by salt formation or the like above pH 5.5. In an exemplary preferred embodiment the second polymer is Eudragit L. In some embodiments, Eudragit L is replaced with cellulose acetate succinate, hydroxy propyl methyl cellulose phthalate, hydroxy propyl methyl cellulose acetate succinate (hypromellose acetate succinate), polyvinyl acetate phthalate (PVAP) and sodium alginate, stearic acid, or combinations thereof.

In preferred embodiments the enteric coating comprises about 90% Eudragit FS and about 10% Eudragit L, about 80% Eudragit FS and about 20% Eudragit L, about 70% Eudragit FS and about 30% Eudragit L, about 60% Eudragit FS and about 40% Eudragit L, about 50% Eudragit FS and about 50% Eudragit L, about 40% Eudragit FS and about 60% Eudragit L, about 30% Eudragit FS and about 70% Eudragit L, about 20% Eudragit FS and about 80% Eudragit L, or about 10% Eudragit FS and about 90% Eudragit L. In preferred embodiments, Eudragit FS and said Eudragit L are present in about a 7:5 to about a 5:7 ratio, and more preferably about a 6:4 to about a 4:6 ratio. In an exemplary preferred embodiment, the enteric coating comprises about 60% Eudragit FS and about 40% Eudragit L.

In some embodiments, a seal coat may be added between the biguanide compound and the enteric coating. The seal coat material may be selected so as to have no effect on the drug release. Suitable materials include, e.g., hydroxypropylmethylcellulose (HPMC). In other embodiments, the seal coat material may be selected to extend the lag phase slowing drug release after the enteric coating is breached. Suitable materials include, e.g. Eudragit E which dissolves in acid but swells at higher pH, and may be used to extend the lag phase after the enteric coating has been breached.

In some embodiments, the seal coating is a mixture of hypromellose, titanium dioxide, polyethylene glycol 400 (macrogol), and polysorbate 80, where the hypromellose is the polymeric coating, titanium dioxide is a coloring agent, polyethylene glycol 400 serves as an anticaking agent, and polysorbate 80 is present as a dispersant (in aqueous suspension) and plasticizer. In other embodiments, the seal coating is a mixture of hypromellose, triacetin, and talc, where the hypromellose is the polymeric coating, triacetin is present as a plasticizer, and the talc is present as an anti-tack agent. In some embidiments, the seal coating is Opadry^{®} White YS-1-7003 (Colorcon). In other embodiments, the seal coating is Opadry^{®} 03K19229 Clear.

The microcapsule gastroretentive systems described in U.S. Pat. Nos. 6,022,562, 5,846,566 and 5,603,957, can be used in the delayed release delivery methods described herein. Microparticles of an active agent or drug are coated by spraying with a material consisting of a mixture of a film-forming polymer derivative, a hydrophobic plasticizer, a functional agent and a nitrogen-containing polymer. The resulting microcapsules are less than or equal to 1000 microns (gm) in size, and in certain cases such microcapsules are between 100 and 500 microns. These microcapsules remain in the small intestine for at least 5 hours.

Film-forming polymer derivatives used in such microcapsules include, but are not limited to, ethylcellulose, cellulose acetate, and non-hydrosoluble cellulose derivates. The nitrogen-containing polymers include, but are not limited to, polyacrylamide, poly-N-vinylamide, poly-N-vinyl-lactam and polyvinylpyrrolidone. The plasticizer used in such microcapsule include, but are not limited to, glycerol esters, phthalates, citrates, sebacates, cetylalcohol esters, castor oil and cutin. The surface-active and/or lubricating agent used in such microcapsule include, but are not limited to, anionic surfactants, such as by way of example the alkali metal or alkaline-earth metal salts of fatty acids, stearic acid and/or oleic acid, nonionic surfactants, such as by way of example, polyoxyethylenated esters of sorbitan and/or polyoxyethylenated esters of sorbitan and/or polyoxyethylenated derivatives of castor oil; and/or lubricants such as stearates, such as by way of example, calcium, magnesium, aluminum stearate, zinc stearate, stearylfumarate, sodium stearylfimarate, and glyceryl behenate.

One non-limiting example of a lower GI delivery formulation comprises a tablet for lower GI delivery. The inner composition of the tablet comprises about 0.01% weight to about 10.0% by weight of a suitable active ingredient; about 50% by weight to about 98% by weight of a hydrocolloid gum obtainable from higher plants; and about 2% by weight to about 50% by weight of a pharmaceutically acceptable excipient such as a binder. Other optional materials may be present that will assist in establishing the desired characteristics of the pharmaceutical composition. These include materials that may enhance absorption of the active ingredient in the lower GI, may protect the active ingredient against degradation, may prevent dissolution, and the like. Optionally surrounding the inner composition of the tablet is a coating that is preferably of enteric polymeric material.

In one embodiment, a formulation comprises an excipient selected from the group consisting of sodium starch glyconate, povidone, corn starch, colloidal silicon dioxide, magnesium stearate, hypromellose, polyethylene glycol, and combinations thereof. In one embidiment, a formulation comprises sodium starch glyconate, povidone, corn starch, colloidal silicon dioxide, and magnesium stearate as excipients. In another embidiment, a formulation comprises povidone, magnesium stearate, hypromellose, and polyethylene glycol as excipients.

The formulation is designed to take advantage of (1) the protective characteristics of the hydrocolloid obtainable from higher plants in the upper GI and (2) the disintegrative characteristics of the hydrocolloid in the lower GI. Thus, the inner composition of the tablet may be one of several designs: (a) it may be a matrix of a therapeutically effective amount of the active ingredient uniformly dispersed throughout in combination with a high percentage of the hydrocolloid and a generally lesser amount of other excipients; (b) it may have a core, in which the active ingredient is concentrated, surrounded by a layer of material that is free of the active ingredient and that has a high percentage of the hydrocolloid and a generally lesser amount of other excipients; (c) it may have a concentration gradient of the active ingredient such that there is a greater amount in the core of the tablet with lesser amounts in multiple layers surrounding the core and very little or no active ingredient in the outer layer. Whether the design of the tablet is that of (a), (b) or (c) above, the specificity for regional delivery to the lower GI is enhanced by enterically coating the tablet with an appropriate enteric coating material.

Suitable hydrocolloids are well known in the art. See for example "The Chemistry of Plant Gums and Mucilages" by Smith and Montgomery from the A.C.S. Monograph series, #141, 1959, Reinhold Publishing Co. and the Eighteenth Edition of The Merck Index. In general, the amount of the hydrocolloid that will be used is an amount that allows the composition to traverse the upper GI tract without significant disintegration and without releasing significant amounts of active ingredient in the upper GI tract, i.e. to provide a delayed-release profile. Generally, that amount of hydrocolloid will be more than about 50% but less than about 98%. Depending on individual variability, whether a patient has eaten or has fasted, and other factors, a tablet will traverse the stomach and upper intestinal tract in about 3 to 6 hours. During this time, little active ingredient (less than 20%, preferably less than 10%) is released from the tablet of this invention. Once the tablet reaches the lower GI, the release of the active ingredient is triggered by enzymatic degradation of the galactomannan gum.

### Timed release systems

In one embodiment, the delayed-release mechanism is a "timed" or temporal release ("TR") system that releases an active agent, for example a biguanide compound, at certain timepoints subsequent to administration. Timed release systems are well known in the art and suitable timed release systems can include any known excipient and/or coating. For example, excipients in a matrix, layer or coating can delay release of an active agent by slowing diffusion of the active agent into an environment. Suitable timed release excipients include but are not limited to, acacia (gum arabic), agar, aluminum magnesium silicate, alginates (sodium alginate), sodium stearate, bladderwrack, bentonite, carbomer, carrageenan, Carbopol, cellulose, microcrystalline cellulose, ceratonia, chondrus, dextrose, furcellaran, gelatin, Ghatti gum, guar gum, galactomannan, hectorite, lactose, sucrose, maltodextrin, mannitol, sorbitol, honey, maize starch, wheat starch, rice starch, potato starch, gelatin, sterculia gum, xanthum gum, Glyceryl behenate (e.g., Compritol 888 ato), Gylceryl distearate (e.g. Precirol ato 5), polyethylene glycol (e.g., PEG 200-4500), polyethylene oxide, adipic acid, gum tragacanth, ethyl cellulose (e.g., ethyl cellulose 100), ethylhydroxyethyl cellulose, ethylmethyl cellulose, methyl cellulose, hydroxyethyl cellulose, hydroxyethylmethyl cellulose (e.g., KlOOLV, K4M, Kl5M), hydroxypropyl cellulose, poly(hydroxyethyl methacrylate), cellulose acetate (e.g. cellulose acetate CA-398-10 NF), cellulose acetate phthalate, cellulose acetate propionate, cellulose acetate butyrate, hydroxypropyl methyl cellulose acetate succinate, hydroxypropyl methyl cellulose phthalate, cellulose butyrate, cellulose nitrate, oxypolygelatin, pectin, polygeline, povidone, propylene carbonate, polyandrides, methyl vinyl ether/maleic anhydride copolymer (PVM/MA), poly(methoxyethyl methacrylate), poly(methoxyethoxyethyl methacrylate), hydroxypropyl cellulose, hydroxypropylmethyl cellulose, sodium carboxymethyl-cellulose (CMC), silicon dioxide, vinyl polymers, e.g. polyvinyl pyrrolidones(PVP: povidone), polyvinyl acetates, or polyvinyl acetate phthalates and mixtures, Kollidon SR, acryl derivatives (e.g. polyacrylates, e.g. cross-linked polyacrylates, methycrylic acid copolymers), Splenda^{®} (dextrose, maltodextrin and sucralose) or combinations thereof. The timed release excipient may be in a matrix with active agent, in another compartment or layer of the formulation, as part of the coating, or any combination thereof. Varying amounts of one or more timed release excipients may be used to achieve a designated release time.

One non-limiting example includes formulations of the TIMERx^{®} system. This controlled release formulation system provides for altered temporal release (SyncroDoseTM) as well as biphasic release (Geminex^{®}). (See, for example, Staniforth & Baichwal, TIMERx®: novel polysaccharide composites for controlled/programmed release of active ingredients in the gastrointestinal tract, Expert Opin. Drug Deliv., 2(3): 587-89 (2005)). Using formulations such as these for the invention described herein, compositions can be created which target the upper gastrointestinal tract, the lower gastrointestinal tract, or both, in addition to temporally controlling the release of such compounds in any of these locations.

In some embodiments, the timed release systems are formulated to release the compound at an onset of about 10 minutes, about 20 minutes, about 30 minutes, about 40 minutes, about 50 minutes, about 60 minutes, about 70 minutes, about 80 minutes, about 90 minutes, about 100 minutes, about 110 minutes, about 120 minutes, about 130 minutes, about 140 minutes, about 150 minutes, about 160 minutes, about 170 minutes, about 180 minutes, about 190 minutes, about 200 minutes, about 210 minutes, about 220 minutes, about 230 minutes, about 240 minutes, about 250 minutes, about 260 minutes, about 270 minutes, about 280 minutes, about 290 minutes, about 300 minutes, about 310 minutes, about 320 minutes, about 330 minutes, about 340 minutes, about 350 minutes, about 360 minutes, about 370 minutes, about 380 minutes, about 390 minutes, about 400, about 400, about 410, or about 420 minutes subsequent to administration. In embodiments with multiple releases, timed release systems are formulated to release at more than one time point. In certain embodiments, the timed release systems are formulated to release at an onset of about 10 minutes, about 30 minutes, about 120 minutes, about 180 minutes and about 240 minutes after administration. In certain embodiments the timed release systems are formulated to release at an onset of about 105 to about 135 minutes, about 165 to about 195 minutes, about 225 to about 255 minutes or a combination of times thereof following administration to a patient.

### Modified Release Formulations

In additional embodiment, the compositions directed to biguanide compound delivery may further employ controlled, sustained, or extended release formulations known collectively as "modified release" formulations. Compositions can be administered by modified release sysems or by delivery devices that are well known to those of ordinary skill in the art. Examples include, but are not limited to, those described in U.S. Pat. Nos. 3,845,770; 3,916,899; 3,536,809; 3,598,123; 4,008,719; 5,674,533; 5,059,595; 5,591,767; 5,120,548; 5,073,543; 5,639,476; 5,354,556; and 5,733,566. Such dosage forms can be used to provide modified release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable modified release formulations known to those of ordinary skill in the art, including those described herein, can be readily selected for use with the active ingredients of the invention. The invention thus encompasses single unit dosage forms suitable for oral administration such as tablets, capsules, gelcaps, and caplets that are further adapted for modified release.

In some embodiments, the modified release systems are formulated to release the compound at a duration of about 30 minutes, about 40 minutes, about 50 minutes, about 60 minutes, about 70 minutes, about 80 minutes, about 90 minutes, about 100 minutes, about 110 minutes, about 120 minutes, about 130 minutes, about 140 minutes, about 150 minutes, about 160 minutes, about 170 minutes, about 180 minutes, about 190 minutes, about 200 minutes, about 210 minutes, about 220 minutes, about 230 minutes, about 240 minutes, about 250 minutes, about 260 minutes, about 270 minutes, about 280 minutes, about 290 minutes, about 300 minutes, about 310 minutes, about 320 minutes, about 330 minutes, about 340 minutes, about 350 minutes, about 360 minutes, about 370 minutes, about 380 minutes, about 390 minutes, about 400, about 400, about 410, or about 420 minutes subsequent to onset of the release. In embodiments with multiple releases, modified release systems are formulated to release at more than one durations of time at different time points.

In one non-limiting example, chitosan and mixtures of chitosan with carboxymethylcellulose sodium (CMC-Na) have been used as vehicles for the sustained release of active ingredients, as described by Inouye et al., Drug Design and Delivery 1: 297-305, 1987. Mixtures of these compounds and agents of the combinations of the invention, when compressed under 200 kg/cm2, form a tablet from which the active agent is slowly released upon administration to a patient. The release profile can be changed by varying the ratios of chitosan, CMC-Na, and active agent(s). The tablets can also contain other additives, including lactose, CaHPO4 dihydrate, sucrose, crystalline cellulose, or croscarmellose sodium.

In another non-limiting example, Baichwal, in U.S. Pat. No. 6,245,356, describes sustained release oral, solid dosage forms that include agglomerated particles of a therapeutically active medicament in amorphous form, a gelling agent, an ionizable gel strength enhancing agent and an inert diluent. The gelling agent can be a mixture of a xanthan gum and a locust bean gum capable of cross-linking with the xanthan gum when the gums are exposed to an environmental fluid. Preferably, the ionizable gel enhancing agent acts to enhance the strength of cross-linking between the xanthan gum and the locust bean gum and thereby prolonging the release of the medicament component of the formulation. In addition to xanthan gum and locust bean gum, acceptable gelling agents that may also be used include those gelling agents well known in the art. Examples include naturally occurring or modified naturally occurring gums such as alginates, carrageenan, pectin, guar gum, modified starch, hydroxypropylmethylcellulose, methylcellulose, and other cellulosic materials or polymers, such as, for example, sodium carboxymethylcellulose and hydroxypropyl cellulose, and mixtures of the foregoing.

In another non-limiting formulation useful for the combinations of the invention, Baichwal and Staniforth in U.S. Pat. No. 5,135,757 describe a free-flowing slow release granulation for use as a pharmaceutical excipient that includes from about 20 to about 70 percent or more by weight of a hydrophilic material that includes a heteropolysaccharide (such as, for example, xanthan gum or a derivative thereof) and a polysaccharide material capable of cross-linking the heteropolysaccharide (such as, for example, galactomannans, and most preferably locust bean gum) in the presence of aqueous solutions, and from about 30 to about 80 percent by weight of an inert pharmaceutical-filler (such as, for example, lactose, dextrose, sucrose, sorbitol, xylitol, fructose or mixtures thereof). After mixing the excipient with a tricyclic compound/corticosteroid combination, or combination agent, of the invention, the mixture is directly compressed into solid dosage forms such as tablets. The tablets thus formed slowly release the medicament when ingested and exposed to gastric fluids. By varying the amount of excipient relative to the medicament, a slow release profile can be attained.

Slow-release formulations can also include a coating which is not readily water-soluble but which is slowly attacked and removed by water, or through which water can slowly permeate. Thus, for example, the combinations of the invention can be spray-coated with a solution of a binder under continuously fluidizing conditions, such as describe by Kitamori et al., U.S. Pat. No. 4,036,948. Examples of water-soluble binders include pregelatinized starch (e.g., pregelatinized corn starch, pregelatinized white potato starch), pregelatinized modified starch, water-soluble celluloses (e.g. hydroxypropyl-cellulose, hydroxymethyl-cellulose, hydroxypropylmethyl-cellulose, carboxymethyl-cellulose), polyvinylpyrrolidone, polyvinyl alcohol, dextrin, gum arabicum and gelatin, organic solvent-soluble binders, such as cellulose derivatives (e.g., cellulose acetate phthalate, hydroxypropylmethyl-cellulose phthalate, ethylcellulose).

In another non-limiting example, Villa et al., in U.S. Pat. No. 6,773,720, describes a modified-release system containing an inner lipophilic matrix where an active ingredient is inglobated and an outer hydrophilic matrix in which the lipophilic matrix is dispersed. An active ingredient, such as a biguanide or related heterocyclic compound, is first inglobated in a low melting lipophlilic excipient or mixture of excipients while heating to soften and/or melt the excipient itself, which thereby incorporates the active ingredient by simple dispersion. After cooling at room temperature, an inert matrix forms, which can be reduced in size to obtain matrix granules containing the active ingredient particles. The inert matrix granules are subsequently mixed together with one or more hydrophilic water-swellable excipients. In this respect, when the composition is contacted with biological fluids, a high viscosity swollen layer is formed, which coordinates the solvent molecules and acts as a barrier to penetration of the aqueous fluid itself inside the new structure. Said barrier antagonizes the staring "burst effect" caused by dissolution of the active ingredient inglobated inside the inert matrix, which is in its turn inside the hydrophilic matrix. One commercially available system of this type is from Cosmo Technologies Limited (Italy) under the trade name MMX^{®} technology. The lipophilic/hydrophilic matrices can be further enterically coated for pH specific delivery.

Formulations for upper intestinal delivery, lower intestinal delivery or both are known in the art. Targeting of active ingredients to various regions of the gut is described, e.g., in The Encyclopedia of Pharmaceutical Technology, by James Swarbrick and James Boylan, Informa Health Care, 1999, at pp. 287-308. Any suitable formulation for gastrointestinal delivery for site-specific delivery and/or specific temporal delivery (i.e. delayed) can be used with the invention and is contemplated herein.

Any of the delivery systems described herein may be used in combination with others to achieve multiple releases and/or specific release profiles. In some embodiments, the biguanide compound is in a formulation that achieves multiple releases in gastrointestinal locations following administration. In certain embodiments, the biguanide compound is in a multiple release formulation that releases at an onset of about 30 minutes, about 120 minutes, about 180 minutes, about 240 minutes, or combinations thereof following administration. In certain embodiments, the biguanide compound is in a multiple release formulation that releases at an onset of about 105 to about 135 minutes, about 165 to about 195 minutes, about 225 to about 255 minutes, or combinations thereof following administration.

In certain embodiments, the biguanide compound is in a multiple release formulation that releases in the jejunum, ileum, lower intestine or combinations thereof following administration.

### Oral Dosage Forms

Oral dosage forms suitable for use in the subject compositions include tablets, hard capsules, push-fit capsules made of gelatin, as well as soft, sealed capsules made of gelatin and a plasticizer, such as glycerol or sorbitol, as well as troches or lozenges. Suitable oral dosage forms can be prepared according to any method known to the art for the manufacture of pharmaceutical compositions, and such compositions can contain one or more agents selected from, by way of non-limiting example, sweetening agents, flavoring agents, coloring agents and preserving agents in order to provide pharmaceutically elegant and palatable preparations.

Tablets contain the active ingredient in admixture with pharmaceutically acceptable excipients which are suitable for the manufacture of tablets. These excipients can be, for example, inert diluents, such as calcium carbonate, sodium carbonate, lactose, calcium phosphate or sodium phosphate; granulating and disintegrating agents, such as microcrystalline cellulose, sodium crosscarmellose, corn starch, or alginic acid; binding agents, for example starch, gelatin, polyvinyl-pyrrolidone or acacia, and lubricating agents, for example, magnesium stearate, stearic acid or talc. Tablets can be made by compression or molding, optionally with one or more accessory ingredients. Compressed tablets can be prepared by compressing in a suitable machine the active ingredient in a free-flowing form such as a powder or granules, optionally mixed with binders (e.g., povidone, gelatin, hydroxypropylmethyl cellulose), inert diluents, preservatives, disintegrants (e.g., sodium starch glycolate, cross-linked povidone, cross-linked sodium carboxymethyl cellulose) or lubricating, surface active or dispersing agents. Molded tablets can be made by molding in a suitable machine a mixture of the powdered compound moistened with an inert liquid diluent. The tablets are coated by known techniques to delay disintegration and absorption in the gastrointestinal tract and thereby minimize systemic bioavailability as described more fully herein.

Formulations for oral use can also be presented as hard gelatin capsules wherein the active ingredient is mixed with an inert solid diluent, for example, calcium carbonate, calcium phosphate or kaolin, or as soft gelatin capsules wherein the active ingredient is mixed with water soluble carrier such as polyethyleneglycol or an oil medium, for example peanut oil, liquid paraffin, or olive oil. Alternatively, push-fit capsules can contain the active ingredients in admixture with filler such as lactose, binders such as starches, and/or lubricants such as talc or magnesium stearate and, optionally, stabilizers. In soft capsules, the active compounds can be dissolved or suspended in suitable liquids, such as fatty oils, liquid paraffin, or liquid polyethylene glycols. In addition, stabilizers can be added. Dragee cores are provided with suitable coatings. For this purpose, concentrated sugar solutions can be used, which can optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments can be added to the tablets or Dragee coatings for identification or to characterize different combinations of active compound doses.

It should be understood that in addition to the ingredients particularly mentioned above, the compounds and compositions described herein can include other agents conventional in the art having regard to the type of formulation in question, for example those suitable for oral administration can include flavoring agents.

Also described herein are compositions in liquid form. Liquid forms include, by way of non-limiting example, neat liquids, solutions, suspensions, dispersions, colloids, foams and the like. In certain instances, liquid forms contain also a nutritional component or base (e.g., derived from milk, yogurt, shake, or juice). In some aspects, the compound are micronized or as nanoparticles in the liquid form. In certain instances, the compounds may be coated to mask taste properties. In other instances, the compounds are coated to modify delivery to the distal small intestine and colon.

Aqueous solutions or suspensions contain the active ingredient(s) in admixture with excipients suitable for the manufacture of aqueous suspensions. Such excipients are suspending agents, for example sodium carboxymethylcellulose, methylcellulose, hydroxypropylmethylcellulose, sodium alginate, polyvinyl-pyrrolidone, gum tragacanth and gum acacia; dispersing or wetting agents can be a naturally-occurring phosphatide, for example lecithin, or condensation products of an alkylene oxide with fatty acids, for example polyoxyethylene stearate, or condensation products of ethylene oxide with long chain aliphatic alcohols, for example heptadecaethylene-oxycetanol, or condensation products of ethylene oxide with partial esters derived from fatty acids and a hexitol such as polyoxyethylene sorbitol monooleate, or condensation products of ethylene oxide with partial esters derived from fatty acids and hexitol anhydrides, for example polyethylene sorbitan monooleate. The aqueous solutions or suspensions can also contain one or more preservatives, for example ethyl, or n-propyl p-hydroxybenzoate, one or more coloring agents, one or more flavoring agents, and one or more sweetening agents, such as sucrose, saccharin or aspartame. In certain instances, the flavoring agents are the compounds.

Oily suspensions can be formulated by suspending the active ingredient(s) in a vegetable oil, for example arachis oil, olive oil, sesame oil or coconut oil, or in mineral oil such as liquid paraffin. The oily suspensions can contain a thickening agent, for example beeswax, hard paraffin or cetyl alcohol. Sweetening agents such as those set forth above, and flavoring agents can be added to provide a palatable oral preparation. These compositions can be preserved by the addition of an antioxidant such as butylated hydroxyanisol or alpha-tocopherol.

Dispersible powders and granules suitable for preparation of an aqueous solutions or suspension by the addition of water provide the active ingredient in admixture with a dispersing or wetting agent, suspending agent and one or more preservatives. Suitable dispersing or wetting agents and suspending agents are exemplified by those already mentioned above. Additional excipients, for example sweetening, flavoring and coloring agents, can also be present. These compositions can be preserved by the addition of an antioxidant such as ascorbic acid.

Compositions can also be in the form of an oil-in-water emulsion. The oily phase can be a vegetable oil, for example olive oil or arachis oil, or a mineral oil, for example liquid paraffin or mixtures of these. Suitable emulsifying agents can be naturally-occurring phosphatides, for example soy bean lecithin, and esters or partial esters derived from fatty acids and hexitol anhydrides, for example sorbitan monooleate, and condensation products of the said partial esters with ethylene oxide, for example polyoxyethylene sorbitan monooleate. The emulsions can also contain sweetening agents, flavoring agents, preservatives and antioxidants.

Syrups and elixirs can be formulated with sweetening agents, for example glycerol, propylene glycol, sorbitol or sucrose. Such formulations can also contain a demulcent, a preservative, flavoring and coloring agents and antioxidant.

Compositions can also be formulated in rectal compositions such as suppositories or retention enemas, e.g., containing conventional suppository bases such as cocoa butter, polyethylene glycol, or other glycerides. These compositions can be prepared by mixing the inhibitors with a suitable non-irritating excipient which is solid at ordinary temperatures but liquid at the rectal temperature and will therefore melt in the rectum to release the drug. Such materials include cocoa butter, glycerinated gelatin, hydrogenated vegetable oils, mixtures of polyethylene glycols of various molecular weights and fatty acid esters of polyethylene glycol.

Accordingly, pharmaceutical compositions are provided comprising the biguanide compound in a delayed-release formulation suitable for oral administration such as a tablet, capsule, pill or lozenge. The pharmaceutical composition is preferably in a unit dosage form suitable for single administration of precise dosages, *e.g.*, 100 mg, 200 mg, 250, mg, 300 mg, 400 mg, 500 mg, 600 mg, 750 mg, 800 mg, or 1000 mg of the desired biguanide compound, particularly metformin, phenformin, buformin or imeglimin or a salt thereof. The pharmaceutical composition may comprise conventional pharmaceutical carriers or excipients and the biguanide compound according to the invention as an active ingredient. They may further comprise other medicinal or pharmaceutical agents, carriers, adjuvants, etc.

Suitable carriers include inert diluents or fillers, water and various organic solvents. The compositions can, if desired, contain additional ingredients such as flavorings, binders, excipients and the like. Thus for oral administration, tablets containing various excipients, such as citric acid can be employed together with various disintegrants such as starch or other cellulosic material, alginic acid and certain complex silicates and with binding agents such as sucrose, gelatin and acacia. Additionally, lubricating agents such as magnesium stearate, sodium lauryl sulfate and talc are often useful for tableting purposes. Other reagents such as an inhibitor, surfactant or solubilizer, plasticizer, stabilizer, viscosity increasing agent, or film forming agent can also be added. Solid compositions of a similar type can also be employed in soft and hard filled gelatin capsules. Materials include lactose or milk sugar and high molecular weight polyethylene glycols. When aqueous suspensions or elixirs are desired for oral administration the active compound therein can be combined with various sweetening or flavoring agents, coloring matters or dyes and, if desired, emulsifying agents or suspending agents, together with diluents such as water, ethanol, propylene glycol, glycerin, or combinations thereof.

### Excipients

Any of the compositions or formulations described herein include any commonly used excipients in pharmaceutics and are selected on the basis of compatibility with the active agent(s) and release profile properties of the desired dosage form. Excipients include, but are not limited to, binders, fillers, flow aids/glidents, disintegrants, lubricants, stabilizers, surfactants, and the like. A summary of excipients described herein, may be found, for example in Remington: The Science and Practice of Pharmacy, Nineteeth Ed (Easton, PA: Mack Publishing Company, 1995); Hoover, John E., Remington's Pharmaceutical Sciences, (Easton, PA: Mack Publishing Co 1975); Liberman, H.A. and Lachman, L., Eds., Pharmaceutical Dosage Forms (New York, NY: Marcel Decker 1980); and Pharmaceutical Dosage Forms and Drug Delivery Systems, Seventh Ed (Lippincott Williams & Wilkins 1999).

Binders impart cohesive qualities and include, e.g., alginic acid and salts thereof; cellulose derivatives such as carboxymethylcellulose, methylcellulose (e.g., Methocel^{®}), hydroxypropylmethylcellulose, hydroxyethylcellulose, hydroxypropylcellulose (e.g., Klucel^{®}), ethylcellulose (e.g., Ethocel^{®}), and microcrystalline cellulose (e.g., Avicel^{®}); microcrystalline dextrose; amylose; magnesium aluminum silicate; polysaccharide acids; bentonites; gelatin; polyvinylpyrrolidone/vinyl acetate copolymer; crospovidone; povidone; starch; pregelatinized starch; tragacanth, dextrin, a sugar, such as sucrose (e.g., Dipac^{®}), glucose, dextrose, molasses, mannitol, sorbitol, xylitol (e.g., Xylitab^{®}), and lactose; a natural or synthetic gum such as acacia, tragacanth, ghatti gum, mucilage of isapol husks, polyvinylpyrrolidone (e.g., Polyvidone^{®} CL, Kollidon^{®} CL, Polyplasdone^{®} XL-10), larch arabogalactan, Veegum^{®}, polyethylene glycol, waxes, sodium alginate, and the like.

Disintegrants facilitate breakup or disintegration of oral solid dosage forms after administration. Examples of disintegrants include a starch, e.g., a natural starch such as corn starch or potato starch, a pregelatinized starch such as National 1551 or Amijel^{®}, or sodium starch glycolate such as Promogel^{®} or Explotab^{®}; a cellulose such as a wood product, methylcrystalline cellulose, e.g., Avice10, Avicel^{®} PH101, Avicel^{®} PH102, Avicel^{®} PH105, Elcema^{®} P100, Emcocel^{®}, Vivacel^{®}, Ming Tia^{®}, and Solka-Floc^{®}, methylcellulose, croscarmellose, or a cross-linked cellulose, such as cross-linked sodium carboxymethylcellulose (Ac-Di-Solt), cross-linked carboxymethylcellulose, or cross-linked croscarmellose; a cross-linked starch such as sodium starch glycolate; a cross-linked polymer such as crospovidone; a cross-linked polyvinylpyrrolidone; alginate such as alginic acid or a salt of alginic acid such as sodium alginate; a clay such as Veegum^{®} HV (magnesium aluminum silicate); a gum such as agar, guar, locust bean, Karaya, pectin, or tragacanth; sodium starch glycolate; bentonite; a natural sponge; a resin such as a cation-exchange resin; citrus pulp; sodium lauryl sulfate; sodium lauryl sulfate in combination starch; and the like.

Lubricants are compounds which prevent, reduce or inhibit adhesion or friction of materials. Exemplary lubricants include, e.g., stearic acid; calcium hydroxide; talc; sodium stearyl fumerate; a hydrocarbon such as mineral oil, hydrogenated castor oil or hydrogenated vegetable oil such as hydrogenated soybean oil (Sterotex^{®}); higher fatty acids and their alkalimetal and alkaline earth metal salts, such as aluminum, calcium, magnesium, zinc; stearic acid, sodium stearates, magnesium stearates, glycerol, talc, waxes, Stearowet^{®} boric acid, sodium benzoate, sodium acetate, sodium chloride, leucine, a polyethylene glycol or a methoxypolyethylene glycol such as CarbowaxTM, ethylene oxide polymers, sodium oleate, glyceryl behenate (E.g. Compritol 888 Ato), glyceryl disterate (Precirol Ato 5), polyethylene glycol, magnesium or sodium lauryl sulfate, colloidal silica such as SyloidTM, Carb-O-Si10, DL-leucine, a starch such as corn starch, silicone oil, a surfactant, and the like.

Flow-aids or glidants improve the flow characteristics of powder mixtures. Such compounds include, e.g., colloidal silicon dioxide such as Cab-o-sil^{®}; tribasic calcium phosphate, talc, corn starch, DL-leucine, sodium lauryl sulfate, magnesium stearate, calcium stearate, sodium stearate, kaolin, and micronized amorphous silicon dioxide (Syloid^{®}) and the like.

Plasticizers aid in coating of oral solid dosage forms. Exemplary plasticizers include, but are not limited to, triethyl citrate, triacetin (glyceryl triacetate), acetyl triethyl citrate, polyethylene glycols (PEG 4000, PEG 6000, PEG 8000), Carbowax 400 (polyethylene glycol 400), diethyl phthalate, diethyl sebacate, acetyltriethylcitrate, oleic acid, glyceralmonosterate, tributyl citrate, acetylated monoglycerides, glycerol, fatty acid esters, propylene glycol, and dibutyl phthalate and the like.

The aforementioned excipients are given as examples only and are not meant to include all possible choices. Other suitable excipient classes include coloring agents, granulating agents, preservatives, anti-foaming agents, solubulizers and the like. Additionally, many excipients can have more than one role or function, or can be classified in more than one group; the classifications are descriptive only, and are not intended to limit any use of a particular excipient.

### Combination therapies

The compositions of the embodiments described herein may be co-administered with known therapies for the treatment of any of the conditions described herein. Co-administration can also provide for additive or synergistic effects, resulting in the need for lower dosages of a known therapy, the compositions described herein, or both. Additional benefits of co-administration include the reduction in toxicities associated with any of the known therapies.

Co-administration includes simultaneous administration in separate compositions, administration at different times in separate compositions, or administration in a composition in which both agents are present. Thus, in some embodiments, compositions described herein and a known therapy are to be administered in a single treatment. In some embodiments, the compositions described herein and a known therapy are admixed in a resulting composition. In some embodiments, compositions described herein and the known therapy are to be administered in separate compositions or administrations.

Administration of compositions described herein and known therapies described herein may be by any suitable means. Administration of a composition described herein and a second compound (e.g., diabetes drug or obesity drug) may be by any suitable means. If the compositions described herein and a second compound are to be administered as separate compositions, they may be administered by the same route or by different routes. If the compositions described herein and a second compound are to be administered in a single composition, they may be administered by any suitable route such as, for example, oral administration. In certain embodiments, compositions of metformin or an analog thereof (including salts, solvates, polymorphs, hydrates, N-oxides, or prodrugs thereof) and second compounds can be administered to the same region or different regions of the gastrointestinal tract. For example, metformin or an analog thereof (including salts, solvates, polymorphs, hydrates, N-oxides, or prodrugs thereof) can be administered in combination with an anti-diabetic drug to be delivered to the duodenum, jejunum, ileum, or colon.

Therapies, drugs and compounds useful for the treatment of hyperglycemia and/or diseases or conditions associated therewith, e.g., diabetes may be administered with the compositions disclosed herein. Diabetic drugs and compounds include, but are not limited to, those that decrease triglyceride concentrations, decrease glucose concentrations, and/or modulate insulin (e.g. stimulate insulin production, mimic insulin, enhance glucose-dependent insulin secretion, suppress glucagon secretion or action, improve insulin action or insulin sensitizers, or are exogenous forms of insulin).

Drugs that decrease triglyceride level include but are not limited to ascorbic acid, asparaginase, clofibrate, colestipol, fenofibrate mevastatin, pravastatin, simvastatin, fluvastatin, or omega-3 fatty acid. Drugs that decrease LDL cholesterol level include but are not limited to clofibrate, gemfibrozil, and fenofibrate, nicotinic acid, mevinolin, mevastatin, pravastatin, simvastatin, fluvastatin, lovastatin, cholestyrine, colestipol or probucol.

In another aspect, compositions of the embodiments described herein may be administered in combination with glucose-lowering compounds.

The medication classes of thiazolidinediones (also called glitazones), sulfonylureas, meglitinides, biguanides, alpha-glucosidase inhibitors, DPP-IV inhibitors, and incretin mimetics have been used as adjunctive therapies for hyperglycemia and diabetes mellitus (type 2) and related diseases.

Drugs that decrease glucose level include but are not limited to glipizides, glyburides, exenatide (Byetta^{®}), incretins, sitagliptin (Januvia^{®}), pioglitizone, glimepiride, rosiglitazone, metformin, vildagliptin, saxagliptin (OnglyzaTM), sulfonylureas, meglitinide (e.g., Prandin^{®}) glucosidase inhibitor, biguanides (e.g., Glucophage^{®}), repaglinide, acarbose, troglitazone, nateglinide, natural, synthetic or recombinant insulin and derivatives thereof, and amylin and amylin derivatives.

When administered sequentially, the combination may be administered in two or more administrations. In an alternative embodiment, it is possible that one or more of the biguanide compounds and one or more additional active ingredients are to be administered by different routes. The skilled artisan will also recognize that a variety of active ingredients may be administered in combination with one or more the biguanide compounds that may act to augment or synergistically enhance the control prevention, amelioration, attenuation, or treatment of obesity or eating disorders or conditions.

According to delayed-release pharmaceutical compositions for use provided herein, when to be co-administered with at least one other obesity reducing (or anti-obesity) or weight reducing drug, the compounds of the disclosure may be: (1) co-formulated and to be administered or delivered simultaneously in a combined formulation; (2) delivered by alternation or in parallel as separate formulations; or (3) by any other combination therapy regimen known in the art. When delivered in alternation therapy, the use provided may comprise delivering the active ingredients sequentially, e.g., in separate solution, emulsion, suspension, tablets, pills or capsules, or by different injections in separate syringes. In general, during alternation therapy, an effective dosage of each active ingredient is to be administered sequentially, i.e., serially, whereas in simultaneous therapy, effective dosages of two or more active ingredients are to be administered together. Various sequences of intermittent combination therapy may also be used.

In certain embodiments, compositions provided herein may be used with other commercially available diet aids or other weight loss and/or anti-obesity agents, such as, by way of example, PYY and PYY agonists, GLP-1 and GLP-1 agonists, a DPP-IV inhibitor, CCK and CCK agonists, exendin and exendin agonists, GIP and GIP agonists, amylin and amylin agonists, ghrelin modulators (e.g., inhibitors) and leptin and leptin agonists. In certain instances, compositions comprising the biguanide compound provided herein are used in combination with amylin, amylin agonists or mimetics. Exemplary amylin agonists or mimetics include pramlintide and related compounds. In certain instances, the compounds and compositions provided herein are used in combination with leptin, leptin agonists or mimetics. Additional leptin agonists or mimetics can be identified using the methods described by U.S. Pat. No. 7,247,427. In further instances, the compounds and compositions provided herein increase leptin sensitivity and increase effectiveness of leptin, leptin agonists or mimetics.

Additional anti-obesity agents suitable for use in the subjet methods include those that are in current development. Other anti-obesity agents include phentermine, fenfluramine, sibutramine, rimonabant, topiramate, zonisamide, bupropion, naltrexone, lorcaserin, or related sympathomimetics and orlistat or other intestinal lipase inhibitors, alone or in combination. Therapies, drugs and compounds useful for the treatment of weight loss, binge eating, food addictions and cravings may be administered with the compositions described herein. For example, the patient may further be administered at least one other drug which is known to suppress hunger or control appetite. Such therapies drugs and compounds include but are not limited to phenteramines such as Meridia^{®} and Xenical^{®}. Additional therapies, drugs and compounds are known in the art and contemplated herein.

As such, in one aspect, the compound may be used as part of a combination therapy for the control, prevention or treatment of obesity or eating disorders or conditions. Compounds used as part of a combination therapy to treat obesity or reduce weight include, but are not limited to, central nervous system agents that affect neurotransmitters or neural ion channels, including antidepressants (bupropion), noradrenalin reuptake inhibitors (GW320659), selective 5HT 2c receptor agonists, antiseizure agents (topiramate, zonisamide), some dopamine antagonists, and cannabinoid-1 receptor antagonists (CB-1 receptor antagonists) (rimonabant); leptin/insulin/central nervous system pathway agents, including leptin analogues, leptin transport and/or leptin receptor promoters, ciliary neurotrophic factor (Axokine), neuropeptide Y and agouti-related peptide antagonists, pro-opiomelanocortin and cocaine and amphetamine regulated transcript promoters, alpha. -melanocyte-stimulating hormone analogues, melanocoritin-4 receptor agonists, and agents that affect insulin metabolism/activity, which include protein-tyrosine phosphatase-1B inhibitors, peroxisome proliferator activated receptor-gamma receptor antagonists, short-acting bromocriptine (ergoset), somatostatin agonists (octreotide), and adiponectin/Acrp30 (Famoxin or Fatty Acid Metabolic Oxidation Inducer); gastrointestinal-neural pathway agents, including those that increase cholecystokinin activity (CCK), PYY activity, NPY activity, and PP activity, increase glucagon-like peptide-1 activity (exendin 4, liraglutide, dipeptidyl peptidase IV inhibitors), and those that decrease ghrelin activity, as well as amylin analogues (pramlintide); agents that may increase resting metabolic rate (selective (3-3 stimulators/agonist, uncoupling protein homologues, and thyroid receptor agonists); other more diverse agents, including melanin concentrating hormone antagonists, phytostanol analogues, functional oils, P57, amylase inhibitors, growth hormone fragments, synthetic analogues of dehydroepiandrosterone sulfate, antagonists of adipocyte 11B-hydroxysteroid dehydrogenase type 1 activity, corticotropin-releasing hormone agonists, inhibitors of fatty acid synthesis (cerulenin and C75), carboxypeptidase inhibitors, indanone/indanols, amino sterols (trodusquemine/trodulamine), and other gastrointestinal lipase inhibitors (ATL962); amphetamines, such as dextroamphetamine; other sympathomimetic adrenergic agents, including phentermine, benzphetamine, phendimetrazine, mazindol, and diethylpropion.

Other compounds include ecopipam; oxyntomodulin (OM); inhibitors of glucose-dependent insulinotropic polypeptide (GIP); gastrin-releasing peptide; neuromedin B; enterostatin; amfebutamone, SR-58611; CP-045598; AOD-0604; QC-BT16; rGLP-1; 1426 (HMR-1426); N¬5984; ISIS-113715; solabegron; SR-147778; Org-34517; melanotan-II; cetilistat; c-2735; c-5093; c¬2624; APD-356; radafaxine; fluasterone; GP-389255; 856464; S-2367; AVE-1625; T-71; oleoyl-estrone; peptide YY [3-36] intranasal; androgen receptor agonists; PYY 3-36; DOV-102677; tagatose; SLV-319; 1954 (Aventis Pharma AG); oxyntomodulin, Thiakis; bromocriptine, PLIVA; diabetes/hyperlipidemia therapy, Yissum; CKD-502; thyroid receptor beta agonists; beta-3 adrenoceptor agonist; CDK-A agonists; galanin antagonist; dopamine D1/D2 agonists; melanocortin modulators; verongamine; neuropeptide Y antagonists; melanin-concentrating hormone receptor antagonists; dual PPAR alpha/gamma agonists; CGEN-P-4; kinase inhibitors; human MCH receptor antagonists; GHS-R antagonists; ghrelin receptor agonists; DG70 inhibitors; cotinine; CRF-BP inhibitors; urocortin agonists; UCL-2000; impentamine; beta.-3 adrenergic receptor; pentapeptide MC4 agonists; trodusquemine; GT-2016; C-75; CPOP; MCH-1 receptor antagonists; RED-103004; aminosterols; orexin-1 antagonists; neuropeptide Y5 receptor antagonists; DRF-4158; PT-15; PTPase inhibitors; A37215; SA-0204; glycolipid metabolites; MC-4 agonist; produlestan; PTP-1B inhibitors; GT-2394; neuropeptide Y5 antagonists; melanocortin receptor modulators; MI,N-4760; PPAR gamma/delta dual agonists; NPY5RA-972; 5-HT2C receptor agonist; neuropeptide Y5 receptor antagonists (phenyl urea analogs); AGRP/MC4 antagonists; neuropeptide Y5 antagonists (benzimidazole); glucocorticoid antagonists; MCHR1 antagonists; Acetyl-CoA carboxylase inhibitors; R-1496; HOB1 modulators; NOX-B11; peptide YY 3-36 (eligen); 5-HT 1 modulators; pancreatic lipase inhibitors; GRC-1087; CB-1 antagonists; MCH-1 antagonists; LY-448100; bombesin BRS3 agonists; ghrelin antagonists; MC4 antagonists; stearoyl-CoA desaturase modulators; H3 histamine antagonists; PPARpan agonists; EP-01492; hormone-sensitive lipase inhibitors; fatty acid-binding protein 4 inhibitors; thiolactone derivatives; protein tyrosine phosphatase 1B inhibitors; MCH-1 antagonist; P-64; PPAR gamma ligands; melanin concentrating hormone antagonists; thiazole gastroprokinetics; PA-452; T-226296; A-331440; immunodrug vaccines; diabetes/obesity therapeutics (Bioagency, Biofrontera Discovery GmbH); P-7 (Genfit); DT-011 M; PTP1B inhibitor; anti-diabetic peptide conjugates; KATP agonists; obesity therapeutics (Lexicon); 5-HT2 agonists; MCH-1 receptor antagonists; GMAD-1/GMAD-2; STG-a-MD; neuropeptide Y antagonist; angiogenesis inhibitors; G protein-coupled receptor agonists; nicotinic therapeutics (ChemGenex); anti-obesity agents (Abbott); neuropeptide Y modulators; melanin concentrating hormone; GW-594884A; MC-4R agonist; histamine H3 antagonists; orphan GPCR modulators; MITO-3108; NLC-002; HE-2300; IGF/IBP-2-13; 5-HT2C agonists; ML-22952; neuropeptide Y receptor antagonists; AZ-40140; anti-obesity therapy (Nisshin Flour); GNTI; melanocortin receptor modulators; alpha-amylase inhibitors; neuropeptide Y1 antagonist; beta-3 adrenoceptor agonists; ob gene products (Eli Lilly & Co.); SWR-0342-SA; beta-3 adrenoceptor agonist; SWR-0335; SP-18904; oral insulin mimetics; beta 3 adrenoceptor agonists; NPY-1 antagonists; .beta.-3 agonists; obesity therapeutics (7TM Pharma); l lbeta-hydroxysteroid dehydrogenase (HSD)1 inhibitors; QRX-431; E-6776; RI-450; melanocortin-4 antagonists; melanocortin 4 receptor agonists; obesity therapeutics (CuraGen); leptin mimetics; A-74498; second-generation leptin; NBI-103; CL-314698; CP-114271; beta-3 adrenoceptor agonists; NMI 8739; UCL-1283; BMS-192548; CP-94253; PD-160170; nicotinic agonist; LG-100754; SB-226552; LY-355124; CKD-711; L-751250; PPAR inhibitors; G-protein therapeutics; obesity therapy (Amylin Pharmaceuticals Inc.); BW-1229; monoclonal antibody (ObeSys/CAT); L-742791; (S)sibutramine; MBU-23; YM-268; BTS-78050; tubby-like protein genes; genomics (eating disorders; Allelix/Lilly); MS-706; GI-264879A; GW-409890; FR-79620 analogs; obesity therapy (Hybrigenics SA); ICI-198157; ESP-A; 5-HT2C agonists; PD-170292; AIT-202; LG-100641; GI-181771; anti-obesity therapeutics (Genzyme); leptin modulator; GHRH mimetics; obesity therapy (Yamanouchi Pharmaceutical Co. Ltd.); SB-251023; CP-331684; BIBO-3304; cholesten-3-ones; LY-362884; BRL-48962; NPY-1 antagonists; A-71378; .RTM.-didesmethylsibutramine; amide derivatives; obesity therapeutics (Bristol-Myers Squibb Co.); obesity therapeutics (Ligand Pharmaceuticals Inc.); LY-226936; NPY antagonists; CCK-A agonists; FPL-14294; PD-145942; ZA-7114; CL-316243; SR-58878; R-1065; BIBP-3226; HP-228; talibegron; FR-165914; AZM-008; AZM-016; AZM-120; AZM-090; vomeropherin; BMS-187257; D-3800; AZM-131; gene discovery (Axys/Glaxo); BRL¬26830A; SX-013; ERR modulators; adipsin; AC-253; A-71623; A-68552; BMS-210285; TAK-677; MPV-1743; obesity therapeutics (Modex); GI-248573; AZM-134; AZM-127; AZM-083; AZM-132; AZM-115; exopipam; SSR-125180; obesity therapeutics (Melacure Therapeutics AB); BRL-35135; SR-146131; P-57; AZM-140; CGP-71583A; RF-1051; BMS-196085; manifaxine; beta-3 agonists; DMNJ (Korea Research Institute of Bioscience and Biotechnology); BVT-5182; LY-255582; SNX¬024; galanin antagonists; neurokinin-3 antagonists; dexfenfluramine; mazindol; diethylpropion; phendimetrazine; benzphetamine; amfebutmone; sertraline; metformin; AOD-9604; ATL-062; BVT¬933; GT389-255; SLV319; HE-2500; PEG-axokine; L-796568; and ABT-239.

In some embodiments, compounds for use in combination with a composition comprising the biguanide compound provided herein include rimonabant, sibutramine, orlistat, PYY or an analog thereof, CB-1 antagonist, leptin, phentermine, and exendin analogs. Exemplary dosing ranges include phentermine resin (30 mg in the morning), fenfluramine hydrochloride (20 mg three times a day), and a combination of phentermine resin (15 mg in the morning) and Lorcaserin(30 mg before the evening meal), and sibutramine (10-20 mg). Weintraub et al. (1984) Arch. Intern. Med. 144:1143-1148.

In further embodiments, compounds for use in combination with a composition provided herein include GPR119 agonists (*e.g*., anandamide; AR-231, 453; MBX-2982; Oleoylethanolamide; PSN-365,963; PSN-632,408; palmitoylethanolamide), GPR120 agonists (e.g., omega-3 fatty acids including, but not limited to, a-linolenic acid, docosapentaenoic acid, docosahexaenoic acid, eicosatrienoic acid, eicosatetraenoic acid, eicosapentaenoic acid, heneicosapentaenoic acid, hexadecatrienoic acid, stearidonic acid, tetracosahexaenoic acid and tetracosapentaenoic acid), and GPR 40, GPR41 and GPR 43 agonists (*e.g*., free fatty acids including short-, medium-, and long-chain saturated and unsaturated fatty acids).

In some embodiments, a composition provided herein is used as an adjunctive therapy to a bariatric surgical procedure. Bariatric surgery is a procedure for weight loss and relates to modifications with the gastrointestinal tract and includes such procedures as gastric banding, sleeve gastrectomy, GI bypass procedure (e.g., roux en Y, biliary duodenal bypass, loop gastric bypass), intragastric balloon, vertical banded, gastroplasty, endoluminal sleeve, biliopancreatic diversion, and the like. In certain instances, the composition provided herein is adjunctive to gastric banding. In certain instances, a composition is adjunctive to GI bypass procedures. In yet other instances, a composition provided herein is adjunctive to sleeve gastrectomy. In certain embodiments, a composition provided herein as an adjunctive therapy to bariatric surgery is administered prior to the bariatric procedure. In certain embodiments, a composition provided herein as an adjunctive therapy to bariatric surgery is administered after the bariatric procedure. In certain instances, when used as adjunctive therapy, the dosage and amounts of a composition provided herein may be adjusted as needed with respect to the bariatric procedure. For example, amounts of a composition provided herein administered as an adjunct therapy to a bariatric procedure may be reduced by one-half of normal dosages or as directed by a medical professional.

Combination therapy can be exploited, for example, in modulating metabolic syndrome (or treating metabolic syndrome and its related symptoms, complications and disorders), wherein the compositions provided herein can be effectively used in combination with, for example, the active agents discussed above for modulating, preventing or treating diabetes, obesity, hyperlipidemia, atherosclerosis, and/or their respective related symptoms, complications and disorders.

### Methods for Evaluating Treatment

### Evaluation of Treatment of Diabetes

The effect of the biguanide compound treatment of the invention on aspects of diabetic disease can be evaluated according to methods known in the art and common practiced by physicians treating diabetic patients.

Efficacy of treatment of diabetes/metabolic syndrome and diabetes-associated conditions with the compositions described herein can be assessed using assays and methodologies known in the art. By way of example, quantitative assessment of renal function and parameters of renal dysfunction are well known in the art. Examples of assays for the determination of renal function/dysfunction include serum creatinine level; creatinine clearance rate; cystatin C clearance rate, 24-hour urinary creatinine clearance, 24-hour urinary protein secretion; Glomerular filtration rate (GFR); urinary albumin creatinine ratio (ACR); albumin excretion rate (AER); and renal biopsy.

Quantitative assessment of pancreatic function and parameters of pancreatic dysfunction or insufficiency are also well known in the art. Examples of assays for the determination of pancreas function/dysfunction include evaluating pancreatic functions using biological and/or physiological parameters such as assessment of islets of Langerhans size, growth and/or secreting activity, beta-cells size, growth and/or secreting activity, insulin secretion and circulating blood levels, glucose blood levels, imaging of the pancreas, and pancreas biopsy, glucose uptake studies by oral glucose challenge, assessment of cytokine profiles, blood-gas analysis, extent of blood-perfusion of tissues, and angiogenesis within tissues.

Additional assays for treatment of diabetes and diabetes-associated conditions are known in the art and are contemplated herein.

### Evaluation of Treatment of Weight Loss, Obesity and Eating Disorders

In treatment of obesity it is desired that weight and/or fat is reduced in a patient. By reducing weight it is meant that the patient loses a portion of his/her total body weight over the course of treatment (whether the course of treatment be days, weeks, months or years). Alternatively, reducing weight can be defined as a decrease in proportion of fat mass to lean mass (in other words, the patient has lost fat mass, but maintained or gained lean mass, without necessarily a corresponding loss in total body weight). An effective amount of the biguanide compound to be administered is an amount effective to reduce a patient's body weight over the course of the treatment, or alternatively an amount effective to reduce the patient's percentage of fat mass over the course of the treatment. In certain embodiments, the patient's body weight is reduced, over the course of treatment, by at least about 1%, by at least about 5%, by at least about 10%, by at least about 15%, or by at least about 20%. Alternatively, the patient's percentage of fat mass is reduced, over the course of treatment, by at least 1%, at least 5%, at least 10%, at least 15%, at least 20%, or at least 25%.

Total body weight and fat content can be measured at the end of the dietary period. In rats, a frequently used method to determine total body fat is to surgically remove and weigh the retroperitoneal fat pad, a body of fat located in the retroperitoneum, the area between the posterior abdominal wall and the posterior parietal peritoneum. The pad weight is considered to be directly related to percent body fat of the animal. Since the relationship between body weight and body fat in rats is linear, obese animals have a correspondingly higher percent of body fat and retroperitoneal fat pad weight.

Methods of treating, reducing, or preventing food cravings in a patient are also described herein, food cravings can be measured by using a questionnaire, whether known in the art or created by the person studying the food cravings. Such a questionnaire would preferably rank the level of food cravings on a numerical scale, with the patient marking 0 if they have no food cravings, and marking (if on a scale of 1-10) 10 if the patient has severe food cravings. The questionnaire would preferably also include questions as to what types of food the patient is craving. Binge eating can be determined or measured using a questionnaire and a Binge Eating Scale (BES). Binge eating severity can be divided into three categories (mild, moderate, and severe) based on the total BES score (calculated by summing the scores for each individual item). Accordingly, methods are described herein for reducing the BES score of a patient comprising administering to a patient in need thereof a compound treatment in an amount effective to reduce the BES score of the patient. As described herein, administration of a compound treatment changes the BES category of the patient, for example, from severe to moderate, from severe to mild, or from moderate to mild.

### Pre-Treatment Evaluation of Patient Hormonal Profile

Described herein are also patients that are pre-evaluated for expression of metabolic hormones using methods described herein. The therapy provided to the individual can thus be targeted to his or her specific needs. In embodiments, a patient's hormonal profile is pre-evaluated and depending on the changes that the physician desires to affect, a certain determined amount of the compound/metabolite combination is administered. The evaluation process can be repeated and the treatment adjusted accordingly at any time during or following treatment.

### Hormone Assays

In embodiments, the levels of hormones assayed in association with the delayed-release pharmaceutical compositions for use of the invention, including, but not limited to, GLP-1, GLP-2, GIP, oxyntomodulin, PYY, CCK, glycentin, insulin, glucagon, ghrelin, amylin, uroguanylin, C-peptide and/or combinations thereof are detected according to standard methods described in the literature. For example, proteins can be measured by immunological assays, and transcription products by nucleic acid amplification techniques. Functional assays described in the art can also be used as appropriate. In embodiments, samples assayed comprise cultured cells, patient cell or tissue samples, patient body fluids, e.g., blood or plasma, etc. Similarly, the levels of analytes (e.g., glucose, triglycerides, HDL, LDL, apoB and the like) assayed in association with the delayed-release pharmaceutical composition for use of the invention are detected according to any known method.

For example, immunofluorescence can be used to assay for GLP-1. Cells can be grown on matrigel-coated cover slips to confluent monolayers in 12-well plates at 37°C, fixed in 4% paraformaldehyde in phosphate-buffered saline (PBS) and incubated with primary antiserum (e.g., rabbit anti-alpha gustducin, 1:150; Santa Cruz Biotechnology, and rabbit anti-GLP-1, Phoenix) overnight at 4°C following permeabilization with 0.4% Triton-X in PBS for 10 minutes and blocking for 1 hour at room temperature. Following three washing steps with blocking buffer, the appropriate secondary antibody is applied (AlexaFluor 488 anti-rabbit immunoglobulin, 1:1000; Molecular Probes) for 1 hour at room temperature. After three washing steps, the cells can be fixed in Vectashield medium and the immunofluorescence visualized.

GLP-1 RNA isolated from cells can be assayed using RT-PCR. RT-PCR RNA isolation from cells can be performed using standard methodology. The RT-PCR reaction can be performed in a volume of 50 pl in a Peltier thermal cycler (PTC-225 DNA Engine Tetrad Cycler; MJ Research), using published primer sequences (Integrated DNA Technologies). Reverse transcription can be performed at 50°C for 30 minutes; after an initial activation step at 95°C for 15 minutes. PCR can be performed by denaturing at 94°C for 1 minute, annealing at 55°C for 1 minute and extension at 72°C for 1 minute for 40 cycles, followed by a final extension step at 72°C for 10 minutes. Negative controls can be included as appropriate, for example, by substituting water for the omitted reverse transcriptase or template. The control can be RNA isolated from, e.g., rat lingual epithelium. PCR products can be separated in 2% agarose gel with ethidium bromide, and visualized under UV light.

Radioimmunoassay (RIA) for total GLP-1 in patient blood samples can be performed as described in the art, e.g., by Laferrere, et al., 2007, "Incretin Levels and Effect are Markedly Enhanced 1 Month after Roux-en-Y Gastric Bypass Surgery in Obese Patients with Type 2 Diabetes, Diabetes Care 30(7): 1709-1716 (using commercially available materials obtained from Phoenix Pharmaceutical, Belmont, CA). The authors describe measuring the effect of GIP and GLP-1 on secretion of insulin by measuring the difference in insulin secretion (area under the curve, or AUC) in response to an oral glucose tolerance test and to an isoglycemic intravenous glucose test.

Measurement of plasma concentrations of GLP-1, GIP, glucagon, insulin, C peptide, pancreatic peptide, nonesterified fatty acids, glutamic acid decarboxylase antibodies, and islet antigen antibodies, is described, e.g., by Toft-Nielsen, et al., 2001, "Determinants of the Impaired Secretion of Glucagon-Like Peptide-1 in Type 2 Diabetic Patients," J. Clin. End. Met. 86(8):3717¬3723. The authors describe the use of radioimmunoassay for GLP-1 to measure plasma concentrations of amidated GLP-1-(7-3 6), using antibody code no. 89390. This assay measures the sum of GLP-1-(7-36) and its metabolite GLP-1-(9-36). The authors describe measurement of GIP using C-terminally directed antibody code no. R65 (RIA), that reacts 100% with a human GIP but not with 8-kDA GIP.

GLP-1 and PYY can be directly assayed in the supernatant from venous effluents as described by, e.g., Claustre, et al. (1999, "Stimulatory effect of(3-adrenergic agonists on ileal L cell secretion and modulation by a-adrenergic activation, J. Endocrin. 162:271-8). (See also Plaisancie' et al., 1994, "Regulation of glucagon-like peptide-1-(7-36) amide secretion by intestinal neurotransmitters and hormones in the isolated vascularly perfused rat colon," Endocrinology 135:2398-2403 and Plaisancie' et al., 1995, "Release of peptide YY by neurotransmitters and gut hormones in the isolated, vascularly perfused rat colon," Scandinavian Journal of Gastroenterology 30:568-574.) In this method, the 199D anti-GLP-1 antibody is used at a 1:250 000 dilution. This antibody reacts 100% with GLP-1-(7-36) amide, 84% with GLP-1-(1-36) amide, and less than 0.1% with GLP-1-(1-37), GLP-1-(7-37), GLP-2, and glucagon. PYY is assayed with the A4D anti-porcine PYY antiserum at a 1: 800 000 dilution.

Methods for assaying GLP-1 and GIP are also described elsewhere in the art, e.g., by Jong, et al., PNAS, 2007.

PYY can also be assayed in blood using a radioimmunoassay as described by, e.g., Weickert, et al., 2006, "Soy isoflavones increase preprandial peptide YY (PYY), but have no effect on ghrelin and body weight in healthy postmenopausal women" Journal of Negative Results in BioMedicine, 5:11. Blood is collected in ice-chilled EDTA tubes for the analysis of glucose, ghrelin, and PYY. Following centrifugation at 1600 g for 10 minutes at 4°C, aliquots were immediately frozen at -20°C until assayed. All samples from individual patients were measured in the same assay. The authors described measuring immunoreactive total ghrelin was measured by a commercially available radioimmunoassay (Phoenix Pharmaceuticals, Mountain View, CA, USA). (See also Weickert, et al., 2006, "Cereal fiber improves whole-body insulin sensitivity in overweight and obese women," Diabetes Care 29:775-780). Immunoreactive total human PYY is measured by a commercially available radioimmunoassay (LINCO Research, Missouri, USA), using 125I-labeled bioactive PYY as tracer and a PYY antiserum to determine the level of active PYY by the double antibody/PEG technique. The PYY antibody is raised in guinea pigs and recognizes both the PYY 1-36 and PYY 3-36 (active) forms of human PYY.

SGLT-1, the intestinal sodium-dependent glucose transporter 1, is a protein involved in providing glucose to the body. It has been reported to be expressed in response to sugar in the lumen of the gut, through a pathway involving T1R3 (Margolskee, et al., 2007 "T1R3 and gustducin in gut sense sugars to regulate expression of Na+-glucose cotransporter 1," Proc Natl Acad Sci USA 104, 15075-15080"). Expression of SGLT-1 can be detected as described, e.g., by Margolskee, et al., for example, using quantitative PCR and Western Blotting methods known in the art. Measurement of glucose transport has been described in the literature, e.g., by Dyer, et al., 1997, Gut 41:56-9 and Dyer, et al., 2003, Eur. J. Biochem 270:3377-88. Measurement of glucose transport in brush border membrane vesicles can be made, e.g., by initiating D-glucose uptake by the addition of 100 pl of incubation medium containing 100 mM NaSCN (or KSCN), 100 mM mannitol, 20 mM Hepes/Tris (pH 7.4), 0.1 mM MgSO4, 0.02% (wt/vol) NaN3, and 0.1 mM D-[U14C]glucose to BBMV (100 µg of protein). The reaction is stopped after 3 sec by addition of 1 ml of ice-cold stop buffer, containing 150 mM KSCN, 20 mM Hepes/Tris (pH 7.4), 0.1 mM MgSO4, 0.02% (wt/vol) NaN3, and 0.1 mM phlorizin. A 0.9-ml portion of the reaction mixture is removed and filtered under vacuum through a 0.22-[tm pore cellulose acetate/nitrate filter (GSTF02500; Millipore, Bedford, MA). The filter is washed five times with 1 ml of stop buffer, and the radioactivity retained on the filter is measured by liquid scintillation counting.

### EXAMPLES

### EXAMPLE 1: ENTEROENDOCRINE PRODUCTION OF PYY, GLP-1 (Active) AND GLP-1 (Total) AND REDUCTION OF GLUCOSE AND INSULIN IS INDEPENDENT OF PLASMA ABSORPTION OF METFORMIN

### Example 1.1 Materials and Methods

Population: Approximately 18 eligible male and female subjects, 18 to 65 years of age, with a BMI of 25.0 to 35.0 kg/m², were randomized in this study. To be eligible, each subject also met the following criteria: (a) was not breastfeeding; (b) had a negative pregnancy test result (human chorionic gonadotropin, beta subunit); (c) surgically sterile, postmenopausal, or if of childbearing potential, practiced appropriate birth control during the entire duration of the study; (d) had a physical examination with no clinically significant abnormalities, including but not limited to the following conditions: (i) Hepatic disease; (ii) Renal disease; (iii) gastrointestinal disease; (iv) Endocrine disorder, including diabetes; (v) Cardiovascular disease; (vi) Seizure disorder; (vii) Organ transplantation; and (viii) Chronic infection; and (e) an ability to understand and willingness to adhere to protocol requirements.

### Formulations

The metformin DR formulation was a US-supplied commercially available film-coated immediate-release tablet containing 500 mg metformin hydrochloride, to which additional coatings (a seal coating and an enteric coating) were applied in order to delay release of the drug in the GI tract until the tablet reached a pH 6.5 region of the distal small intestine. The seal coating was applied at a nominal level of 2% of the core tablet weight and the enteric coating was applied at a nominal level of 2.4% of the core tablet weight. The tablets were white, biconvex, circular-shaped coated tablets, each containing 500 mg metformin hydrochloride. Inactive ingredients in the commercially available tablet included povidone, magnesium stearate, hypromellose, and polyethylene glycol. Inactive ingredients in the additional coating systems included hypromellose, triacetin, talc, methacrylic acid copolymer (Eudragit^{®} L30 D-55), poly(methyl acrylate-co-methyl methacrylate-co-methacrylic acid) 7:3:1 (Eudragit^{®} FS 30 D), sodium lauryl sulfate, polysorbate 80, glyceryl monostearate, and triethyl citrate.

The metformin IR formulation was the identical US-supplied commercially available film-coated immediate-release tablet containing 500 mg metformin hydrochloride, to which only the additional seal coating is applied. No delayed-release (enteric) coating was applied. Inactive ingredients in the additional seal coating system included hypromellose, triacetin and talc.

The metformin formulations were supplied to the site as bulk tablets packaged in screw cap containers labeled with container number and lot number. All study medications were stored in cool and dry conditions as indicated on the label, and used only as directed by study personnel. Study medication was dispensed by the unblinded site pharmacist or study personnel according to the randomization scheme at the beginning of each treatment period.

### Administration

Study medication was dispensed by an unblinded site pharmacist or study personnel according a randomization scheme at Visits 2 and 4. At the end of Visits 2 and 4, subjects were discharged from the clinic with assigned study medications and with instructions for self-administration until they returned for their next study visit (Visit 3 or 5).

Study medication was administered orally as intact tablets (swallowed whole, not chewed or crushed), and with water. The first dose and the last two doses of study medication for each treatment period were administered to subjects by qualified study site personnel (first dose at Visits 2 and 4 and last two doses at Visits 3 and 5). Subjects self-administered the assigned study medications according to instructions until they returned for their next study visit (Visit 3 or 5). Study site personnel contacted subjects by telephone on the second day of dosing of each treatment period to assess compliance and adverse events through non-directed questioning. If the subject was experiencing significant gastrointestinal symptoms, at the investigator's discretion, subjects were instructed not to dose escalate.

The procedures performed during the study are listed in Tables 1-3 below.

**Table 1: Study Plan (Protocol LCPOC6)**

| Evaluation | Screen | Treatment Period 1 | | | Treatment Period 2 | | | Early Termination |
|---|---|---|---|---|---|---|---|---|
| | | Baseline of Period 1 Visit 2 | Day 2 of Treatment Period Phone Call [1] | End of Period 1 Visit 3 | Baseline of Period 2 Visit 4 | Day 2 of Treatment Period Phone Call [1] | End of Period 2/ Study Termination Visit 5 | |
| Fast (>8 Hours Overnight) | X | X | | X | X | | X | |
| Informed Consent | X | | | | | | | |
| Complete Medical History | X | | | | | | | |
| Physical Examination and Height | X | | | | | | | |
| Bodv Weight and Vital Signs | X | X | | X | X | | X | X |
| Chemistry. Hematology, Urinalysis | X | | | | | | X | X |
| Pregnancy Test (Females) [2] | X | | | | | | X | X |
| Randomization | | X | | | | | | |
| Timed Blood Sampling [3] | | X | | X | X | | X | |
| Studv Medication Administration [4] | | X | | X | X | | X | |
| Dispense Studv Medication | | X | | | X | | | |
| Study Medication Compliance Assessment and Collection | | | | X | | | X | |
| Dose Escalation Phone Call | | | X | | | X | | |
| Concomitant Medications Assessment | X | X | | X | X | | X | X |

| | | | | | | | | |
|---|---|---|---|---|---|---|---|---|
| [1] Phone calls to assess compliance and adverse events through non-directed questioning and to remind subjects to dose escalate [2] Pregnancy test required on all female subjects unless subject has had a hysterectomy or is postmenopausal. [3] GLP-1, PYY, plasma glucose, insulin, and triglycerides at Visits 2 and 4; GLP-1, PYY, plasma glucose, insulin, triglycerides and metformin at Visits 3 and 5. | | | | | | | | |

After meal challenge at Visit 2 and Visit 4. Evening dose on Day 4 and morning dose on Day 5 at Visit 3 and Visit 5.

**Table 2: Schedule of Standardized Breakfast and Blood Sampling Profile at Visit 2 and Visit 4**

| Time (minutes) | Collect 6-mL blood samples [1] | Standardized Breakfast Administration [2] |
|---|---|---|
| -15 | X | |
| -5 | X | |
| 0 | | X |
| 30 | X | |
| 45 | X | |
| 60 | X | |
| 90 | X | |
| 120 | X | |
| 150 | X | |
| 180 | X | |
| 710 | X | |
| 240 | X | |
| 270 | X | |
| 300 | X | |
| 330 | X | |

| | | |
|---|---|---|
| [1] 6-mL blood volume total per sampling time point for assessment of PYY, GLP-1, plasma glucose, insulin, and triglycerides. [2] Subjects are to be instructed to consume the standardized breakfast within 20 minutes. | | |

**Table 3: Day 5 Schedule of Dosing, Standardized Breakfast and Blood Sampling Profile at Visit 3 and Visit 5**

| Time (minutes) | Collect 6-mL blood samples [1] | Standardized Breakfast Administration [2] | Dose Study Medication | Collect 2-mL blood sample [3] |
|---|---|---|---|---|
| -245 | | | | X |
| -240 | | | X | |
| -120 | | | | X |
| -15 | X | | | |
| -5 | X | | | X |
| 0 | | X | | |
| 30 | X | | | X |
| 45 | X | | | X |
| 60 | X | | | X |
| 90 | X | | | X |
| 120 | X | | | X |
| 150 | X | | | X |
| 180 | X | | | X |
| 210 | X | | | X |
| 240 | X | | | X |
| 270 | X | | | X |
| 300 | X | | | X |
| 330 | X | | | X |
| 360 | | | | X |
| 420 | | | | X |
| 480 | | | | X |

| | | | | |
|---|---|---|---|---|
| [1] 6-mL blood volume total per sampling time point for assessment of PYY, GLP-1, plasma glucose, insulin, and triglycerides. [2] Subjects are to be instructed to consume the standardized breakfast within 20 minutes. [3] 2-mL blood volume total per sampling time point for assessment of metformin. | | | | |

### Pharmacodynamic assessments

Blood samples were collected according to the schedules presented in Tables 1, 2, and 3, and as described above. Fasting and postprandial plasma concentrations of gut hormones GLP-1 and PYY, as well as concentrations of plasma glucose, insulin, and triglycerides were measured by analytical methods. Blood samples from each visit was processed and stored at - 70°C for future exploratory analysis of additional hormones.

### Pharmacokinetic assessments

Blood samples were collected according to the schedules presented in Tables 1, 2, and 3, and as described above. Plasma metformin concentrations were measured by analytical methods. Blood samples from each visit were processed and stored at -70°C for future exploratory analysis of additional hormones.

### Clinical Laboratory Evaluations

Samples were collected according to the schedules presented in Tables 1, 2 and 3, and in the preceding section.

### Chemistry

Chemistry assessments included the following: urea nitrogen, creatinine, total protein, albumin, uric acid, total bilirubin, alkaline phosphatase, alanine aminotransferase, aspartate aminotransferase, gamma glutamyltranspeptidase, creatine phosphokinase, glucose, sodium, potassium, chloride, bicarbonate, phosphorus, lactate, and calcium (or other approved routine chemistry panels.

### Hematology

Hematology assessments included the following: red cell count, hemoglobin, hematocrit, white cell count, platelets, differential count, mean cell volume, mean corpuscular hemoglobin, and mean corpuscular hemoglobin concentration (or other approved routine hematology assessments).

### Urinalysis

Urinalysis assessments included the following: pH, specific gravity, glucose, blood, ketones, and protein (or other approved routine urinalysis).

### Pregnancy Testing

All female subjects, regardless of childbearing status (unless subject was postmenopausal or had a hysterectomy), provided blood or urine for pregnancy tests. Study medication was not administered unless a negative result was obtained.

### Vital Signs and Other Observations Related to Safety

Clinically significant abnormalities in vital signs and other observations related to safety were followed up by the investigator and evaluated with additional tests if necessary, until the underlying cause was diagnosed or resolution occurred.

### Vital Signs

Vital sign measurements included sitting systolic and diastolic blood pressure, heart rate, and body temperature. Vital signs were measured after the subject rested for approximately 5 minutes and with the subject in a sitting position. The blood pressure measurement was repeated after at least 30 seconds and the average of the two readings recorded.

### EXAMPLE 1.2: Results

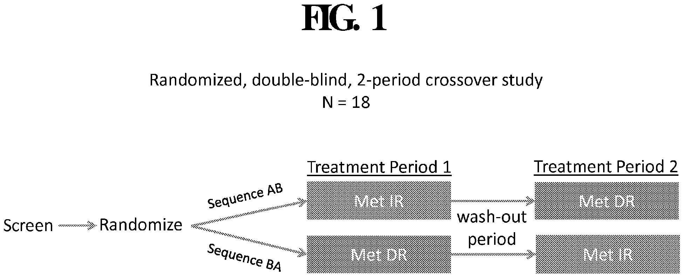

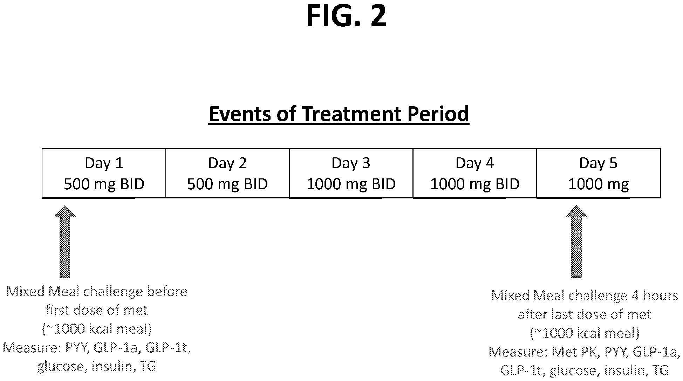

The study design and event timeline are shown in FIGs. 1-2. Shown in Tables 4 and 5 below are the resulting subject disposition and population (Table 4) and the demographic and baseline characteristics of 18 subjects (Table 5).

**Table 4: Subject Disposition and Population**

| **Parameter** | **Result** |
|---|---|
| Randomized | 18 |
| Completed | 17 |
| Withdrawal (positive drug test) | 1 |
| Evaluable Population | 16 |

| | |
|---|---|
| • *2 subjects excluded from evaluable population; 1 withdrawn and 1 could not complete test meal at end of Treatment Period 2* | |

**Table 5: Demographic and Baseline Characteristics (n=18)**

| **Parameter** | **Result** |
|---|---|
| Gender (M/F) | 9 / 9 |
| Mean Age (yr) ± SD | 44 ± 10 |
| Race | 9 Caucasian, 7 Hispanic, 2 black |
| Mean BMI (kg/m2) ± SD | 29.3 ± 2.8 |

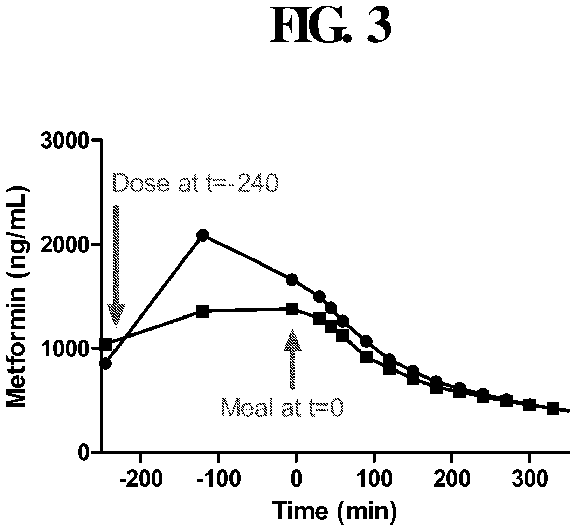

FIG. 3 demonstrates that ingestion of Metformin DR minimized adsorption of metformin in the plasma compared to Metformin IR. The area under the curve (AUC) and Cmax values for Metformin DR and Metformin IR are provided in Table 6 below.

**Table 6: Metformin Plasma Pharmacokinetics**

| | **LS Mean Ratio ReMet /Metformin** | **P Value** |
|---|---|---|
| **Abs AUC** | 0.83 | 0.02 |
| **Abs Cmax** | 0.73 | 0.003 |
| **Incremental Cmax** | 0.45 | <0.001 |

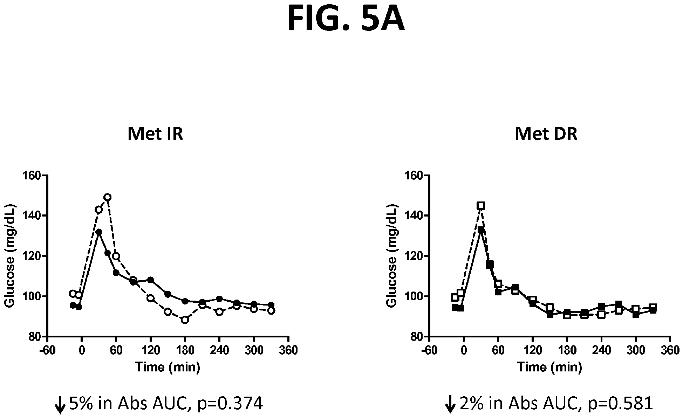

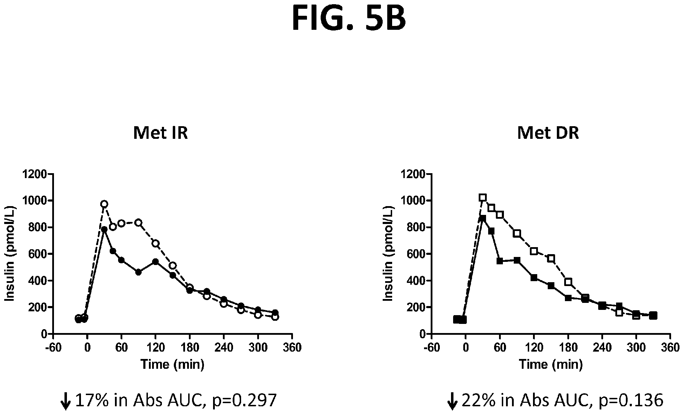

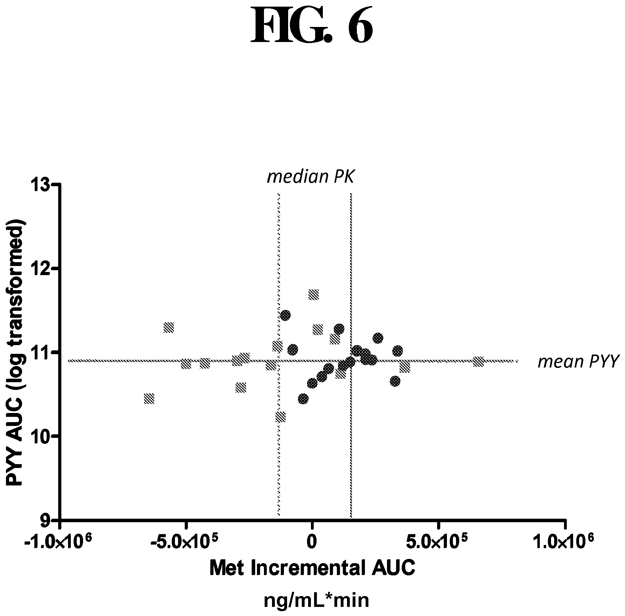

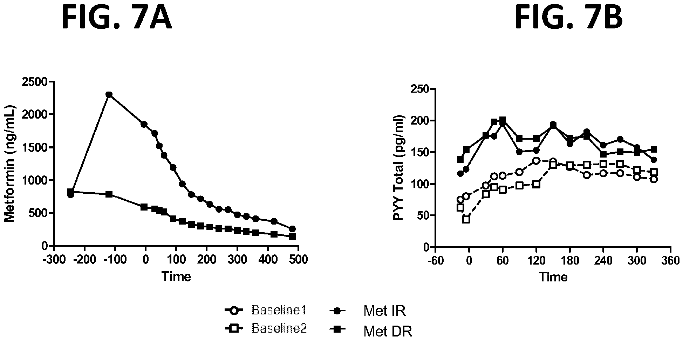

FIG. 4A-C shows an increase in meal-enhanced gut hormones in 16 subjects after treatment of Metformin DR comparable to that of Metformin IR, although treatment with Metformin DR minimized the systemic level of metformin compared to Metformin IR (FIG. 3). Additionally, FIGs. 5A-B show a reduction in meal-enhanced glucose and insulin after treatment with Metformin DR in 16 subjects comparable to that of Metformin IR. FIG. 6 shows that treatment with Metformin DR results in a similar PYY response as Metformin IR, but has a lower systemic exposure. FIGs. 7A-B show that the metformin PK/PD relationship was dissociable in at least one patient.

### EXAMPLE 2 - A RANDOMIZED, CROSSOVER STUDY TO ASSESS STEADY-STATE PK AND PD OF DELAYED-RELEASE AND IMMEDIATE RELEASE METFORMIN IN SUBJECTS WITH TYPE 2 DIABETES MELLITUS

This randomized, crossover study assessed the steady-state pharmacokinetics and pharmacodynamics (glucose, insulin, glucagon-like peptide-1 [GLP-1], and peptide YY [PYY], of 500 mg and 1000 mg metformin delayed-release (Metformin DR), 1000 mg metformin immediate-release (Metformin IR), and 500 mg Metformin IR + 1000 mg Metformin DR in subjects with type 2 diabetes mellitus. Subjects managing their diabetes with oral anti-diabetic therapy must have been off of those medications for at least the fourteen days immediately prior to randomization.

Each treatment period was five days long and separated by washout intervals of seven days. Each treatment period contained a standardized breakfast and lunch profile on Day 1 prior to administration of study drug (baseline assessment) and an identical profile on the morning of Day 5 (on-drug assessment).

### EXAMPLE 2.1: Materials and Methods

Subjects were evaluated for the effects of each treatment on circulating PYY, GLP-1, glucose, and insulin concentrations over approximately 10 hours in response to two standardized meals (-500 kcal standardized breakfast at t=0 min, and ∼1000 kcal standardized lunch at t = 300 min) using standard protocols. Metformin pharmacokinetics over an approximately 11-hour sampling period were also evaluated.

Population: Most randomized subjects were White (79.2%), and half were female (50.0%). The mean age was 51.3 years, the mean weight was 93.4 kg, and the mean BMI was 33.3 kg/m² at baseline. Nineteen of the 24 subjects completed the study.

The primary population for pharmacokinetic and pharmacodynamic analyses was the Evaluable Population (N=19), defined as all subjects who completed all treatment periods consistent with protocol procedures. The primary population for safety analyses was the Intent-to-Treat (ITT) Population (N=24), defined as all subjects who received at least one dose of study medication.

### Formulations

The metformin DR formulation was a US-supplied commercially available film-coated immediate-release tablet containing 500 mg metformin hydrochloride, to which additional coatings (a seal coating and an enteric coating) were applied in order to delay release of the drug in the GI tract until the tablet reaches a pH 6.5 region of the distal small intestine. The seal coating was applied at a nominal level of 2% of the core tablet weight and the enteric coating was applied at a nominal level of 3.8% of the core tablet weight. The tablets are white, biconvex, circular-shaped coated tablets, each containing 500 mg metformin hydrochloride. Inactive ingredients in the commercially available tablet included povidone, magnesium stearate, hypromellose, and polyethylene glycol. Inactive ingredients in the additional Elcelyx coating systems included hypromellose, triacetin, talc, methacrylic acid copolymer (Eudragit^{®} L30 D-55), poly(methyl acrylate-co-methyl methacrylate-co-methacrylic acid) 7:3:1 (Eudragit^{®} FS 30 D), sodium lauryl sulfate, polysorbate 80, glyceryl monostearate, and triethyl citrate.

The metformin IR formulation was the identical US-supplied commercially available film-coated immediate-release tablet containing 500 mg metformin hydrochloride, to which only the additional seal coating is applied. No delayed-release (enteric) coating was applied. Inactive ingredients in the additional seal coating system included hypromellose, triacetin and talc.

The metformin formulations were supplied to the site as bulk tablets packaged in screw cap containers labeled with container number and lot number. All study medications were stored in cool and dry conditions as indicated on the label, and used only as directed by study personnel. Study medication was dispensed by the unblinded site pharmacist or study personnel according to the randomization scheme at the beginning of each treatment period.

### Administration

Study medication was administered orally as intact tablets (swallowed whole) with water at the beginning of the breakfast and dinner meals. Subjects self-administered their assigned study medications on the evening of Day 1 through the morning of Day 4 according to instructions provided on Day 1 by the study site staff. The last two doses of study medication for each treatment period (evening of Day 4 and morning of Day 5) were administered to subjects by qualified study site personnel. In order to reduce gastrointestinal side effects, all treatment regimens initiated treatment at 500 mg/dose for the first 3 doses, followed by an increase to the randomized dose (500 mg/dose, 1000 mg, or 1500 mg/dose) for the remainder of the study period. Study site personnel contacted subjects by telephone on the second day of dosing of each treatment period to assess compliance and adverse events through non-directed questioning and to remind them to dose-escalate if appropriate.

### EXAMPLE 2.2: Results

### Pharmacokinetic Evaluations

### Pharmacokinetic Profiles

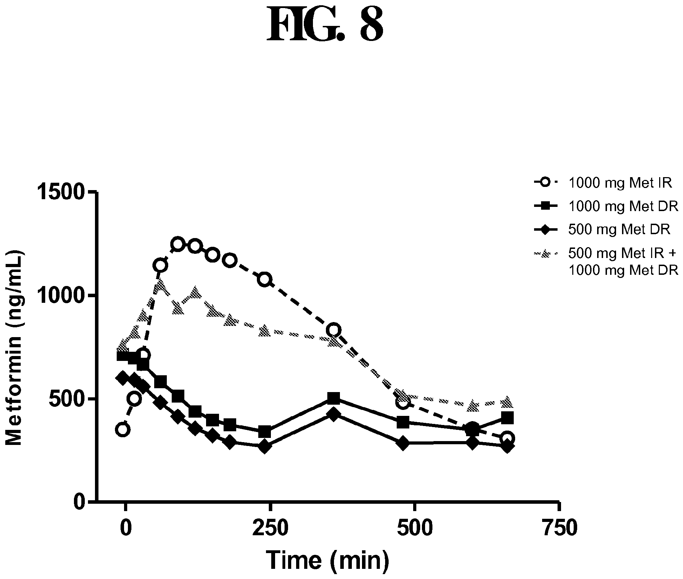

Figure 8 presents the mean plasma metformin concentrations at Day 5 by treatment and time point. On Day 5, the pre-dose mean concentration of Metformin IR at t=0 was 350 ng/mL, which is consistent with steady-state trough concentrations published in the literature. After the administration of Metformin IR at t=-1 minute, there was a rapid increase in metformin concentrations that peaked at 1249 ng/mL 90 min after the dose followed by a steady decline for the remainder of the sampling period.

The pre-dose concentrations for both doses of Metformin DR were approximately 2 times higher than those for Metformin IR (716 ng/mL for 1000 mg DR and 602 ng/mL for 500 ng/mL DR vs. 350 ng/dL for 1000 mg IR). Following the administration of both doses of metformin DR at t=-1 minute, there was a decrease in metformin concentrations for the first 240 minutes followed by a small rise in metformin concentrations after the standardized lunch meal, which then plateaued for the remainder of the sampling period. The entire 11-hour metformin profiles remained below the pre-dose concentrations measured at t=0. The absorption profiles for Metformin DR dosing with the evening meal were slowed relative to doses administered with the breakfast meal, consistent with slowed intestinal transit during the sleeping hours. Metformin DR concentrations for the 500-mg dose were lower than the 1000-mg dose at all time points although the reductions were less than dose-proportional. This observation is consistent with the lack of dose-proportionality reported for Metformin IR and could be due to a saturable absorption process in the gut.

The Metformin DR + Metformin IR treatment group had the highest pre-dose concentrations of the four treatment groups (761 ng/mL). Following the administration of study medication at t=-1 minute, metformin concentrations rapidly rose in a manner similar to metformin IR but generally remained below the Metformin IR concentration curve for the first 500 minutes. For the remainder of the sampling period, concentrations plateaued but where higher than those observed with the other treatments.

### Pharmacokinetic Parameters

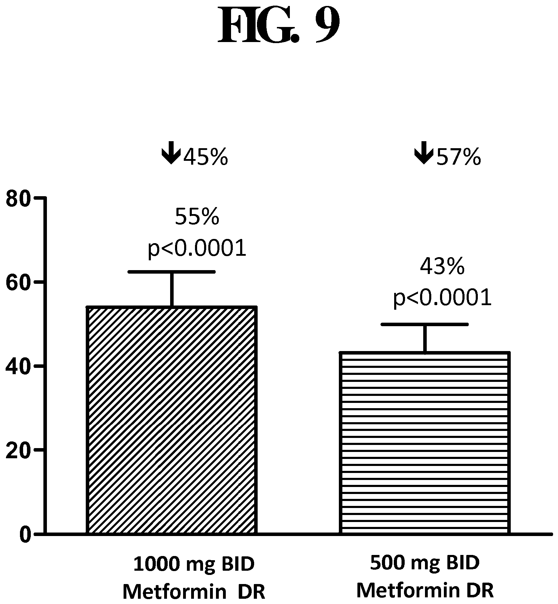

Table 7 and Figure 9 present the relative bioavailability of metformin by treatment versus Metformin IR at Day 5. Compared to the Metformin IR formulation the metformin exposure from t = 0 to time of last concentration after study medication administration (AUC₀₋ₜ) was statistically significantly reduced by 45.2% with 1000 mg Metformin DR (% mean ratio of 54.8; p<0.0001) and 56.6% with 500 mg Metformin DR (% mean ratio of 43.4; p<0.0001). Compared to Metformin IR, Cₘₐₓ was also was statistically significantly reduced by 34.9% with 1000 mg Metformin DR (% mean ratio of 65.1; p<0.0001) and 47.7% with 500 mg metformin DR (% mean ratio of 52.3; p<0.0001).

The Metformin DR + IR treatment resulted in exposures similar to that of the 1000 mg Metformin IR (% mean ratio of 90.9; p=0.2271) despite an increase in daily dose of 50%.

**Table 7. Relative Bioavailability of Metformin by Treatment versus Metformin IR at Day 5 - Evaluable Population**

| **Statistic** | | **1000 mg Met IR (N = 19)** | **1000 mg Met DR (N = 19)** | **500 mg Met DR (N = 19)** | **500 mg Met IR + 1000 mg Met DR (N = 19)** |
|---|---|---|---|---|---|
| **AUC₀₋ₜ (ng*h/mL)** | | | | | |
| Geometric LS mean | | 8325 | 4559 | 3614 | 7567 |

| % ratio [1] | | | | | |
|---|---|---|---|---|---|
| | Geometric LS mean | NA | 54.8 | 43.4 | 90.9 |
| | 90% CI | NA | 48.1,62.4 | 38.1,49.5 | 79.8,103.6 |
| | p value | NA | <0.0001 | <0.0001 | 0.2271 |

| **Cmax₀₋ₜ (ng/mL)** | | | | | |
|---|---|---|---|---|---|
| Geometric LS mean | | 1283 | 836 | 671 | 1150 |

| % ratio [1] | | | | | |
|---|---|---|---|---|---|
| | Geometric LS mean | NA | 65.1 | 52.3 | 89.6 |
| | 90% CI of % ratio | NA | 56.5, 75.0 | 45.4,60.3 | 77.8, 103.3 |
| | p value of % ratio | NA | <0.0001 | <0.0001 | 0.2016 |

| | | | | | |
|---|---|---|---|---|---|
| Abbreviations: NA = not applicable; t= last quantifiable concentration following dose administration. Note: Intra subject CV% was 24.2 for AUC₀₋ₜ and 26.3 for Cₘₐₓ. [1] (1000 mg Met IR, 1000 mg Met DR, or 500 mg Met DR) / 1000 mg Met IR. | | | | | |

### Pharmacodynamic Evaluations

### PYY Total

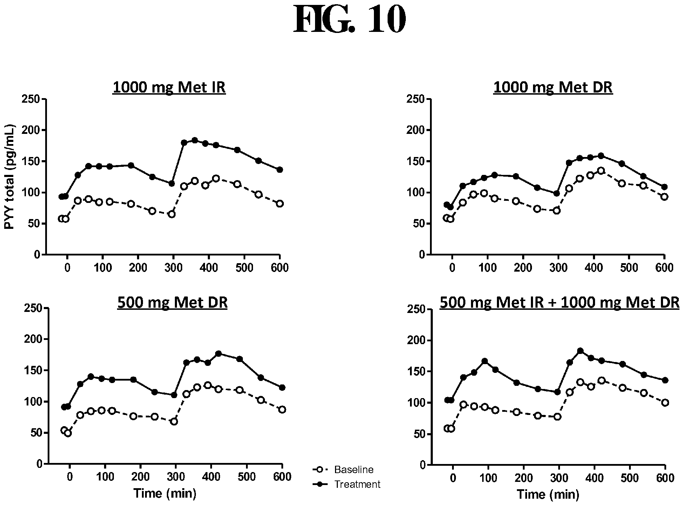

Figure 10 and Table 8 present the mean plasma PYY total concentration profiles at baseline and Day 5 by treatment and time point and the corresponding analysis of pharmacodynamic parameters, respectively. Baseline plasma PYY total concentrations were similar between treatments at most time points. Additionally, all metformin treatments statistically significantly increased PYY total exposure and peak concentrations (p<0.01 for all), with percent ratios (Day5/Day1) for AUC₀₋ₜ and Cmax ranging from 1.26 to 1.55. Fasting plasma PYY total concentrations were also statistically significantly increased from baseline at Day 5 for each treatment (Table 9, p<0.01 for all). These results indicate that all of the treatments studied elicited similar PYY total responses to two standardized meals.

**Table 8. Pharmacodynamic Analysis of Plasma PYY Total (pg/mL) - Within-Treatment Comparison Based on Ratios - Evaluable Population**

| **Statistic** | | **1000 mg Met IR (N = 19)** | **1000 mg Met DR (N = 19)** | **500 mg Met DR (N = 19)** | **500 mg Met IR + 1000 mg Met DR (N = 19)** |
|---|---|---|---|---|---|
| **AUC₀₋ₜ (pg/mL*min)** | | | | | |
| BL geo. LS mean (SE) | | 51487 (5104) | 51518 (5579) | 50932 (5587) | 51985 (5614) |
| EOT geo. LS mean (SE) | | 79654 (7897) | 71218 (7712) | 74546 (8178) | 77270 (8344) |

| % ratio [1] | | | | | |
|---|---|---|---|---|---|
| | Geo. LS mean (SE) | 1.55 (0.09) | 1.38 (0.09) | 1.46 (0.06) | 1.49 (0.06) |
| | 95% CI | 1.36, 1.75 | 1.22, 1.57 | 1.34, 1.59 | 1.36, 1.62 |
| | p value | <0.0001 | <0.0001 | <0.0001 | <0.0001 |

| **Cmax₀₋ₜ (pg/mL)** | | | | | |
|---|---|---|---|---|---|
| BL geo. LS mean (SE) | | 124 (13) | 135 (16) | 122 (13) | 129 (15) |
| EOT geo. LS mean (SE) | | 190 (19) | 169 (20) | 169 (18) | 184 (21) |

| % ratio [1] | | | | | |
|---|---|---|---|---|---|
| | Geo. LS mean (SE) | 1.53 (0.10) | 1.26 (0.09) | 1.38 (0.08) | 1.43 (0.06) |
| | 95% CI | 1.34,1.75 | 1.08,1.47 | 1.23,1.55 | 1.31,1.56 |
| | p value | <0.0001 | 0.0056 | <0.0001 | <0.0001 |

| | | | | | |
|---|---|---|---|---|---|
| Abbreviations: BL = baseline (Day 1); EOT = end of treatment (Day 5); geo. = geometric; t= last quantifiable concentration following dose administration. [1] EOT (Day 5) / BL (Day 1) for each treatment | | | | | |

**Table 9. Fasting Plasma PYY Total (pg/mL) at Baseline and Day 5 - Evaluable Population**

| **Statistic** | | **1000 mg Met IR (N = 19)** | **1000 mg Met DR (N = 19)** | **500 mg Met DR (N = 19)** | **500 mg Met IR + 1000 mg Met DR (N = 19)** |
|---|---|---|---|---|---|
| BL LS mean (SE) | | 59.47 (10.22) | 56.26 (8.32) | 53.39 (11.42) | 59.11 (12.90) |
| EOT LS mean (SE) | | 94.75 (10.22) | 75.80 (8.32) | 91.13 (11.42) | 92.92 (12.90) |
| | LS mean diff (SE) | 35.28 (6.64) | 19.53 (6.17) | 37.73 (10.41) | 33.81 (9.91) |
| | 95% CI | 21.28, 49.28 | 6.51,32.56 | 15.77, 59.69 | 12.90, 54.71 |
| | p value | <.0001 | 0.0057 | 0.0021 | 0.0033 |

| | | | | | |
|---|---|---|---|---|---|
| Abbreviations: BL = baseline (Day 1); EOT = end of treatment (Day 5). | | | | | |

### GLP-1 Active

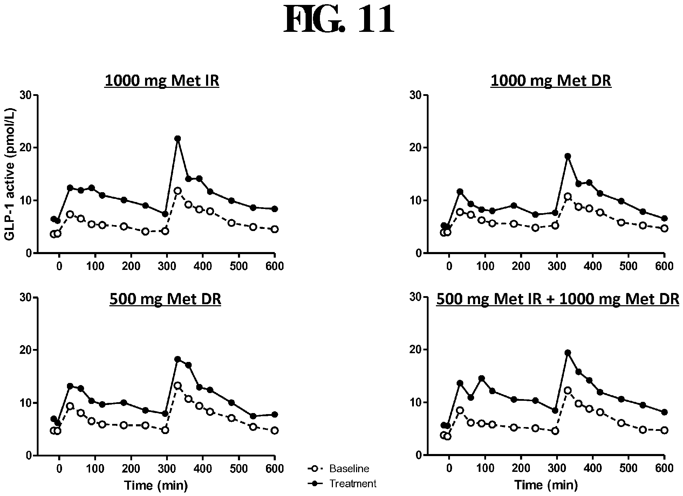

Figure 11 and Table 10 present the mean plasma GLP-1 active concentration profiles at baseline and Day 5 by treatment and time point and the corresponding analysis of pharmacodynamic parameters, respectively. Baseline plasma GLP-1 active concentrations were similar between treatments at most time points. Additionally, all metformin treatments statistically significantly increased GLP-1 active exposure and peak concentrations (p<0.01 for all), with percent ratios (Day5/Day1) for AUC0-t and Cmax ranging from 1.42 to 1.88. Fasting plasma GLP-1 total concentrations were also statistically significantly increased from baseline at Day 5 for each treatment (Table 11, p<0.05 for all). These results indicate that all of the treatments studied elicited similar GLP-1 active responses to two standardized meals.

**Table 10 Pharmacodynamic Analysis of Plasma GLP-1 Active (pmol/L) - Within-Treatment Comparison Based on Ratios - Evaluable Population**

| **Statistic** | | **1000 mg Met IR (N = 19)** | **1000 mg Met DR (N = 19)** | **500 mg Met DR (N = 19)** | **500 mg Met IR + 1000 mg Met DR (N = 19)** |
|---|---|---|---|---|---|
| **AUC₀₋ₜ (pmol/L*min)** | | | | | |
| BL geo. LS mean (SE) | | 3031 (386) | 3059 (405) | 3547 (447) | 3277 (380) |
| EOT geo. LS mean (SE) | | 5655 (719) | 4953 (655) | 5993 (755) | 6158 (714) |

| % ratio [1] | | | | | |
|---|---|---|---|---|---|
| | Geo. LS mean (SE) | 1.87 (0.18) | 1.62 (0.11) | 1.69 (0.15) | 1.88 (0.19) |
| | 95% CI | 1.52,2.29 | 1.40, 1.87 | 1.41,2.03 | 1.52,2.33 |
| | p value | <0.0001 | <0.0001 | <0.0001 | <0.0001 |

| **Cmax₀₋ₜ (pmol/L)** | | | | | |
|---|---|---|---|---|---|
| BL geo. LS mean (SE) EOT geo. LS mean (SE) | | 11.3 (1.4) | 10.6 (1.3) | 13.9 (1.5) | 12.0 (1.3) |
| | | 19.2 (2.3) | 17.3 (2.1) | 19.7 (2.1) | 21.1 (2.3) |

| % ratio [1] | | | | | |
|---|---|---|---|---|---|
| | Geo. LS mean (SE) | 1.70 (0.16) | 1.64 (0.17) | 1.42 (0.14) | 1.76 (0.19) |
| | 95% CI | 1.40,2.07 | 1.32, 2.03 | 1.15, 1.76 | 1.40,2.21 |
| | p value | <0.0001 | 0.0001 | 0.0025 | <0.0001 |

| | | | | | |
|---|---|---|---|---|---|
| Abbreviations: BL = baseline (Day 1); EOT = end of treatment (Day 5); geo. = geometric; t= last quantifiable concentration following dose administration. [1] EOT (Day 5) / BL (Day 1) -1 for each treatment. | | | | | |

**Table 11 Fasting Plasma GLP-1 Active (pmol/L) at Baseline and Day 5 - Evaluable Ponulation**

| **Statistic** | | **1000 mg Met IR (N = 19)** | **1000 mg Met DR (N = 19)** | **500 mg Met DR (N = 19)** | **500 mg Met IR + 1000 mg Met DR (N = 19)** |
|---|---|---|---|---|---|
| BL LS mean (SE) | | 3.79 (1.16) | 3.93 (1.19) | 4.73 (1.31) | 3.69 (1.04) |
| EOT LS mean (SE) | | 6.32 (1.16) | 5.10 (1.19) | 6.62 (1.31) | 5.64 (1.04) |
| | LS mean diff (SE) | 2.53 (0.83) | 1.17 (0.54) | 1.89 (0.45) | 1.95 (0.91) |
| | 95% CI | 0.80, 4.26 | 0.03, 2.31 | 0.96, 2.83 | 0.03, 3.87 |
| | p value | 0.0067 | 0.0444 | 0.0005 | 0.0466 |

| | | | | | |
|---|---|---|---|---|---|
| Abbreviations: BL = baseline (Day 1); EOT = end of treatment (Day 5). | | | | | |

### Glucose

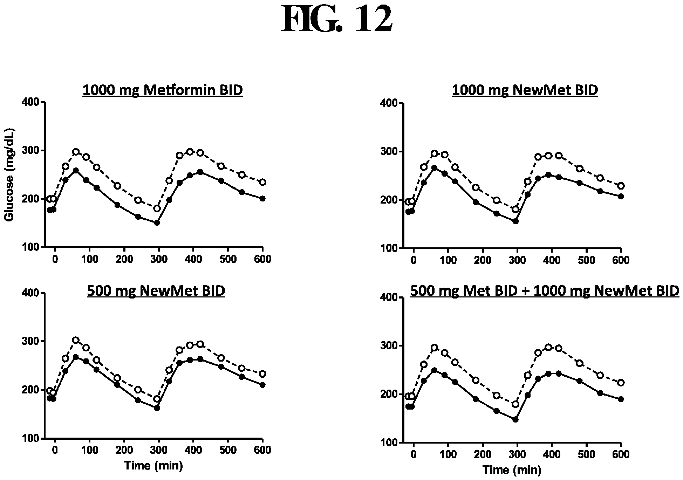

Figure 12 and Table 12 present mean plasma glucose concentration profiles at baseline and Day 5 by treatment and timepoint and the corresponding pharmacodynamic parameters by meal, respectively.

Baseline plasma glucose concentrations were similar between treatments at most time points. Additionally, all metformin treatments statistically significantly decreased glucose exposure and peak concentrations for both meal intervals to a similar extent (p<0.001 for all).

**Table12 Pharmacodynamic Analysis of Plasma Glucose (mg/dL) by Meal Interval - Within-Treatment Comparison Based on Ratios - Evaluable Population**

| **Statistic** | | **1000 mg Met IR (N = 19)** | **1000 mg Met DR (N = 19)** | **500 mg Met DR (N = 19)** | **500 mg Met IR + 1000 mg Met DR (N = 19)** |
|---|---|---|---|---|---|
| ***Breakfast Interval*** | | | | | |
| **AUC₀₋ₜ₂₉₅ (mg/dL*min)** | | | | | |
| BL geo. LS mean (SE) | | 66642 (5480) | 66257 (5815) | 65755 (5906) | 66507 (5617) |
| EOT geo. LS mean (SE) | | 57007 (4688) | 59269 (5201) | 60346 (5420) | 56658 (4785) |

| % ratio [1] | | | | | |
|---|---|---|---|---|---|
| | Geo. LS mean (SE) | 0.86 (0.02) | 0.90 (0.02) | 0.92 (0.01) | 0.85 (0.02) |
| | 95% CI | 0.81,0.91 | 0.86,0.93 | 0.89,0.95 | 0.81, 0.90 |
| | p value | <0.0001 | <0.0001 | <0.0001 | <0.0001 |

| **Cmax₀₋ₜ₂₉₅ (mg/dL)** | | | | | |
|---|---|---|---|---|---|
| BL geo. LS mean (SE) EOT geo. LS mean (SE) | | 291 (21) | 290 (22) | 292 (24) | 290 (20) |
| | | 255 (19) | 261 (20) | 263 (21) | 248 (17) |

| % ratio [1] | | | | | |
|---|---|---|---|---|---|
| | Geo. LS mean (SE) | 0.88 (0.02) | 0.90 (0.02) | 0.90 (0.01) | 0.85 (0.02) |
| | 95% CI | 0.83,0.92 | 0.86,0.95 | 0.88,0.93 | 0.81, 0.90 |
| | p value | <0.0001 | 0.0004 | <0.0001 | <0.0001 |

| ***Lunch Interval*** | | | | | |
|---|---|---|---|---|---|
| **AUCₜ₂₉₅₋ₜ (pg/mL*min)** | | | | | |
| BL geo. LS mean (SE) | | 76286 (6051) | 75132 (6199) | 74566 (6634) | 74799 (5972) |
| EOT geo. LS mean (SE) | | 65558 (5200) | 66330 (5473) | 68480 (6093) | 63495 (5070) |

| % ratio [1] | | | | | |
|---|---|---|---|---|---|
| | Geo. LS mean (SE) | 0.86 (0.02) | 0.88 (0.02) | 0.92 (0.02) | 0.85 (0.03) |
| | 95% CI | 0.82,0.91 | 0.85,0.92 | 0.88,0.95 | 0.79, 0.91 |
| | p value | <0.0001 | <0.0001 | 0.0002 | 0.0001 |

| **Cmax**_{**t295**-t} **(pg/mL)** | | | | | |
|---|---|---|---|---|---|
| BL geo. LS mean (SE) | | 295 (22) | 288 (21) | 287 (23) | 293 (22) |
| EOT geo. LS mean (SE) | | 250 (19) | 255 (19) | 265 (22) | 245 (19) |

| % ratio [1] | | | | | |
|---|---|---|---|---|---|
| | Geo. LS mean (SE) | 0.85 (0.03) | 0.89 (0.02) | 0.93 (0.02) | 0.84 (0.03) |
| | 95% CI | 0.80, 0.90 | 0.85, 0.92 | 0.89, 0.96 | 0.78, 0.90 |
| | p value | <0.0001 | <0.0001 | 0.0002 | <0.0001 |

| | | | | | |
|---|---|---|---|---|---|
| Abbreviations: BL = baseline (Day 1); EOT = end of treatment (Day 5); t= last quantifiable concentration following dose administration. [1] EOT (Day 5) / BL (Day 1) for each treatment. | | | | | |

Table 13 presents the pharmacodynamic parameters for glucose from t = 0 to time of last concentration after study medication administration. Consistent with the pharmacodynamic parameters for the breakfast and lunch intervals, all metformin treatments statistically significantly decreased glucose exposure and peak concentrations (p<0.001 for all), with percent ratios (Day5/Day1) for AUC₀₋ₜ and Cmax ranging from 0.84 to 0.92.

**Table13 Pharmacodynamic Analysis of Plasma Glucose (mg/dL) and Insulin (pmol/L) - Within-Treatment Comparison Based on Ratios - Evaluable Population**

| **Statistic** | | **1000 mg Met IR (N = 19)** | **1000 mg Met DR (N = 19)** | **500 mg Met DR (N = 19)** | **500 mg Met IR + 1000 mg Met DR (N = 19)** |
|---|---|---|---|---|---|
| ***Glucose*** | | | | | |
| **AUC₀₋ₜ (mg/dL*min)** | | | | | |
| BL geo. LS mean (SE) | | 143041 (11408) | 141572 (11884) | 140503 (12403) | 141502 (11477) |
| EOT geo. LS mean (SE) | | 122748 (9789) | 125742 (10556) | 129029 (11390) | 120255 (9754) |

| % ratio [1] | | | | | |
|---|---|---|---|---|---|
| | Geo. LS mean (SE) | 0.86 (0.02) | 0.89 (0.01) | 0.92 (0.01) | 0.85 (0.02) |
| | 95% CI | 0.82,0.90 | 0.86,0.92 | 0.89,0.95 | 0.80, 0.90 |
| | p value | <0.0001 | <0.0001 | <0.0001 | <0.0001 |

| **Cmax₀₋ₜ (mg/dL)** | | | | | |
|---|---|---|---|---|---|
| BL geo. LS mean (SE) | | 301 (22) | 301 (22) | 301 (24) | 304 (22) |
| EOT geo. LS mean (SE) | | 265 (19) | 269 (19) | 277 (22) | 256 (19) |

| % ratio [1] | | | | | |
|---|---|---|---|---|---|
| | Geo. LS mean (SE) | 0.88 (0.03) | 0.89 (0.02) | 0.92 (0.01) | 0.84 (0.02) |
| | 95% CI | 0.83,0.93 | 0.86,0.93 | 0.90,0.95 | 0.79,0.90 |
| | p value | 0.0002 | <0.0001 | <0.0001 | <0.0001 |

| ***Insulin*** | | | | | |
|---|---|---|---|---|---|
| **AUC₀₋ₜ (pmol/L*min)** | | | | | |
| BL geo. LS mean (SE) | | 191826 (26987) | 176384 (30776) | 199339 (28758) | 191204 (26683) |
| EOT geo. LS mean (SE) | | 186379 (26145) | 175190 (30567) | 194650 (28049) | 184975 (25814) |

| % ratio [1] | | | | | |
|---|---|---|---|---|---|
| | Geo. LS mean (SE) | 0.97 (0.05) | 0.99 (0.04) | 0.98 (0.03) | 0.97 (0.04) |
| | 95% CI | 0.88, 1.08 | 0.92, 1.08 | 0.91, 1.04 | 0.89, 1.05 |
| | p value | 0.5587 | 0.8622 | 0.4551 | 0.4070 |

| **Cmax₀₋ₜ (pmol/L)** | | | | | |
|---|---|---|---|---|---|
| BL geo. LS mean (SE) | | 594 (88) | 664 (112) | 604 (96) | 598 (92) |
| EOT geo. LS mean (SE) | | 539 (80) | 586 (99) | 578 (92) | 539 (83) |

| % ratio [1] | | | | | |
|---|---|---|---|---|---|
| | Geo. LS mean (SE) | 0.91 (0.06) | 0.88 (0.09) | 0.96 (0.06) | 0.90 (0.06) |
| | 95% CI | 0.79, 1.04 | 0.72, 1.08 | 0.85, 1.08 | 0.79, 1.03 |
| | p value | 0.1462 | 0.2167 | 0.4649 | 0.1110 |

| | | | | | |
|---|---|---|---|---|---|
| Abbreviations: BL = baseline (Day 1); EOT = end of treatment (Day 5); t= last quantifiable concentration following dose administration. [1] EOT (Day 5) / BL (Day 1) for each treatment. | | | | | |

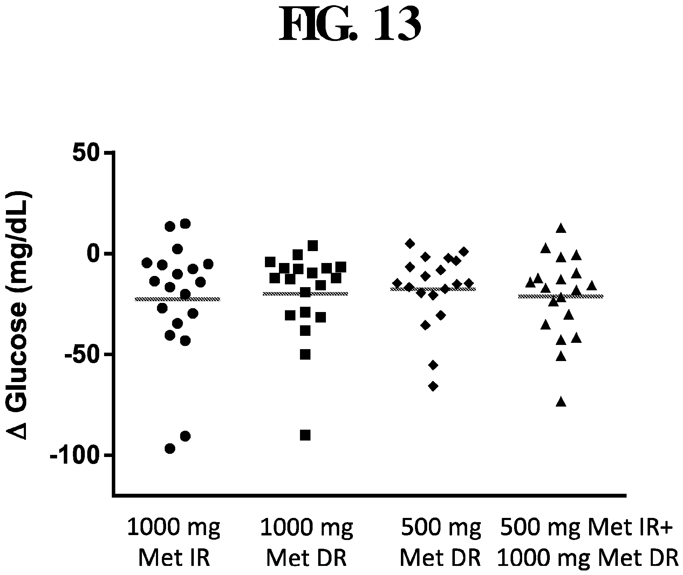

Table 14 presents the LS mean (SE) and Figure 13 presents the individual change in fasting plasma glucose concentrations from baseline to Day 5 by treatment. Baseline fasting glucose concentrations were similar and ranged from 196 mg/dL to 200 mg/dL among the treatment groups. All treatment groups achieved statistically significant reductions (p<0.01 for all) in fasting plasma glucose after 5 days of treatment. As shown in Figure 13, the LSM and distribution of individual responses were similar between treatment groups.

**Table 14. Fasting Plasma Glucose (mg/dL) at Baseline and Day 5 - Evaluable Population**

| **Statistic** | | **1000 mg Met IR (N = 19)** | **1000 mg Met DR (N = 19)** | **500 mg Met DR (N = 19)** | **500 mg Met IR + 1000 mg Met DR (N = 19)** |
|---|---|---|---|---|---|
| BL LS mean (SE) | | 200.3 (16.2) | 197.0 (16.7) | 198.7 (17.4) | 195.9 (15.4) |
| EOT LS mean (SE) | | 177.8 (16.2) | 177.1 (16.7) | 182.2 (17.4) | 174.7 (15.4) |
| | LS mean diff (SE) | -22.5 (6.8) | -19.9 (5.0) | -16.4 (3.8) | -21.2 (4.7) |
| | 95% CI | -36.8, -8.16 | -30.5, -9.3 | -24.5, -8.4 | -31.1, -11.2 |
| | p value | 0.0040 | 0.0009 | 0.0004 | 0.0003 |

| | | | | | |
|---|---|---|---|---|---|
| Abbreviations: BL = baseline (Day 1); EOT = end of treatment (Day 5). | | | | | |

### Insulin

Tables 15 and 16 present the pharmacodynamic parameters for insulin and baseline and Day 5 fasting plasma insulin concentrations, respectively. There were no statistically significant changes in insulin exposure, peak concentrations, or fasting concentrations for any of the treatments (p>0.05 for all). Maintenance of insulin concentrations despite the lower circulating glucose concentrations is indicative of an incretin effect.

**Table 15 Pharmacodynamic Analysis of Insulin (pmol/L) - Within-Treatment Comparison Based on Ratios - Evaluable Population**

| **Statistic** | | **1000 mg Met IR (N = 19)** | **1000 mg Met DR (N = 19)** | **500 mg Met DR (N = 19)** | **500 mg Met IR + 1000 mg Met DR (N = 19)** |
|---|---|---|---|---|---|
| **AUC₀₋ₜ (pmol/L*min)** | | | | | |
| BL geo. LS mean (SE) | | 191826 (26987) | 176384 (30776) | 199339 (28758) | 191204 (26683) |
| EOT geo. LS mean (SE) | | 186379 (26145) | 175190 (30567) | 194650 (28049) | 184975 (25814) |

| % ratio [1] | | | | | |
|---|---|---|---|---|---|
| | Geo. LS mean (SE) | 0.97 (0.05) | 0.99 (0.04) | 0.98 (0.03) | 0.97 (0.04) |
| | 95% CI | 0.88, 1.08 | 0.92, 1.08 | 0.91, 1.04 | 0.89, 1.05 |
| | p value | 0.5587 | 0.8622 | 0.4551 | 0.4070 |

| **Cmax₀₋ₜ (pmol/L)** | | | | | |
|---|---|---|---|---|---|
| BL geo. LS mean (SE) | | 594 (88) | 664 (112) | 604 (96) | 598 (92) |
| EOT geo. LS mean (SE) | | 539 (80) | 586 (99) | 578 (92) | 539 (83) |

| % ratio [1] | | | | | |
|---|---|---|---|---|---|
| | Geo. LS mean (SE) | 0.91 (0.06) | 0.88 (0.09) | 0.96 (0.06) | 0.90 (0.06) |
| | 95% CI | 0.79, 1.04 | 0.72, 1.08 | 0.85, 1.08 | 0.79, 1.03 |
| | p value | 0.1462 | 0.2167 | 0.4649 | 0.1110 |

| | | | | | |
|---|---|---|---|---|---|
| Abbreviations: BL = baseline (Day 1); EOT = end of treatment (Day 5); t= last quantifiable concentration following dose administration. [1] EOT (Day 5) / BL (Day 1) for each treatment. | | | | | |

**Table 16. Fasting Insulin (pmol/L) at Baseline and Day 5 - Evaluable Population**

| **Statistic** | | **1000 mg Met IR (N = 19)** | **1000 mg Met DR (N = 19)** | **500 mg Met DR (N = 19)** | **500 mg Met IR + 1000 mg Met DR (N = 19)** |
|---|---|---|---|---|---|
| BL LS mean (SE) | | 183.8 (42.3) | 187.7 (29.0) | 166.7 (34.5) | 169.8 (29.9) |
| EOT LS mean (SE) | | 151.9 (42.3) | 138.1 (29.0) | 157.8 (34.5) | 147.0 (29.9) |
| | LS mean diff (SE) | -31.8 (30.8) | -49.6 (18.1) | -8.8 (13.2) | -22.8 (8.6) |
| | 95% CI | -96.5, 32.8 | -87.7, -11.6 | -36.6, 18.9 | -40.8, -4.8 |
| | p value | 0.3146 | 0.0135 | 0.5109 | 0.0160 |

| | | | | | |
|---|---|---|---|---|---|
| Abbreviations: BL = baseline (Day 1); EOT = end of treatment (Day 5). | | | | | |

### Safety Evaluations

Table 17 summarizes treatment-emergent adverse events by SOC, preferred term, and most recent treatment at onset.

Consistent with the metformin prescribing information, adverse events were primarily gastrointestinal in nature with nausea, vomiting, and retching occurring only in the treatment groups receiving Metformin IR with or without Metformin DR. Diarrhea was reported across all treatment groups and appeared to be dose-dependent with the greatest incidence with Metformin IR + Metformin DR (7 subjects, 33.3%) and the lowest incidence with the lowest dose of Metformin DR (2 subjects, 10.0%). Of note, all gastrointestinal events in the 500 mg Metformin DR group occurred during the post-treatment washout period while off study drug.

Nervous system disorders such as dizziness and headache were also more frequent with Metformin IR than either DR dosage. Overall, fewer gastrointestinal and nervous system disorder adverse events were reported with the Metformin DR than metformin IR, indicating that the reduced systemic exposure to metformin achieved by bypassing the proximal small intestine improved tolerability.

**Table 17 Summary of Treatment-Emergent Adverse Events by SOC and Preferred Term and Treatment at Onset - ITT Population**

| **SOC Preferred Term** | | **1000 mg Met IR (N = 22) n (%)** | **1000 mg Met DR (N = 20) n (%)** | **500 mg Met DR (N = 20) n (%)** | **500 mg Met IR + 1000 mg Met DR (N = 21) n (%)** |
|---|---|---|---|---|---|
| **Any TEAE** | | **6 (27.3)** | **5 (25.0)** | **4 (20.0)** | **10 (47.6)** |
| **Gastrointestinal Disorders** | | **5 (22.7)** | **3 (15.0)** | **2 (10.0)** | **8 (38.1)** |
| | Abdominal Discomfort | 0 (0) | 0 (0) | 0 (0) | 1 (4.8) |
| | Abdominal Distension | 0 (0) | 0 (0) | 0 (0) | 1 (4.8) |
| | Abdominal Pain | 0 (0) | 0 (0) | 1 (5.0) | 1 (4.8) |
| | Diarrhea | 3 (13.6) | 3 (15.0) | 2 (10.0) | 7 (33.3) |
| | Dyspepsia | 1 (4.5) | 0 (0) | 1 (5.0) | 1 (4.8) |
| | Frequent Bowel | 0 (0) | 0 (0) | 0 (0) | 1 (4.8) |
| | Movements | | | | |
| | Nausea | 2 (9.1) | 0 (0) | 0 (0) | 3 (14.3) |
| | Retching | 1 (4.5) | 0 (0) | 0 (0) | 0 (0) |
| | Vomiting | 2(9.1) | 0 (0) | 0(0) | 0(0) |
| **General Disorders And Administration Site Conditions** | | **0 (0)** | **0(0)** | **1 (5.0)** | **0 (0)** |
| | Fatigue | 0 (0) | 0 (0) | 1 (5.0) | 0 (0) |
| **Infections And Infestations** | | **0 (0)** | **0(0)** | **0 (0)** | **1 (4.8)** |
| | Oral Herpes | 0 (0) | 0 (0) | 0 (0) | 1 (4.8) |
| **Investigations** | | **0 (0)** | **0(0)** | **0 (0)** | **1 (4.8)** |
| | Weight Decreased | 0 (0) | 0 (0) | 0 (0) | 1 (4.8) |
| **Musculoskeletal And Connective Tissue Disorders** | | **0 (0)** | **1 (5.0)** | **0 (0)** | **0 (0)** |
| | Pain In Extremity | 0 (0) | 1 (5.0) | 0 (0) | 0 (0) |
| **Neoplasms Benign, Malignant And Unspecified (Incl Cysts And Polyps)** | | **0 (0)** | **1 (5.0)** | **0 (0)** | **0 (0)** |
| | Gastrointestinal | 0 (0) | 1 (5.0) | 0 (0) | 0 (0) |
| | Stromal Tumour | | | | |
| **Nervous System Disorders** | | **5 (22.7)** | **1 (5.0)** | **1 (5.0)** | **0 (0)** |
| | Dizziness | 3 (13.6) | 0 (0) | 0 (0) | 0 (0) |
| | Headache | 2 (9.1) | 1 (5.0) | 1 (5.0) | 0 (0) |
| | Sinus Headache | 1 (4.5) | 0 (0) | 0 (0) | **0 (0)** |
| **Renal And Urinary Disorders** | | **0 (0)** | **0(0)** | **0 (0)** | **1 (4.8)** |
| | Pollakiuria | 0 (0) | 0 (0) | 0 (0) | 1 (4.8) |
| **Skin And Subcutaneous Tissue Disorders** | | **0 (0)** | **0(0)** | **0 (0)** | **1 (4.8)** |
| | Hyperhidrosis | 0 (0) | 0 (0) | 0 (0) | 1 (4.8) |

### EXAMPLE 2.3: Discussion

In this study, metformin concentrations in plasma were measured over 11 hours at steady-state on the 5th day (Figure 1) of BID dosing (pre-breakfast and pre-supper) with 1000 mg immediate-release metformin (Metformin IR), 500 mg Metformin DR and 1000 mg Metformin DR, or a combination of 500 mg Metformin IR and 1000 mg Metformin DR. All subjects had type 2 diabetes and received each treatment in a randomized crossover design with a one week washout between treatments.

The observed profiles indicated lower circulating amounts of metformin when using the Metformin DR compared to Metformin IR. The Day 5 pre-dose concentration of metformin with Metformin IR on the morning of Day 5 was 350 ng/mL, which is consistent with steady-state trough concentrations published in the literature. After the administration of Metformin IR on the morning of Day 5, there was a rapid increase in metformin concentration that peaked 90 min after the dose followed by a steady decline for the remainder of the sampling period.

With Metformin DR dosing, the highest concentration of metformin was observed prior to the dose on the morning of Day 5, which was approximately 2 times higher at that time point than those for Metformin IR. Following administration of either dose of Metformin DR, there was a decrease in metformin concentration for the first 240 minutes followed by a small rise in metformin concentration at 360 minutes, which plateaued for the remainder of the sampling period. The entire 11-hour Metformin DR PK profiles remained below the pre-dose concentrations measured at t=0. These results indicate that the absorption profiles for Metformin DR dosing with the evening meal were slowed relative to doses administered with the breakfast meal, consistent with slowed intestinal transit during the sleeping hours. Thus, concentrations throughout the first 240 minutes of the Day 5 profile were predominantly a result of absorption from the Day 4 evening dose and concentrations from 240 minutes through 660 mins were predominantly a result of absorption from the Day 5 morning dose.

### EXAMPLE 3: ANALYSIS OF PHARMACOKINETIC DIFFERENCES BETWEEN MORNING AND EVENING DOSING

To better characterize the pharmacokinetic differences between morning and evening doses, the study of Example 3 was designed to obtain 36-hour PK profiles of Metformin DR at doses of 500 and 1000 mg given at the evening and breakfast meals in healthy subjects. Subjects also received 1000 mg Metformin IR with the evening and breakfast meals and 2000 mg metformin extended-release (Metformin XR) with the evening meal during separate treatment periods. All subjects received each treatment in a randomized crossover design with a one week washout between treatments.

The metformin DR formulation was a US-supplied commercially available film-coated immediate-release tablet containing 500 mg metformin hydrochloride, to which additional coatings (a seal coating and an enteric coating) were applied in order to delay release of the drug in the GI tract until the tablet reaches a pH 6.5 region of the distal small intestine. The seal coating was applied at a nominal level of 2% of the core tablet weight and the enteric coating was applied at a nominal level of 3.8% of the core tablet weight. The tablets are white, biconvex, circular-shaped coated tablets, each containing 500 mg metformin hydrochloride. Inactive ingredients in the commercially available tablet included povidone, magnesium stearate, hypromellose, and polyethylene glycol. Inactive ingredients in the additional coating systems included hypromellose, triacetin, talc, methacrylic acid copolymer (Eudragit^{®} L30 D-55), poly(methyl acrylate-co-methyl methacrylate-co-methacrylic acid) 7:3:1 (Eudragit^{®} FS 30 D), sodium lauryl sulfate, polysorbate 80, glyceryl monostearate, and triethyl citrate. The metformin IR and metformin XR formulations were commercially available formulations (Aurobindo Pharma Limited and Bristol-Myers Squibb respectively) without any modification.

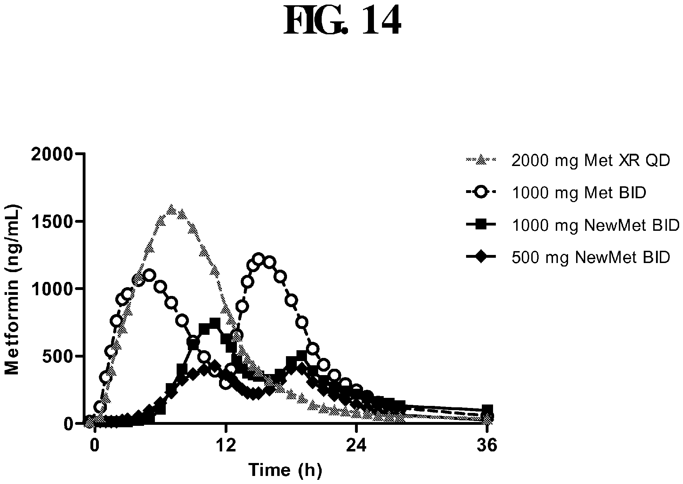

As shown in Figure 14, both doses of Metformin DR resulted in substantially less systemic metformin than was observed with either Metformin IR or Metformin XR. Of note, the total plasma metformin exposure as measured by AUC of 1000 mg Metformin IR BID and 2000 mg Metformin XR QD (total daily doses of 2000 mg) were very similar, consistent with the previously established bioequivalence between the two formulations. The Metformin DR profile over the first 12 hours showed that there is a delay in systemic absorption of Metformin DR, with the first quantifiable plasma concentration occurring approximately 6-7 hours after the dose. The highest concentration was achieved approximately 11 hours after the evening dose. After a second dose with Metformin DR in the morning, the plasma concentration of metformin decreased until approximately 15 h post first dose, followed by a small rise corresponding to approximately 3 hours after the second dose.

As noted above, the data indicate that Metformin IR and both doses of Metformin DR have slightly greater bioavailability after an evening dose than the morning dose, perhaps as a result of slower intestinal transit during the sleeping hours.

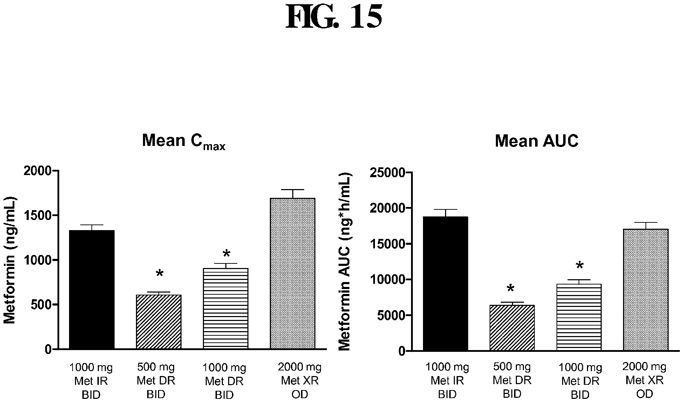

Table 18 shows the Mean (CV%) plasma pharmacokinetic parameters of metformin following oral administration of each treatment and Figure 15 compares the mean (SEM) values of Cₘₐₓ (left panel) and AUC_{0-36 hr} (right panel). Both doses of Metformin DR resulted in substantial reductions in exposure as well as a delay in absorption of 6-7 hours.

**Table 18 Mean (CV%) Plasma Pharmacokinetic Parameters of Metformin Following Oral Administration of Treatment A, B, C, and D - Evaluable Population**

| **PK Parameters** | **1000 mg Met IR BID (Treatment A)** | **500 mg Met DR BID (Treatment B)** | **1000 mg Met DR BID (Treatment C)** | **2000 mg Met XR QD (Treatment D)** |
|---|---|---|---|---|
| N | 19 | 19 | 19 | 19 |
| AUC₀₋₂₄ (ng*h/mL) | 17361 (24.3) | 5541 (31.9) | 7634 (31.9) | 16406 (24.5) |
| AUC₀₋ₜ (ng*h/mL) | 18709 (24.3) | 6164 (32.9) | 9014 (29.5) | 16989 (24.8) |
| AUC_{0-∞} (ng*h/mL) | 19423 (23.6) | 6690 (30.4)^{b} | 10277 (25.6)^{b} | 17398 (24.7) |
| Cₘₐₓ (ng/mL) | 1328 (20.6) | 607 (24.0) | 905 (26.8) | 1688 (25.0) |
| tₘₐₓ^{a} (h) | 15.0 (4.00, 16.0) | 11.0 (6.02, 19.0) | 11.0 (7.00, 19.0) | 7.05 (6.00, 11.0) |
| t_{lag}^{a} (h) | 0.00 (0.00, 0.500) | 6.02 (1.50, 10.0) | 7.00 (3.00, 8.00) | 0.00 (0.00, 2.00) |
| t_{1/2} (h) | 8.26 (31.0) | 6.19 (49.4)^{b} | 11.2 (39.9)^{b} | 6.09 (45.5) |

| | | | | |
|---|---|---|---|---|
| ^{a}median (min, max) ^{b}n=18 ^{c}n=17 | | | | |

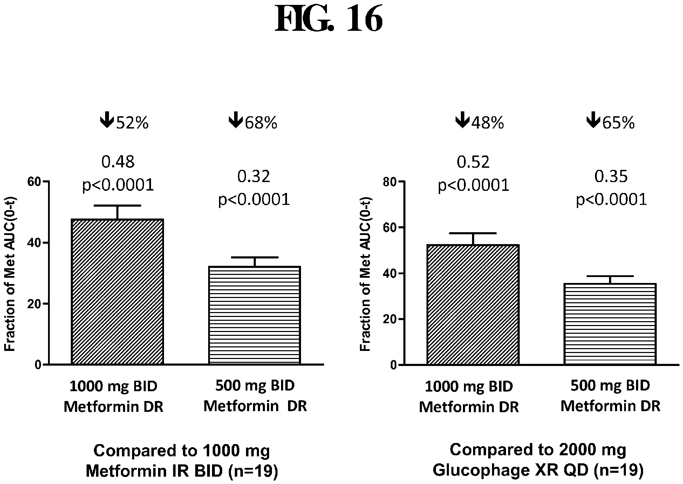

Geometric LSM ratios and 90% confidence intervals for the ln-transformed Cₘₐₓ, AUC₀₋ₜ, and AUC_{0-∞} from the Metformin DR treatments (500 mg BID [Treatment B] and 1000 mg BID [Treatment C]) relative to the Metformin IR (1000 mg BID [Treatment A]) are shown in Table 19 and the relative bioavailability is plotted in the left panel of Figure 16. These results indicate that the rate and extent of exposure (Cₘₐₓ, AUC₀₋ₜ and AUC_{0-∞}) from 500 mg BID Metformin DR were approximately 55%, 68% and 67% lower, respectively, than those from 1000 mg BID Metformin IR. At 1000 mg BID Metformin DR (Treatment C, total daily dose of 2000 mg metformin) the rate and extent of exposure (Cₘₐₓ, AUC₀₋ₜ and AUC_{0-∞}) were approximately 33%, 52% and 47% lower, respectively, than those from 1000 mg BID Metformin IR (Treatment A, total daily dose of 2000 mg metformin). Similar reductions in the rate and extent of exposure were observed when 500 mg BID and 1000 mg BID of Metformin DR were compared to 2000 mg QD of Metformin XR (Table 20; Figure 16, right panel).

**Table 19 Relative Bioavailability of Metformin Following Oral Administration of 500 mg BID and 1000 mg BID Metformin DR Treatment compared to 1000 mg BID Metformin IR- Evaluable Population**

| **PK Parameter** | **Geometric Least-Square Means** | | | **% Ratio of LSmeans (90% CI)** | | **p-value** | |
|---|---|---|---|---|---|---|---|
| | **A** | **B** | **C** | **B/A** | **C/A** | **B/A** | **C/A** |
| **AUC₀₋ₜ (ng*h/mL)** | 18116 | 5816 | 8611 | 32.1 (29.30-35.18) | 47.5 (43.38-52.09) | SS | SS |
| **AUC**_{**0-**∞} **(ng*h/mL)** | 19981 | 6644 | 10586 | 33.3 (30.37-36.40) | 53.0 (48.36-58.05) | SS | SS |
| **Cₘₐₓ (ng/mL)** | 1294 | 586 | 865 | 45.3 (40.88-50.13) | 66.8 (60.34-74.00) | SS | SS |

| | | | | | | | |
|---|---|---|---|---|---|---|---|
| SS: Statistically significant (p-value <0.0001) Treatment A: 1000 mg Metformin IR BID (2 x 500 mg metformin HCl tablets [immediate-release]) Treatment B: 500 mg Metformin DR BID (1 x 500 mg metformin HCl tablet [delayed-release pH 6.5 enteric-coated]) Treatment C: 1000 mg Metformin DR BID (2 x 500 mg metformin HCl tablets [delayed-release pH 6.5 enteric-coated]) | | | | | | | |

**Table 20 Relative Bioavailability of Metformin Following Oral Administration of 500 mg BID and 1000 mg BID Metformin DR Treatment compared to 2000 mg QD Metformin XR - Evaluable Population**

| **PK Parameter** | **Geometric Least-Square Means** | | | **% Ratio of LSmeans (90% CI)** | | **p-value** | |
|---|---|---|---|---|---|---|---|
| | **B** | **C** | **D** | **BID** | **C/D** | **BID** | **C/D** |
| **AUC₀₋ₜ (ng*h/mL)** | 5816 | 8611 | 16450 | 35.4 (32.27-38.74) | 52.3 (47.77-57.36) | SS | SS |
| **AUC**_{**0-**∞} **(ng*h/mL)** | 6644 | 10586 | 17873 | 37.2 (33.93-40.73) | 59.2 (54.10-64.84) | SS | SS |
| **Cₘₐₓ (ng/mL)** | 586 | 865 | 1631 | 35.9 (32.43-39.77) | 53.0 (47.88-58.71) | SS | SS |

| | | | | | | | |
|---|---|---|---|---|---|---|---|
| SS: Statistically significant (p-value <0.0001) Treatment B: 500 mg Metformin DR BID (1 x 500 mg metformin HCl tablet [delayed-release pH 6.5 enteric-coated]) Treatment C: 1000 mg Metformin DR BID (2 x 500 mg metformin HCl tablets [delayed-release pH 6.5 enteric-coated]) Treatment D: 2000 mg Metformin XR QD (4 x 500 mg metformin HCl tablets [extended-release]) | | | | | | | |

Taken together, the pharmacokinetic results of Examples 2 and 3 indicate that delivery of metformin to the lower bowel by administering Metformin DR reduces 24 hour bioavailability by approximately 50% relative to Metformin IR and Metformin XR at the same daily dose. Greater reductions in exposure were observed when the Metformin DR dose was reduced from a total daily dose of 2000 mg to 1000 mg, without a reduction in efficacy. In addition, the time of Metformin DR dosing (with the morning or evening meals) meaningfully affected the timing of metformin release in the intestine (3 vs. 6- 7 hours post-dose, respectively) and provides an explanation for the observation from the study in Example 2 that, the Metformin DR trough values observed prior to the morning dose were higher than the trough values observed 12 hours after the morning dose.

In the Example 2 study, while the systemic exposure to metformin was substantially reduced with Metformin DR (45% with 2000 mg/day and -60% with 1000 mg/day, relative to 2000 mg/day of Metformin IR), the full glucose lowering effects of Metformin IR (2000 mg/day) were maintained. Given that the full glucose lowering effect was observed at both 2000 mg and 1000 mg daily of Metformin DR, lower doses are viable, allowing for more elegant dosage forms than are currently available with existing products (Metformin IR and Metformin XR (i.e., smaller tablets, fully effective fixed dose combinations, once daily dosing). Moreover, unlike Metformin IR, Metformin DR was not associated with any nausea and vomiting at either dose.

### EXAMPLE 4: MODIFICATION OF COAT PERCENTAGE TO IMPROVE PHARMACOKINETICS

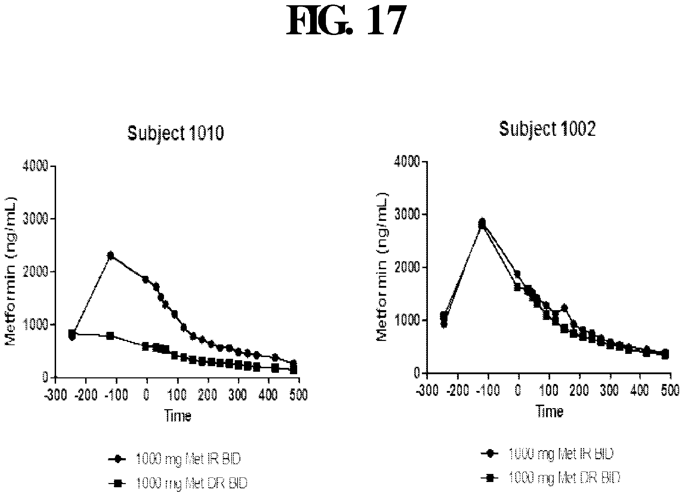

In Example 1, the mean pharmacokinetic profiles demonstrated that the delayed-release metformin formulation resulted in a blunting and a delay in the absorption of metformin following a given dose compared to the immediate-release formulation. However, the individual PK profiles showed that in some cases (3/16), the PK profile on Day 5 of dosing did not differ between 1000 mg BID Metformin DR and 1000 mg BID Metformin IR. This suggested that, in some cases, the enteric coating (at the nominal level of 2.4% of the core tablet weight) was insufficient to prevent release of metformin in the stomach. The left panel of Figure 17 shows a typical example of the Metformin DR and IR PK profiles with a clear blunting and delay of the metformin PK profile following a dose of Metformin DR at t=-240 min. The right panel shows one of the three examples with no delay in release.

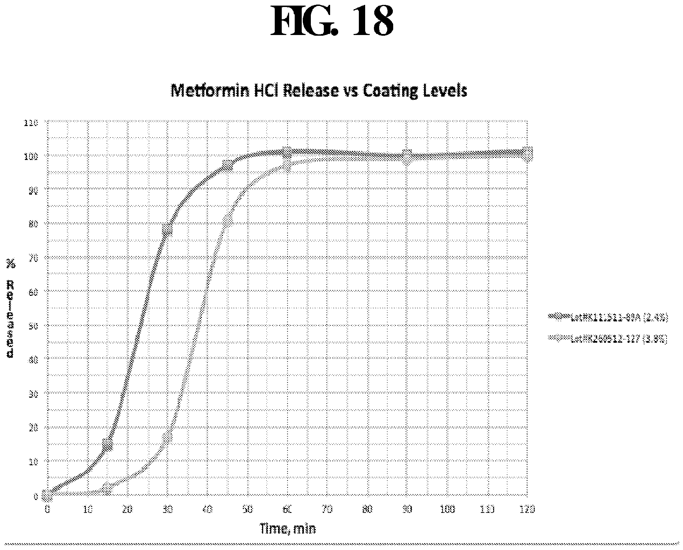

To develop a coating that was more resistant to transient increases in stomach pH that sometimes accompanies meals, the formulation was revised to provide a nominal enteric coating level of 3.8% of the core tablet weight with the same nominal 2% seal coating. The dissolution performance of the tablets was tested using the USP Apparatus 2 (paddles rotating at 50 or 100 rpm). The tablets were enclosed in Japanese Sinkers. For the first 2 h of the test, 0.1M HCl was used as the test medium with a paddle speed of 100 rpm. After 2 h, the test medium was changed to 0.07M phosphate buffer, pH 6.8 with a paddle speed of 50 rpm. Samples were withdrawn at regular intervals from the dissolution vessels and the appearance of metformin hydrochloride was monitored spectrophotometrically. As shown in Figure 18, both the 2.4% and 3.8% nominal DR coat formulations resisted drug release for a period of up to 2 hours in acid. Tablets coated with the DR coatings at a 2.4% level (Batch # K111511-89A) showed a drug release of about 80% at 30 mins and 100 % at 60 mins in pH 6.8 buffer. Tablets coated with the DR coatings at a level of 3.8% (Batch # K260512-127) showed a lag phase in drug release for the first 15 minutes with < 20% release at 30 mins and 100 % at 60 mins.

The revised formulation (3.8% nominal DR coating) was utilized in both Example 2 and Example 3. All subjects (N=38) showed the characteristic delay in peak concentrations consistent with a delayed-release formulation.

### EXAMPLE 5: IN VITRO DISSOLUTION PROFILES OF EXEMPLARY FORMULATIONS ACCORDING TO THE PRESENT INVENTION

Two and three stage *in vitro* dissolution profiles were performed on the formulations as described in Table 21 below and analyzed according to the protocol described in Example 4.

**Table 21: Exemplary formulations used in two and three stage in vitro dissolution analysis**

| Raw Data from Development Report and Halo Manufacturing Summary Report | | | | |
|---|---|---|---|---|
| | Kydes-127 500 mg | Halo-626 500 mg | Halo-625 300mg | Kydes -89A |
| core tablet weight (mg/tablet) | 530.25 | 580.3 | 545 | 527.4 |
| seal coated tablet weight (mg/tablet) | 536.06 | 592 | 556 | 537.4 |
| enteric coated tablet weight (mg/tablet) | 556.55 | 609.7 | 572.6 | 550.35 |

The Kydes-89A formulation was employed in Example 1 above whereas the Kydes-127 formulation was employed in Examples 2 and 3 above. The enteric coating is identical between the Kydes and the Halo-manufactured compositions and is composed of a mixture of two acrylate coating systems, Eudragit^{®} LC 30 D-55 and Eudragit^{®} FS 30 D (Evonik), plus a coating aid material (PlasACRYL^{™}, Emerson Resources) and triethyl citrate.

The Eudragit^{®} L30 D-55 conforms to Methacrylic Acid Copolymer Dispersion, NF and consists of a 30% dispersion of poly(methacrylic acid-co-ethyl acrylate) 1:1 in water, with Sodium Lauryl Sulfate NF 0.7% and Polysorbate 80 NF 2.3% on solid substance as emulsifiers. This polymer provides an enteric coat that is insoluble in acid and designed to dissolve above pH 5.5. The Eudragit^{®} FS 30 D is a 30% dispersion of poly(methyl acrylate co-methyl methacrylate-co-methacrylic acid) 7:3:1 in water, with Sodium Lauryl Sulfate NF 0.3% and Polysorbate 80 NF 1.2% on solid substance as emulsifiers. This polymer provides an enteric coat that is insoluble in acid and designed to dissolve above pH 7. Combinations of the two Eudragits provide dissolution at pHs between 5.5 and 7.0. Earlier work indicated that a 60:40 combination of Eudragit L30 D-55 and FS 30 D would provide tablet dissolution near pH 6.5, corresponding to that expected in the distal small intestine.

The PlasACRYL^{™} T20 is a 20% emulsion of Glyceryl Monostearate NF/Polysorbate 80 NF/ Triethyl Citrate NF in water. It is designed as a plasticizer / anti-tack agent to aid in the application and functionality of enteric coatings such as the Eudragits. Triethyl citrate is commonly used by the pharmaceutical industry in tablet coatings as a plasticizer. The amount of triethyl citrate included in the enteric coating system was based on experience and common industry practice. The amount of PlasACRYL included in the enteric coating system (approximately 10%) was based on manufacturers' recommendations.

The seal coating for the Halo formulations is Opadry^{®} White YS-1-7003 (Colorcon), a mixture of hypromellose, titanium dioxide, polyethylene glycol 400 (macrogol), and polysorbate 80. The hypromellose is the polymeric coating, titanium dioxide is a coloring agent, polyethylene glycol 400 serves as an anticaking agent, and polysorbate 80 is present as a dispersant (in aqueous suspension) and plasticizer. The seal coating on the tablets was changed from Opadry^{®} 03K19229 Clear, which was used on the Kydes formulations, to an opaque white coating to ensure blinding between the various active strengths and placebo tablets. The Opadry^{®} 03K19229 Clear (Colorcon) used in the Kydes formulations is a mixture of hypromellose, triacetin, and talc. The hypromellose is the polymeric coating, triacetin is present as a plasticizer, and the talc is present as an anti-tack agent. A seal coating of 2.0% w/w (on the commercial tablet basis) was chosen for both formulations based on experience and typical pharmaceutical industry practice for tablet seal coatings.

The enteric coating level was reduced to 3.1% w/w (based on core tablet weight) on the Halo manufactured tablets from the 3.8% w/w level previously used at Kydes so that the *in vitro* metformin HCl release profile from the delayed-release tablets from the two manufacturers matched each other. This need for a reduced coating level was attributed to the Halo tablets being smooth-surfaced, as compared to the presence of alphanumeric characters debossed on the Kydes-coated tablets (i.e., on the commercially sourced core tablets). It is hypothesized that the edges of the debossed characters result in thin regions in the enteric coating that result in a faster dissolution profile. Accordingly, debossing, marking or otherwise engraving the face of the core tablet prior to applicaton of the enteric coating needs to be considered and relative levels of enteric coating adjusted accordingly as demonstrated herein.

The target *in vitro* release profile consists of virtually complete protection against dissolution at low pH (0.1 N HCl) and dissolution above pH 6.5.

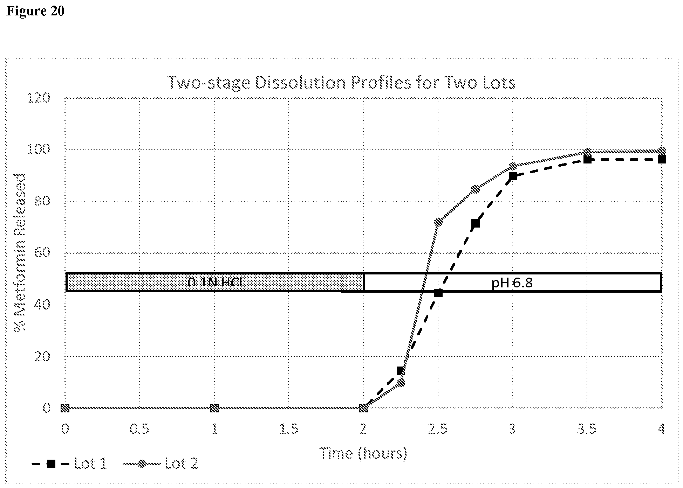

Data in Table 22 shows percent release by weight of metformin in a two-stage *in vitro* dissolution analysis as measured in a USP Type II apparatus in aqueous medium at 37°C as described in Example 4. Lot 1 corresponds to Halo 626 and is a tablet comprising 500 mg metformin and Lot 2 corresponds to Kydes 127 also comprising 500 mg metformin *(See* Figure 20).

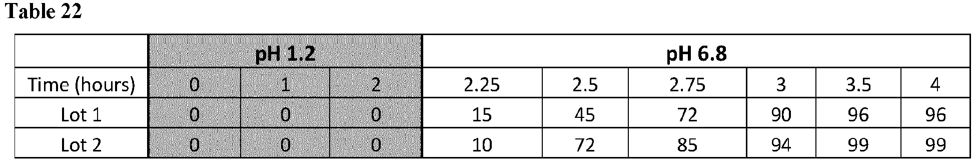

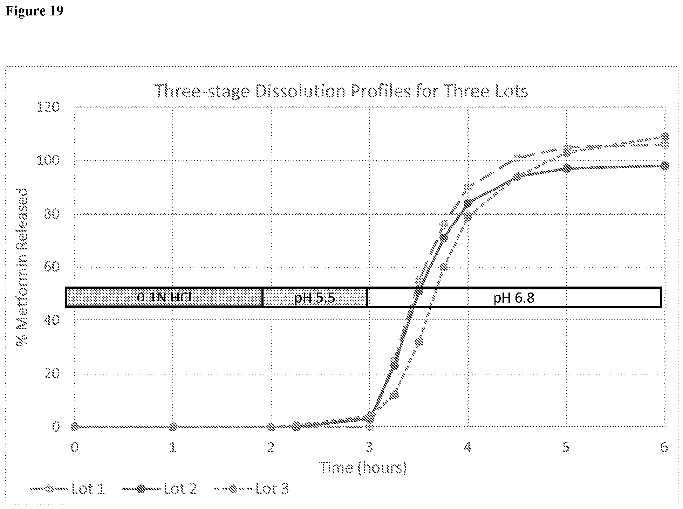

Data in Table 23 shows percent release by weight of metformin three-stage *in vitro* dissolution analysis as measured in a USP Type II apparatus in a medium at 37°C as described in Example 4. Lot 1 corresponds to Kydes-127 comprising 500 mg metformin, Lot 2 corresponds to Halo-626 comprising 500 mg metformin, and Lot 3 corresponds to Halo-625 comprising 300 mg metformin *(See* Figure 19).

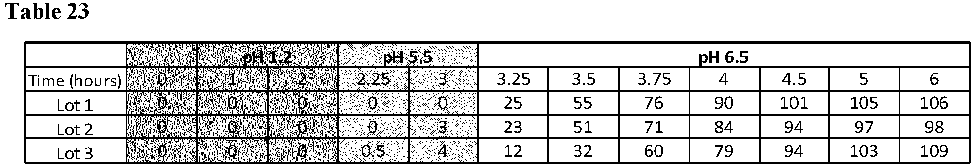

The percent weight gain and coating thickness in mg/cm² are calculated as shown in the formulations exemplified in Table 24 below.

**Table 24: sample calculation of percent weight gain and coating thickness**

| | Kydes-127 500 mg | Halo-626 500 mg | Halo-625 300mg | Kydes-89A |
|---|---|---|---|---|
| Tablet weight (mg) | 530.25 | 580.3 | 545 | 527.4 |
| % weight gain of starting tablet | 3.86% | 3.05% | 3.05% | 2.46% |
| weight gain (mg) | 20.5 | 17.7 | 16.6 | 13.0 |
| Tablet area (cm²) | 3.061 | 3.061 | 3.061 | 3.061 |
| Tablet area (in²) | 0.474382 | 0.474382 | 0.474382 | 0.474382 |
| mg/cm² | 6.694 | 5.783 | 5.424 | 4.231 |

## Claims

1. A delayed-release pharmaceutical composition for biguanide compound delivery, comprising an oral dosage form having a core comprising a therapeutically effective amount of a biguanide compound and an enteric coating surrounding said core that delays release of said biguanide compound after ingestion until reaching the distal small intestine, wherein said oral dosage form is an enterically-coated tablet or capsule, wherein said enteric coating is applied to said oral dosage form to about 3% to about a 6% (wt/wt) weight gain, and wherein less than 15% of the biguanide compound is released after the composition contacts an aqueous medium of 0.1 N HCI for two hours and is subsequently transferred to an aqueous medium at a pH of about 6.8 for a lag phase of at least ten minutes, and at least 60% of the biguanide compound is released after the lag phase and within 60 minutes at pH 6.8, and at least 90% of the biguanide compound is released within 90 to 120 minutes at pH 6.8, measured in an USP Type II apparatus in a medium at 37°C

2. The pharmaceutical composition according to claim 1, wherein said enteric coating has a coating weight of at least about 4.5 mg/cm², more preferably at least about 5.0 mg/cm².

3. The pharmaceutical composition according to any one of the preceding claims, wherein said oral dosage form releases less than about 10% or 5% of the therapeutically effective amount of said biguanide compound after contacting an aqueous medium at a pH of less than about 2 for two hours, followed by contacting an aqueous medium at a pH equal to or less than about 5.5 for 30 to 45 minutes.

4. The pharmaceutical composition according to any one of the preceding claims, wherein said oral dosage form releases less than about 15%, 10%, or 5% of the therapeutically effective amount of said biguanide compound during said lag phase.

5. The pharmaceutical composition according to any one of the preceding claims, wherein said enteric coating comprises a polymer that is insoluble in acidic media, but dissolves above pH 7.0, particularly wherein said polymer is Eudragit^{®} FS

6. The pharmaceutical composition according to any one of the preceding claims, wherein said enteric coating further comprises a polymer that is insoluble at pH 5.5 and below, but dissolves above pH 5.5, particularly wherein said polymer is Eudragit^{®} L, more particularly wherein said Eudragit^{®} FS and said Eudragit^{®} L are present in about a 7:5 to about a 5:7 ratio, most particularly wherein (i) said Eudragit^{®} FS and said Eudragit^{®} L are present in about a 6:4 to about a 4:6 ratio, or wherein (ii) said enteric coating comprises about 60% Eudragit^{®} FS and about 40% Eudragit^{®} L.

7. The pharmaceutical composition according to any one of the preceding claims, (i) further comprising a seal coating between the core and the enteric coating, providing a total coating thickness of at least about 4% to 8% (wt/wt) weight gain, more preferably at least about a 4.5% to 6% (wt/wt) weight gain, or (ii) further comprising a seal coating between the core and the enteric coating, providing a total coating thickness of at least about 21 mg/cm² to 41 mg/cm², more preferably at least about 23 mg/cm² to at least about 35 mg/cm².

8. The pharmaceutical composition according to claim 7, wherein the seal coating comprises hypromellose, polyethylene glycol 400, polysorbate 80, triacetin, talc or a combination thereof.

9. The pharmaceutical composition according to any one of the preceding claims, wherein said biguanide compound is selected from the group consisting of metformin, phenformin, buformin or imeglimin or a salt thereof.

10. The pharmaceutical composition according to any one of the preceding claims, wherein said biguanide compound comprises metformin or a salt thereof, particularly wherein said biguanide comprises metformin hydrochloride.

11. The pharmaceutical composition according to any one of claims 1-9, wherein said biguanide compound comprises imeglimin or a salt thereof.

12. The pharmaceutical composition according to any one of claims 1-10, wherein said lag phase is at least about 15 or 20 minutes after contacting a pH of at least about 6.8.

13. The pharmaceutical composition according to any one of the preceding claims, wherein said dosage form releases greater than 95%, greater than 99% or 100% of the therapeutically effective amount of said biguanide compound after contacting a pH of about 6.8 for a total of 90 to 120 minutes.

14. The pharmaceutical composition according to any one of claims 1-13 for use in a method of reducing the risk of adverse events resulting from biguanide compound administration, particularly wherein the adverse event is lactic acidosis, or wherein said adverse event is a gastrointestinal complication selected from the group consisting of nausea, diarrhea, dyspepsia, and vomiting.

15. The pharmaceutical composition according to any one of claims 1-13 for use in a method of treating metabolic disorders in a patient in need thereof, particularly wherein said patient has a contraindication for the biguanide compound, more particularly wherein the patient has a contraindication selected from the group consisting of a hypoxic condition, impaired lactate clearance, and impaired clearance of the biguanide compound, most particularly wherein the patient has moderate renal impairment, severe renal impairment, or end stage renal disease which results in impaired clearance of the biguanide compound.

16. The pharmaceutical composition for use according to claim 15, wherein the patient in need thereof has hyperglycemia, more particular wherein the hyperglycemia is chronic, most particular wherein the hyperglycemia is caused by type II diabetes.

17. The pharmaceutical composition according to any one of claims 1-13 for use in a method of reducing the onset of diabetes in a subject with pre-diabetes.

18. The pharmaceutical composition according to any one of claims 1-13 for use in a method of inducing weight loss in a subject.

19. The pharmaceutical composition according to any one of claims 1-10 for use in a method of treating type I diabetes.

20. The pharmaceutical composition according to any one of claims 1-10 for use in a method of treating dyslipidemia.

21. The pharmaceutical composition according to any one of claims 1-10 for use in a method of treating obesity.

## Patentansprüche

1. Pharmazeutische Zusammensetzung mit verzögerter Freisetzung für die Abgabe von Biguanidverbindungen, umfassend eine orale Darreichungsform mit einem Kern, der eine therapeutisch wirksame Menge einer Biguanidverbindung umfasst, und einer magensaftresistenten Beschichtung, die den Kern umgibt und die Freisetzung der Biguanidverbindung nach der Einnahme verzögert, bis sie den distalen Dünndarm erreicht, wobei die orale Darreichungsform eine magensaftresistent beschichtete Tablette oder Kapsel ist, wobei die magensaftresistente Beschichtung auf die orale Darreichungsform bis zu einer Gewichtszunahme von etwa 3 % bis etwa 6 % (Gewicht/Gewicht) aufgebracht ist und wobei weniger als 15 % der Biguanidverbindung freigesetzt wird, nachdem die Zusammensetzung für zwei Stunden mit einem wässrigen Medium aus 0,1 N HCl in Kontakt ist und anschließend für eine Verzögerungsphase von mindestens zehn Minuten in ein wässriges Medium mit einem pH-Wert von etwa 6,8 überführt wird, und mindestens 60 % der Biguanidverbindung nach der Verzögerungsphase und innerhalb von 60 Minuten bei einem pH-Wert von 6,8 freigesetzt wird, und mindestens 90 % der Biguanidverbindung innerhalb von 90 bis 120 Minuten bei einem pH-Wert von 6,8 freigesetzt wird, gemessen in einem USP Typ II Gerät in einem Medium bei 37°C.

2. Die pharmazeutische Zusammensetzung nach Anspruch 1, wobei die magensaftresistente Beschichtung ein Beschichtungsgewicht von mindestens etwa 4,5 mg/cm², vorzugsweise von mindestens etwa 5,0 mg/cm² aufweist.

3. Die pharmazeutische Zusammensetzung nach einem der vorherigen Ansprüche, wobei die orale Darreichungsform weniger als etwa 10 % oder 5 % der therapeutisch wirksamen Menge der Biguanidverbindung freisetzt, nachdem sie zwei Stunden mit einem wässrigen Medium mit einem pH-Wert von weniger als etwa 2 in Kontakt gebracht wird, gefolgt von einem 30- bis 45-minütigen Kontakt mit einem wässrigen Medium mit einem pH-Wert von etwa 5,5 oder weniger.

4. Die pharmazeutische Zusammensetzung nach einem der vorherigen Ansprüche, wobei die orale Darreichungsform weniger als etwa 15 %, 10 % oder 5 % der therapeutisch wirksamen Menge der Biguanidverbindung während der Verzögerungsphase freisetzt.

5. Die pharmazeutische Zusammensetzung nach einem der vorherigen Ansprüche, wobei die magensaftresistente Beschichtung ein Polymer umfasst, das in sauren Medien unlöslich ist, sich aber oberhalb von pH 7,0 auflöst, insbesondere wobei das Polymer Eudragit ^{®} FS ist.

6. Die pharmazeutische Zusammensetzung nach einem der vorherigen Ansprüche, wobei die magensaftresistente Beschichtung weiterhin ein Polymer umfasst, das bei einem pH-Wert von 5,5 und darunter unlöslich ist, sich aber oberhalb von pH 5,5 auflöst, insbesondere wobei das Polymer Eudragit ^{®} L ist, vorzugsweise wobei das Eudragit ^{®} FS und das Eudragit ^{®} L in einem Verhältnis von etwa 7:5 bis etwa 5:7 vorhanden sind, vorzugsweise wobei (i) das Eudragit ^{®} FS und das Eudragit ^{®} L in einem Verhältnis von etwa 6:4 bis etwa 4:6 vorhanden sind, oder wobei (ii) die magensaftresistente Beschichtung etwa 60% Eudragit ^{®} FS und etwa 40% Eudragit ^{®} L umfasst.

7. Die pharmazeutische Zusammensetzung nach einem der vorherigen Ansprüche, (i) ferner umfassend eine Versiegelungsbeschichtung zwischen dem Kern und der magensaftresistenten Beschichtung, die eine Gesamtbeschichtungsdicke von mindestens etwa 4 % bis 8 % (Gew./Gew.) Gewichtszunahme, vorzugsweise mindestens etwa 4,5 % bis 6 % (Gew./Gew.) Gewichtszunahme, ergibt, oder (ii) ferner umfassend eine Versiegelungsbeschichtung zwischen dem Kern und der magensaftresistenten Beschichtung, die eine Gesamtbeschichtungsdicke von mindestens etwa 21 mg/cm² bis 41 mg/cm², vorzugsweise mindestens etwa 23 mg/cm² bis mindestens etwa 35 mg/cm², ergibt.

8. Die pharmazeutische Zusammensetzung nach Anspruch 7, wobei die Versiegelungsbeschichtung Hypromellose, Polyethylenglykol 400, Polysorbat 80, Triacetin, Talkum oder eine Kombination davon umfasst.

9. Die pharmazeutische Zusammensetzung nach einem der vorherigen Ansprüche, wobei die Biguanidverbindung ausgewählt ist aus der Gruppe bestehend aus Metformin, Phenformin, Buformin oder Imeglimin oder einem Salz davon.

10. Die pharmazeutische Zusammensetzung nach einem der vorherigen Ansprüche, wobei die Biguanidverbindung Metformin oder ein Salz davon umfasst, insbesondere wobei das Biguanid Metforminhydrochlorid umfasst.

11. Die pharmazeutische Zusammensetzung nach einem der Ansprüche 1-9, wobei die Biguanidverbindung Imeglimin oder ein Salz davon umfasst.

12. Die pharmazeutische Zusammensetzung nach einem der Ansprüche 1-10, wobei die Verzögerungsphase mindestens etwa 15 oder 20 Minuten nach Kontakt mit einem pH-Wert von mindestens etwa 6,8 beträgt.

13. Die pharmazeutische Zusammensetzung nach einem der vorherigen Ansprüche, wobei die Darreichungsform mehr als 95 %, mehr als 99 % oder 100 % der therapeutisch wirksamen Menge der Biguanidverbindung freisetzt, nachdem sie für insgesamt 90 bis 120 Minuten mit einem pH-Wert von etwa 6,8 in Kontakt gebracht wurde.

14. Die pharmazeutische Zusammensetzung nach einem der Ansprüche 1-13 zur Verwendung in einem Verfahren zur Verringerung des Risikos von unerwünschten Nebenwirkungen, die sich aus der Verabreichung einer Biguanidverbindung ergeben, insbesondere wobei die unerwünschte Nebenwirkung eine Laktatazidose ist, oder wobei die unerwünschte Nebenwirkung eine gastrointestinale Komplikation ist ausgewählt aus der Gruppe bestehend aus Übelkeit, Diarrhöe, Dyspepsie und Erbrechen.

15. Die pharmazeutische Zusammensetzung nach einem der Ansprüche 1-13 zur Verwendung in einem Verfahren zur Behandlung von Stoffwechselstörungen bei einem Patienten, insbesondere wenn der Patient eine Kontraindikation für die Biguanidverbindung aufweist, vor allem wenn der Patient eine Kontraindikation aufweist ausgewählt aus der Gruppe bestehend aus einem hypoxischen Zustand, einem beeinträchtigten Laktatabbau und einem beeinträchtigten Abbau der Biguanidverbindung, insbesondere wobei der Patient eine moderate Nierenbeeinträchtigung, eine schwere Nierenbeeinträchtigung oder eine Nierenerkrankung im Endstadium aufweist, die zu einem beeinträchtigten Abbau der Biguanidverbindung führt.

16. Die pharmazeutische Zusammensetzung zur Verwendung gemäß Anspruch 15, wobei der Patient an Hyperglykämie leidet, insbesondere wobei die Hyperglykämie chronisch ist, insbesondere wobei die Hyperglykämie durch Typ-II-Diabetes verursacht wird.

17. Die pharmazeutische Zusammensetzung nach einem der Ansprüche 1-13 zur Verwendung in einem Verfahren zur Verringerung des Auftretens von Diabetes bei einem Patienten mit Prädiabetes.

18. Die pharmazeutische Zusammensetzung nach einem der Ansprüche 1-13 zur Verwendung in einem Verfahren zur Herbeiführung einer Gewichtsabnahme bei einer Person.

19. Die pharmazeutische Zusammensetzung nach einem der Ansprüche 1-10 zur Verwendung in einem Verfahren zur Behandlung von Typ-I-Diabetes.

20. Die pharmazeutische Zusammensetzung nach einem der Ansprüche 1-10 zur Verwendung in einem Verfahren zur Behandlung von Dyslipidämie.

21. Die pharmazeutische Zusammensetzung nach einem der Ansprüche 1-10 zur Verwendung in einem Verfahren zur Behandlung von Fettleibigkeit.

## Revendications

1. Composition pharmaceutique à libération retardée pour une administration de composé biguanide, comprenant une forme galénique orale ayant un coeur comprenant une quantité thérapeutiquement efficace d'un composé biguanide et un enrobage gastro-résistant entourant ledit coeur qui retarde la libération dudit composé biguanide après ingestion jusqu'à atteinte de l'intestin grêle distal, dans laquelle ladite forme galénique orale est un comprimé ou une gélule à enrobage gastro-résistant, ledit enrobage gastro-résistant est appliqué à ladite forme galénique à un gain de poids d'environ 3 % à environ 6 % (p/p), et dans laquelle moins de 15 % du composé biguanide est libéré après que la composition entre en contact avec un milieu aqueux à 0,1 N de HCl pendant deux heures et est transférée subséquemment dans un milieu aqueux à un pH d'environ 6,8 pendant une phase de latence d'au moins dix minutes, et au moins 60 % du composé biguanide est libéré après la phase de latence et dans les 60 minutes à un pH de 6,8 et au moins 90 % du composé biguanide est libéré dans les 90 à 120 minutes à pH 6,8, mesuré dans un appareil USP de type Il dans un milieu à 37°C.

2. Composition pharmaceutique selon la revendication 1, dans laquelle ledit enrobage gastro-résistant a un poids d'enrobage d'au moins environ 4,5 mg/cm², préférentiellement d'au moins environ 5,0 mg/cm².

3. Composition pharmaceutique selon l'une quelconque des revendications précédentes, dans laquelle ladite forme galénique orale libère moins d'environ 10 % ou 5 % de la quantité thérapeutiquement efficace dudit composé biguanide après la mise en contact d'un milieu aqueux à un pH de moins d'environ 2 pendant deux heures, suivie par la mise en contact d'un milieu aqueux à un pH égal ou inférieur à environ 5,5 pendant 30 à 45 minutes.

4. Composition pharmaceutique selon l'une quelconque des revendications précédentes, dans laquelle ladite forme galénique orale libère moins d'environ 15 %, 10 % ou 5 % de la quantité thérapeutiquement efficace dudit composé biguanide au cours de ladite phase de latence.

5. Composition pharmaceutique selon l'une quelconque des revendications précédentes, dans laquelle ledit enrobage gastro-résistant comprend un polymère qui est insoluble en milieux acides mais se dissout au-dessus de pH 7,0, en particulier dans laquelle ledit polymère est l'Eudragit^{®} FS.

6. Composition pharmaceutique selon l'une quelconque des revendications précédentes, dans laquelle ledit enrobage gastro-résistant comprend en outre un polymère qui est insoluble à pH 5,5 et en dessous, mais se dissout au-dessus de pH 5,5, en particulier dans laquelle ledit polymère est l'Eudragit^{®} L, plus particulièrement dans laquelle lesdits Eudragit^{®} FS et Eudragit^{®} L sont présents en un rapport d'environ 7:5 à environ 5:7, de manière la plus particulière dans laquelle (i) lesdits Eudragit^{®} FS et Eudragit^{®} L sont présents à un rapport d'environ 6:4 à environ 4:6, ou dans laquelle (ii) ledit enrobage gastro-résistant comprend environ 60 % d'Eudragit^{®} FS et environ 40 % d'Eudragit^{®} L.

7. Composition pharmaceutique selon l'une quelconque des revendications précédentes, (i) comprenant en outre un enrobage de scellement entre le coeur et l'enrobage gastro-résistant, donnant une épaisseur d'enrobage totale d'au moins environ 4 % à 8 % (p/p) de gain de poids, préférentiellement d'au moins environ 4,5 % à 6 % (p/p) de gain de poids, ou (ii) comprenant en outre un enrobage de scellement entre le coeur et l'enrobage gastro-résistant, donnant une épaisseur d'enrobage totale d'au moins environ 21 mg/cm² à 41 mg/cm², préférentiellement d'au moins environ 23 mg/cm² à au moins environ 35 mg/cm².

8. Composition pharmaceutique selon la revendication 7, dans laquelle l'enrobage de scellement comprend de l'hypromellose, du polyéthylène glycol 400, du polysorbate 80, de la triacétine, du talc ou une combinaison de ceux-ci.

9. Composition pharmaceutique selon l'une quelconque des revendications précédentes, dans laquelle ledit composé biguanide est choisi dans le groupe consistant en métformine, phénformine, buformine ou iméglimine ou un sel de celles-ci.

10. Composition pharmaceutique selon l'une quelconque des revendications précédentes, dans laquelle ledit composé biguanide comprend de la métformine ou un sel de celle-ci, en particulier dans laquelle ledit biguanide comprend du chlorhydrate de métformine.

11. Composition pharmaceutique selon l'une quelconque des revendications 1 à 9, dans laquelle ledit composé biguanide comprend de l'iméglimine ou un sel de celle-ci.

12. Composition pharmaceutique selon l'une quelconque des revendications 1 à 10, dans laquelle ladite phase de latence est d'au moins environ 15 ou 20 minutes après la mise en contact d'un pH d'au moins environ 6,8.

13. Composition pharmaceutique selon l'une quelconque des revendications précédentes, dans laquelle ladite forme galénique libère plus de 95 %, plus de 99 % ou 100 % de la quantité thérapeutiquement efficace dudit composé biguanide après la mise en contact d'un pH d'environ 6,8 pendant un total de 90 à 120 minutes.

14. Composition pharmaceutique selon l'une quelconque des revendications 1 à 13 pour une utilisation dans une méthode de réduction des événements négatifs résultant de l'administration du composé biguanide, en particulier dans laquelle l'évènement négatif est une acidose lactique, ou dans laquelle ledit événement négatif est une complication gastrointestinale choisie dans le groupe consistant en la nausée, la diarrhée, la dyspepsie, et les vomissements.

15. Composition pharmaceutique selon l'une quelconque des revendications 1 à 13 pour une utilisation dans une méthode de traitement de troubles métaboliques chez un patient qui en a besoin, en particulier dans laquelle ledit patient a une contre-indication pour le composé biguanide, de manière plus particulière dans laquelle le patient a une contre-indication choisie dans le groupe se composant d'un état hypoxique, d'une clairance altérée du lactate, et d'une clairance altérée du composé biguanide, de manière la plus particulière dans laquelle le patient a une insuffisance rénale modérée, une insuffisance rénale sévère, ou une maladie rénale à un stade terminal qui aboutit à une clairance altérée du composé biguanide.

16. Composition pharmaceutique pour une utilisation selon la revendication 15, dans laquelle le patient qui en a besoin est atteint d'hyperglycémie, de manière plus particulière dans laquelle l'hyperglycémie est chronique, de manière la plus particulière dans laquelle l'hyperglycémie est provoquée par le diabète de type II.

17. Composition pharmaceutique selon l'une quelconque des revendications 1 à 13 pour une utilisation dans une méthode de réduction de l'apparition du diabète chez un sujet atteint de pré-diabète.

18. Composition pharmaceutique selon l'une quelconque des revendications 1 à 13 pour une utilisation dans une méthode d'induction de perte de poids chez un sujet.

19. Composition pharmaceutique selon l'une quelconque des revendications 1 à 10 pour une utilisation dans une méthode de traitement du diabète de type I.

20. Composition pharmaceutique selon l'une quelconque des revendications 1 à 10 pour une utilisation dans une méthode de traitement de la dyslipidémie.

21. Composition pharmaceutique selon l'une quelconque des revendications 1 à 10 pour une utilisation dans une méthode de traitement de l'obésité.
